(19) **Europäisches Patentamt European Patent Office Office européen des brevets**

(11) **EP 1 117 695 B1**

(12) **FASCICULE DE BREVET EUROPEEN**

(45) Date de publication et mention de la délivrance du brevet:
**20.05.2015 Bulletin 2015/21**

(21) Numéro de dépôt: **99932921.2**

(22) Date de dépôt: **20.07.1999**

(51) Int Cl.:
*A61K 31/721* (2006.01)   *A61K 31/765* (2006.01)
*A61K 31/795* (2006.01)   *C08B 37/02* (2006.01)
*C08G 63/688* (2006.01)

(86) Numéro de dépôt international:
**PCT/FR1999/001774**

(87) Numéro de publication internationale:
**WO 2000/005270 (03.02.2000 Gazette 2000/05)**

(54) **POLYMERES BIOCOMPATIBLES, LES COMPOSITIONS LES CONTENANT ET LEUR UTILISATION POUR LA PRÉPARATION DE MÉDICAMENTS**

BIOVERTRÄGLICHE POLYMERE, DIESE ENTHALTENDE ZUSAMMENSETZUNGEN UND DEREN VERWENDUNG ZUR HERSTELLUNG VON ARZNEIMITTELN

BIOCOMPATIBLE POLYMERS, COMPOSITIONS CONTAINING SAME AND THEIR USE FOR THE MANUFACTURE OF DRUGS

(84) Etats contractants désignés:
**AT BE CH CY DE DK ES FI FR GB GR IE IT LI LU MC NL PT SE**

(30) Priorité: **21.07.1998 FR 9809309**

(43) Date de publication de la demande:
**25.07.2001 Bulletin 2001/30**

(60) Demande divisionnaire:
**10182823.4 / 2 289 970**

(73) Titulaires:
• **Barritault, Denis**
 **75001 Paris (FR)**
• **Caruelle, Jean-Pierre**
 **94100 Saint-Maur-des-Fossés (FR)**

(72) Inventeurs:
• **Barritault, Denis**
 **75001 Paris (FR)**
• **Caruelle, Jean-Pierre**
 **94100 Saint-Maur-des-Fossés (FR)**

(74) Mandataire: **Novagraaf Technologies**
**Bâtiment O2**
**2, rue Sarah Bernhardt**
**CS90017**
**92665 Asnières-sur-Seine Cedex (FR)**

(56) Documents cités:
**EP-A- 0 557 887     DE-B- 1 152 396
FR-A- 894 508**

• **MAIGA-REVEL O ET AL: "NEW INVESTIGATIONS ON HEPARIN-LIKE DERIVAIZED DEXTRANS: CMDBS, SYNERGISTIC ROLE OF BENZYLAMIDE AND SULFATE SUBSTITUENTS IN ANTICOAGULANT ACTIVITY" CARBOHYDRATE POLYMERS, vol. 32, no. 2, 1 février 1997 (1997-02-01), pages 89-93, XP000686949 ISSN: 0144-8617**
• **LEBOUCHER-DURAND M -A ET AL: "Poly(beta-malic acid) derivatives with unsaturated lateral groups: epoxidation as model reaction of the double bonds reactivity" REACTIVE & FUNCTIONAL POLYMERS, vol. 31, no. 1, 1 août 1996 (1996-08-01), page 57-65 XP004052633 ISSN: 1381-5148**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

**Description**

La présente invention concerne de nouveaux polymères biocompatibles, les compositions les contenant <u>et leur utilisation</u>.

On connaît dans l'art antérieur des polymères dérivés de dextranes obtenus par substitution par des résidus carboxyméthyle, carboxyméthyle-benzylamide et carboxyméthyle-benzylamide-sulfonate. Ces polymères, leur procédé de préparation et leur propriété ont été décrits dans le brevet Français No. 2 461 724 ainsi que dans le brevet US No. 4 740 594. Parmi ces polymères, certains miment les propriétés de l'héparine et peuvent être utilisés en tant que produits de substitution du plasma grâce à leurs propriétés anti-coagulante et anti-complémentaire. D'autres miment une propriété différente de l'héparine qui consiste en une stabilisation, une protection et une potentialisation de l'activité biologique *in vitro* des facteurs de croissance de la famille des FGF (Tardieu et coll., Journal of Cellular Physiology, 1992, 150, Pages 194 à 203).

Par ailleurs, le brevet Français No. 2 644 066 décrit l'utilisation de dérivés de dextrane carboxyméthyl-benzylamide-sulfonate, désignés CMDBS, seuls ou associés aux FGFs pour la cicatrisation.

Plus récemment, il a été proposé dans les brevets Français No. 2 718 023, No. 2 718 024 et No. 2 718 026 d'utiliser des polymères capables de protéger, stabiliser et potentialiser les facteurs de croissance à affinité pour l'héparine, tels les facteurs de croissance des fibroblastes ou FGF et du Transforming Growth Factor bêta (TGF$\beta$), comme médicament pour le traitement des lésions respectivement du tractus digestif, du système nerveux, des tissus musculaires. Pour illustrer cet effet protecteur des dérivés de dextrane CMDBS, ces brevets rapportent les résultats de la digestion protéolytique par de la trypsine du FGF1, du FGF2 ou du TGF$\beta$. Ces propriétés de protection, de stabilisation et de potentialisation des facteurs de croissance à affinité pour l'héparine ont permis de caractériser une nouvelle classe de polymères, désignés HBGFPP pour "Heparin-Binding Growth Factor Protector and Potentiator", présentant une activité cicatrisante et réparatrice des tissus musculaires, nerveux et du tractus digestif et dépourvus, aux doses utilisées, d'activités anticoagulantes.

Outre, les applications des HBGFPP ci-dessus, le brevet Français No. 2 718 025 propose l'utilisation de ces polymères comme médicament pour le traitement des inflammations. Cette activité anti-inflammatoire est illustrée par l'activité d'inhibition *in vitro* d'enzymes protéolytiques impliquées dans la réaction inflammatoire comme l'élastase leucocytaire ou la plasmine et *in vivo* par des études histologiques démontrant une réaction cellulaire inflammatoire réduite dans les tissus traités par des dérivés de dextrane CMDBS.

L'article de Maiga-Revel et al. de 1997 (Carbohydrate polymers, vol. 32, pages 89-93) décrit, outre les CMDBS déjà abordés dans le présent mémoire, quatre dérivés du dextran désignés CMDSu dans lesquels D représente du dextran, CM des groupes carboxyméthyles et Su des groupes sulfates. Cet article décrit également que les CMDSu ont une activité anti-coagulante inférieure à 3,5 UI/mg. Enfin, la demande de brevet DE 11 52 396 décrit des dérivés de dextrane fonctionnalisés par des groupes carboxyalkyles et sulfates qui présentent une activité capable de prévenir la coagulation du sang, une activité anti-complémentaire ainsi qu'une activité sur la calcification du plasma et sur le temps de thrombine.

Toutefois, les dérivés de dextrane CMDBS sont des composés dont la synthèse est difficile et qui présentent le risque de relarguer des résidus connus pour leurs effets toxiques comme la benzylamine.

En outre, les applications des HBGFPP et donc du CMDBS proposées dans l'art antérieur sont limitées à la réparation et à la cicatrisation de certains tissus seulement.

La présente invention vise précisément à palier ces inconvénients en offrant de nouveaux polymères biocompatibles faciles à préparer et présentant les propriétés des HBGFPP mais aussi, de façon inattendue, de nouvelles propriétés permettant des champs d'application notamment thérapeutiques très étendus qui ne sont pas limités à quelques types de d'organes, de tissus ou de cellules particuliers.

Les polymères de l'invention seront aussi désignés ci-après "RGTA" pour "ReGeneraTing Agents" (Agents de régénération).

<u>Les polymère de l'invention</u>

Des polymères décrits ont une masse molaire supérieure à environ 5 000 Daltons et sont constitués par un enchaînement de motifs identiques ou différents, de formule générale (I) suivante :

$$A_a \, X_x \, Y_y$$

dans laquelle :

- A représente un monomère;
- X représente un groupement carboxyle fixé sur un monomère A et répondant à la formule suivante :-R-COO-R', où R est une liaison ou une chaîne hydrocarbonée aliphatique, éventuellement ramifiée et/ou insaturée et qui peut contenir un ou plusieurs cycles aromatiques à l'exception de la benzylamine et de la benzylamine sulfonate, et R' représente un atome d'hydrogène ou un cation,

- Y représente un groupement sulfate ou sulfonate fixé sur un monomère A et répondant à l'une des formules suivantes : -R-O-SO3 -R', -R-N-SO3-R', -R-SO3-R', où R est une liaison ou une chaîne hydrocarbonée aliphatique, éventuellement ramifiée et/ou insaturée et qui peut contenir un ou plusieurs cycles aromatiques à l'exception de la benzylamine et de la benzylamine sulfonate, et R' représente un atome d'hydrogène ou un cation,
- a représente le nombre de monomères A, qui est tel que la masse desdits polymères de formule (I) est supérieure à environ 5 000 daltons sont des polymères biocompatibles constitués par un enchaînement de motifs identiques ou différents, de formule générale (II) suivante :

$$A_a \, X_x \, Y_y \, Z_z$$

dans laquelle :

- A est un monomère de glucose;
- X est $CH_2COOH$ ou $CH_2COO^-Na^+$,
- Y est $-SO_3H$ ou $-SO_3 \, Na^+$,
- Z représente des groupements chimiques fonctionnels à l'exception de la benzylamine et de la benzylamine sulfonate, identiques ou différents et différents de X et Y, choisis parmi les acides aminés, les acides gras, les alcools gras, les céramides, des dérivés de ceux-ci, les séquences nucléotidiques d'adressage, les agents thérapeutiques et les agents de diagnostic,
les groupements X et Y étant fixés sur le monomère A,
- a représente le nombre de monomères A, la masse desdits polymères de formule AaXxYy est supérieure à 5 000 daltons
- x représente le taux de substitution de l'ensemble des monomères A par les groupements X, lequel est compris entre environ 20 et 150%,
- y représente le taux de substitution de l'ensemble des monomères A par les groupements Y, qui est compris entre environ 30 et 150%,
- z représente le taux de substitution de l'ensemble des monomères A par des groupements Z, où 0%<z<50%.
- x représente le taux de substitution de l'ensemble des monomères A par les groupements X, lequel est compris entre environ 20 et 150%, et de préférence est de l'ordre de 50%,
- y représente le taux de substitution de l'ensemble des monomères A par les groupements Y, qui est compris entre environ 30 et 150%, et de préférence est de l'ordre de 100%.

Avantageusement dans la formule (I), le radical R des groupement X et Y, est choisi parmi un groupement alkyle, allyle, aryle, linéaires ou ramifiés.

Dans la définition des taux de substitutions ci-dessus, on entend par un taux de substitution x de 100 %, le fait que chaque monomère A du polymère de l'invention contient statistiquement un groupement X. De même, on entend par un taux de substitution y de 100 %, le fait que chaque monomère du polymère de l'invention contient statistiquement un groupement Y. Les taux de substitution supérieurs à 100 % traduisent le fait que chaque monomère porte statistiquement plus d'un groupement du type considéré; à l'inverse, les taux de substitution inférieurs à 100 % traduisent le fait que chaque monomère porte statistiquement moins d'un groupement du type considéré.

Les polymères offrent l'avantage remarquable de présenter *in vivo* une dégradabilité lente, ce qui les différencient des héparanes sulfates qui sont des produits naturellement et rapidement dégradés par l'héparinase ou l'héparitinase. En outre, les polymères de l'invention ne contiennent pas ou ne libèrent après dégradation des produits toxiques contrairement au CMDBS qui possèdent des groupements benzylamines.

Les monomères A qui constituent les éléments de base des polymères de formule I peuvent être identiques ou différents, et sont choisis parmi tout type de monomomère, comme par exemple ceux proposés pour les HBGFPP tels que des sucres, des esters, des alcools, des acides aminés, des nucléotides, etc.... Parmi ceux-ci, on préfère plus particulièrement les oses et notamment le glucose.

Le nombre de monomères A est défini de façon à ce que la masse des polymères de l'invention soit supérieure à environ 5 000 daltons. En conséquence, les RGTA peuvent être constitués de diverses structures polymérisables homomériques comme hétéromériques comme par exemple : les copolymères polyesters de biosynthèse ou de synthèse chimique comme les polyesters aliphatiques ou ceux d'origine naturelle comme les polyhydroxyalcanoates. Il peut aussi s'agir de polysaccharides et de leurs dérivés qu'ils soient d'origine bactérienne comme la cellulose, le xanthane, le dextrane, etc..., extraits des végétaux unicellulaires ou pluricellulaires comme l'amidon, la cellulose végétale ou les alginates et leurs dérivés, les fucanes et leurs dérivés, etc... ou encore extraits des animaux comme l'acide hyaluronique ou la chitine, etc... ou de synthèse obtenu par exemple par copolymérisation. Des protéines de synthèse comme des poly acides aminés naturels ou chimiquement modifiés, comme par exemple le collagène, peuvent également entrer dans la constitution des polymères de l'invention.

Le choix de la structure polymérique des RGTA repose sur les différentes formes de présentation susceptibles d'être utilisées selon le tissu ou l'organe à traiter ou des caractéristiques physico-chimiques comme la solubilité, la conformation spatiale avec des solutions, des suspensions, des gels, des poudres des éponges, des films, des matériaux modelables compacts, poreux semi-poreux ou non poreux, délitables ou non érodables ou non, colonisables ou non, etc...

L'héparine ou ses fragments à faible propriété anticoagulante et les héparanes sulfatés naturels et leurs fragments sont exclus du champ de la présente invention, car :

- Leur activité anti-coagulante peut être supérieure à 50 UI. En effet, les polymères de l'invention sont dépourvus d'activité anticoagulante significative, c'est à dire qu'ils présentent une activité inférieure à 50 UI/milligramme comparée à celle de l'héparine dont l'activité est de l'ordre de 150 à 170 UI/milligramme.
- Ils présentent une biodégradabilité trop rapide sous l'action d'enzymes de type héparinase ou héparitinase pour permettre les effets biologiques obtenus dans le cadre de la présente invention.

Les polymères décrit peuvent également comprendre des groupements chimiques fonctionnels, désignés Z, différents de X et Y, capables de conférer aux polymères de l'invention des propriétés biologiques ou physicochimiques supplémentaires. Ainsi, les groupement Z sont utiles pour conférer aux RGTA une meilleure solubilité ou lipophilie permettant une meilleure diffusion ou pénétration tissulaire, par exemple accroître l'amphiphilie, permettre le franchissement de la barrière hématoencéphalique, etc... A titre d'exemple, les groupements Z, identiques ou différents, peuvent être des acides aminés, des acides gras, des alcools gras, des céramides, ou des dérivés de ceux-ci, ou encore des séquences nucléotidiques d'adressages, etc...

Les groupement Z peuvent aussi représenter des agents actifs identiques ou différents, comme des agents thérapeutiques ou de diagnostic, par exemple un anti-inflammatoire, un anti-microbien, un antibiotique, etc..., ou encore une enzyme, un facteur de croissance, etc....

Des polymères de l'invention dans lesquels Z est présent répondent à la formule II suivante :

$$A_a \, X_x \, Y_y \, Z_z$$

dans laquelle, dans laquelle :

- A est un monomère de glucose;
- X est $CH_2COOH$ ou $CH_2COO^-Na^+$,
- Y est $-SO_3H$ ou $-SO_3^-Na^+$,
- Z représente des groupements chimiques fonctionnels à l'exception de la benzylamine et de la benzylamine sulfonate, identiques ou différents et différents de X et Y, choisis parmi les acides aminés, les acides gras, les alcools gras, les céramides, des dérivés de ceux-ci, les séquences nucléotidiques d'adressage, les agents thérapeutiques et les agents de diagnostic,
  les groupements X et Y étant fixés sur le monomère A,
- a représente le nombre de monomères A, la masse desdits polymères de formule AaXxYy est supérieure à 5 000 daltons
- x représente le taux de substitution de l'ensemble des monomères A par les groupements X, lequel est compris entre environ 20 et 150%,
- y représente le taux de substitution de l'ensemble des monomères A par les groupements Y, qui est compris entre environ 30 et 150%,
- z représente le taux de substitution de l'ensemble des monomères A par des groupements Z, où $0\% < z \leq 50\%$.

les groupements Z peuvent être fixés par covalence directement sur les monomères A ou fixés par covalence sur les groupements X et/ou Y.

Mais, les groupements Z peuvent aussi être conjugués aux polymères de formule (I) par des liaisons autres que covalentes, comme des interactions ioniques ou hydrophiles, selon la nature de A, X et Y. Les polymères de l'invention peuvent alors constituer un système de vectorisation de Z. Ainsi, les polymères de l'invention sont utiles pour véhiculer un agent actif Z, comme un facteur de croissance ou une enzyme, au niveau de tissus lésés ou souffrant définis plus loin dans le cadre des applications des polymères de l'invention.

Les polymères dans lesquels les groupes R de X et Y ou les groupements Z sont susceptibles d'induire directement ou après dégradation des effets toxiques n'entrent pas dans le cadre de la présente invention. On peut citer parmi les groupements Z exclus de l'invention, ceux qui constituent des agents mutagènes ou connus pour être carcinogènes ou ayant d'autres toxicités. Ceci est le cas de la benzylamine qui peut être relarguée à partir du groupement benzylamide contenu dans le CMDBS et dont les propriétés carcinogènes sont bien connues de l'homme de l'art (Wiessler M, et al., Carcinogenesis. 1983; 4(7): 867-871 ; Singer GM, et al., J Med. Chem. 1983; 26(3): 309-312). En conséquence, le CMDBS est exclu des polymères de l'invention.

L'invention concerne aussi les compositions pharmaceutiques ou de diagnostic contenant au moins un polymère de formule (II) défini ci-dessus associé dans ladite composition avec un véhicule pharmaceutiquement acceptable. Ces compositions sont utilisables chez l'homme comme chez l'animal. En effet, comme les HBGFPP décrits dans l'art antérieur, les polymères de l'invention présentent les propriétés suivantes:

- Ils sont dépourvus d'activité anticoagulante significative, ce qui signifie qu'ils présentent une activité inférieure à 50 UI/milligramme comparée à celle de l'héparine dont l'activité est de l'ordre de 150 à 170 UI/milligramme.
- Ils stabilisent et potentialisent les facteurs de croissance présentant une affinité pour l'héparine et en particulier à titre d'exemple le FGF1 et/ou le FGF2 et le TGF β.
- Ils protègent ces facteurs contre des agents protéolytiques comme la trypsine.
- Ils inhibent les activités protéasiques impliquées dans le processus inflammatoire comme par exemple élastase leucocytaire ou la plasmine.
- Ils exercent un effet cicatrisant dans au moins les modèles présentés dans les brevets suscités, sur les muscles, les nerfs ou le tractus digestif.

Les polymères de l'invention constituent donc une nouvelle classe d'agents favorisant la réparation des tissus musculaires, nerveux et du tractus digestif et présentant, comme cela est rapporté dans le brevet US No. 5 693 625 à propos du CMDBS, des propriétés sur la cicatrisation cutanée et de la cornée ou sur l'os plat tel que décrit par Blanquaert F, et al. (Bone, 1995; 17(6): 499-506). Mais, les polymères de l'invention présentent aussi des propriétés inattendues par rapport au CMDBS. On peut citer à titre d'exemple, l'effet du CMDBS sur la réparation des défauts osseux dans le crâne (Blanquaert F, et al., Bone, 1995; 17(6): 499-506)), qui a priori, n'était pas transposable sur l'os long. L'os plat et l'os long sont de nature totalement différente car :

- Ils sont d'origine embryologique différente. L'os plat est un dérivé des cellules du tissu conjonctif alors que l'os long dérive des cellules cartilagineuses.
- L'os plat est un os spongieux et ne contient pas de canal médullaire par opposition à l'os long.
- L'os plat ne cicatrise pas naturellement lorsqu'il y a une perte de substance comme lors d'une trépanation comme le montre l'absence de cicatrisation sur des cranes provenant de civilisations préhistoriques comme les Incas ou les Egyptiens (voir par exemple Evidence for stone age cranial surgery,7000 BC, Nature, 1997, 367, 360). Or, de manière surprenante, les polymères de l'invention présentent des effets sur la réparation de défauts osseux très importants, de l'ordre du tiers de la longueur de la diaphyse d'un fémur de rat. Il a été observé non seulement une réparation avec la reformation d'un fût osseux mais aussi que celui ci était rempli de moelle osseuse, c'est à dire que le traitement par le RGTA a conduit à la reformation d'un véritable canal médullaire alors qu'en l'absence de RGTA le défaut est simplement comblé de matériel osseux désorganisé. Il en est de même des incisions cutanées car cette réparation est d'une telle qualité qu'il n'y a pratiquement plus la trace d'une cicatrice.

L'invention se rapporte donc tout particulièrement aux polymères biocompatibles de (II) décrits précédemment dépourvus d'activité anti-coagulante significative. Ces polymères présentent une activité anti-coagulante inférieure à 10 UI/mg à des doses uniques ou hebdomadaires comprises :

- entre 0,001 mg et 1 mg par cm$^3$ lorsque l'application est locale,
- entre 0,1 mg/kg et 100 mg/kg lorsque l'administration est effectuée par voie systémique, par exemple intraveineuse,
- entre 0,2 mg/kg et 500 mg/kg lorsque l'administration est effectuée par voie intramusculaire,
- entre 0,1 mg/kg à 5 g/kg pour une administration per os.

L'activité anti-coagulante des polymères de l'invention aux dose indiquées ci-dessus est, pour un homme de 70 kg, inférieure à :

- 10 UI pour une application locale,
- 100 UI pour une application intraveineuse,
- 500 UI pour une application intramusculaire.

De façon remarquables, les polymères de l'invention sont dépourvus d'activité anti-coagulante significative aux dose ci-dessus, mais présentent la capacité de préserver et/ou de restaurer l'homéostasie tissulaire. L'invention se rapporte donc tout particulièrement à l'utilisation des polymères ci-dessus pour la préparation d'un médicament utile pour la prévention ou le traitement des dysfonctionnements de l'homéostasie tissulaire et ne présentant pas d'activité anti-coagulante significative.

De manière tout aussi surprenante, les Inventeurs ont mis en évidence que les polymères de l'invention peuvent être administrés par des voies autres que celles décrites dans l'art antérieur pour les CMDBS qui n'envisageait qu'une administration locale, au niveau du tissu lésé. Ainsi, il est possible de choisir parmi les polymères de l'invention ceux qui sont appropriés, par exemple du fait leur solubilité, au mode d'administration choisi : intra veineuse, intra artérielle, intra musculaire, intra péritonéenne, intra oculaire, dans le liquide céphalorachidien ou directement dans le système nerveux central de façon à s'affranchir de la barrière hématoencéphalique ou encore par voie orale; toutes ces voies d'administration, éventuellement même combinées, se sont révélées particulièrement efficaces.

L'invention se rapporte donc à toute composition pharmaceutique dans laquelle le polymère de l'invention est associé à un véhicule pharmaceutique sous une forme permettant l'administration per os, per cutanée, sous cutanée, topique, intramusculaire, intraveineuse ou intra artérielle ou dans tous les compartiments liquidiens de l'organisme. Les travaux menés dans le cadre de la présente invention ont permis de mettre en évidence que les RGTA agissent plus particulièrement dans des fenêtres de doses en dehors desquelles des effets biologiques toujours satisfaisants ne sont pas obtenus. Selon les voies d'administration et le type de lésion, les doses efficaces optimales sont celles définies ci-dessous.

Avantageusement, de manière à recouvrir toute la surface d'une plaie ou à remplir le volume d'une lésion tissulaire, les compositions pharmaceutiques de l'invention sont dosées de façon à permettre une administration de 0,001 à 1 milligramme de polymère par centimètre carré ou de 0,005 à 1 milligramme de polymère par centimètre cube de tissu à traiter. Ainsi, les composition de l'invention contiennent de préférence entre 0,01 et 10 milligramme de polymère par millilitre de solution physiologique de dissolution.

Pour une administration par voie systémique (veineuse, artérielle), par voie intra-péritonéale, intra oculaire, dans le liquide céphalorachidien, dans le liquide intracochléaire ou dans tous fluides péri ou intra tissulaires, les compositions de l'invention sont dosées de façon à permettre une administration de 0,1 et 100 milligrammes de polymère par kilogramme de poids d'homme ou d'animal à traiter.

Pour une administration par voie intramusculaire, les compositions de l'invention sont dosées de façon à permettre une administration comprises entre 0,2 et 500 milligrammes de polymère par kilogramme de poids d'homme ou d'animal à traiter, et de préférence entre 1 et 50 mg par kg.

Pour une administration per os, les compositions de l'invention sont dosées de façon à permettre une administration comprise entre 1 et 5000 milligrammes de polymère par kilogramme de poids d'homme ou d'animal à traiter, et de préférence entre 10 et 500 mg par kg.

Outre, les propriétés des HBGFPP rapportées précédemment, à savoir qu'ils présentent une activité cicatrisante et réparatrice sur les tissus musculaires, nerveux et sur ceux du tractus digestif et qu'ils peuvent être utilisés comme médicament pour le traitement des inflammations, les Inventeurs ont mis en évidence les effets remarquables des polymères de l'invention sur la régulation de l'homéostasie de la masse et de la fonctionnalité des tissus et des organes, en montrant qu'ils agissent sur la régénération, la protection, la préservation et le vieillissement tissulaire et cellulaire *in vivo* et *ex vivo,* ainsi que des activités antifibrotiques et protectrices contre les effets délétères des ischémies, des radiations ionisantes et des produits oxydants induits lors de pathologies ou de stress ou encore apportés dans l'alimentation. En conséquence, les RGTA doivent être considérés comme des régulateurs de l'homéostasie tissulaire en ce qu'ils régulent la masse des tissus régénérés et leur fonctionnalité et qu'ils agissent sur le remaniement matriciel en ce qu'ils exercent un effet antifibrotique et cicatriciel dans les tissus et organes soumis à des processus destructeurs aigus comme chroniques.

Ces propriétés des RGTA se caractérisent par des effets uniques sur la qualité et la vitesse des réparations tissulaires. Ces effets se traduisent par une reconstitution quasi complète et à l'identique de la structure tissulaire d'origine (avant lésion) et par l'absence presque totale de traces cicatricielles comme en particulier de signes de fibrose ou de perte de son intégrité fonctionnelle. Plus particulièrement, les travaux de recherche rapportés plus loin dans la partie expérimentale ont mis en évidence les propriétés de protection et de régénération tissulaire *in vivo* des RGTA dans les modèles suivants :

- lésion de plaies cutanées,
- régénération de tissus osseux comme les os longs avec l'exemple du fémur,
- protection contre la perte de tissu osseux et de régulation de son remaniement dans un modèle de parodontie chronique,
- protection contre les effets délétères des rayonnements ionisants,
- protection contre les effets délétères des ischémies quelque soit leur localisation.

Ainsi, les propriétés ci-dessous des polymères de l'invention ont plus particulièrement été mises en évidence :

- Un effet protecteur contre les effets délétères liés au stress oxydatif et aux agents oxydants. Les polymères de l'invention agissent en particulier comme des agents protecteurs et potentialisateurs d'enzymes comme la super oxyde dismutase ou SOD. Cette propriété permet d'envisager l'utilisation des polymères de l'invention pour la préparation d'un médicament destiné au traitement et/ou à la prévention des lésions et souffrances induites par un stress oxydatif et les agents oxydants. Mais cette propriété permet également d'utiliser les polymères de l'invention seul ou en association avec un autre composé comme agent de préservation d'aliments ou de nutriments contenant naturellement des antioxydants ou auxquels ont été ajoutés des agents antioxydants comme la SOD animale ou végétale.

- Un effet de préservation et de protection contre les ischémies. Cette propriété permet d'envisager l'utilisation des polymères de l'invention pour la préparation d'un médicament destiné au traitement et/ou à la prévention des pathologies associées à une hypoxie, à une dégénérescence cellulaire comme les neuropathies, les myopathies, les hépatopathies, les néphropathies, les cardiopathies, etc.. et aux peroxydations des molécules comme les lipides. Cette propriété permet également d'utiliser les polymères de l'invention dans des compositions de préservation de la fonctionnalité des tissus et organes plus particulièrement en vue de leur conservation, des transports d'organes ex vivo, des transplantations d'organes, de leurs greffes comme de celles de prothèses.

- Une activité inhibitrice des enzymes de la famille des calpaïnes. Cette propriété permet d'envisager l'utilisation des polymères de l'invention pour la préparation d'un médicament destiné au traitement et/ou à la prévention des maladies cardiaques et nerveuses.

- Une activité inhibitrice des enzymes de dégradation de l'héparine ou des héparanes sulfatés comme l'héparinase ou l'héparitinase. Cette propriété permet d'envisager l'utilisation des polymères de l'invention pour la préparation d'un médicament destiné au traitement et/ou à la prévention des maladies associées à une croissance anarchique des cellules et aux processus pathologiques en relation avec une angiogenèse.

- Une activité de protection des cellules contre les effets des radiations ionisantes et de régulation tant quantitative que qualitative des constituants de la matrice cellulaire comme par exemple les collagènes, permettant d'augmenter la survie et le fonctionnement des cellules. Cette activité permet d'envisager l'utilisation des polymères de l'invention pour la préparation d'un médicament destiné au traitement et/ou à la prévention des effets délétères des radiations ionisantes ainsi que l'utilisation de ces polymères dans la préparation de produit cosmétologiques.

- Une activité d'agent anti-fibrotique qui se manifeste *in vitro* et *in vivo* par des effets régulateurs sur la prolifération des cellules mésenchymateuses comme les cellules musculaires lisses, les fibroblastes ou les cellules hépatiques et la qualité du type de collagène qu'elles sécrètent, ainsi qu'une activité sur la qualité phénotypique des collagènes synthétisés par ces cellules et par la réduction notable des séquelles cicatricielles fibrotiques. Cette activité permet d'envisager l'utilisation des polymères de l'invention pour la préparation d'un médicament destiné au traitement et/ou à la prévention des pathologies pulmonaires rénales, hépatiques, cardiaques, vasculaires, dermatologiques, des greffes et de leur intégration fonctionnelle, des dérives multiples liées aux parasistismes.

Ainsi, d'une manière inattendue, les inventeurs ont découvert que les RGTA présentaient la capacité de:

- protéger, stabiliser les activités enzymatiques de la Super Oxyde Dismutase ou SOD.
- potentialiser les activités enzymatiques de la Super Oxyde Dismutase ou SOD

Cet enzyme peut donc être protégée soit *in situ* et *in vivo* soit *ex vivo*. Les RGTA peuvent donc être utilisés seuls comme agents protecteurs de la SOD endogène et à ce titre protéger celle ci dans toutes ses fonctions ou en association avec la SOD dans les indications connues de l'homme de l'art dans le domaine thérapeutique ou cosmétique. Ils agissent, par cette propriété, également comme préservateurs d'aliments ou de nutrients ou nutriments.

Les RGTA agissent donc comme des agents protecteurs et réparateurs des effets délétères associés au stress tissulaire. Ainsi, lors d'une ischémie quelqu'en soit l'origine, ou lors de la réponse à une agression cellulaire ou tissulaire par exemple sous l'effet de rayonnement ionisants ou en réponse à une invasion par un agent pathogène, de quelque nature qu'il soit viral, microbien, parasitaire ou même provoquant des pathologies de type ESST (Encéphalopathies spongiformes subaigue transmissible) comme celle provoquée par le prion, ou encore lors de rupture vasculaire provoquant des hémorragies dont les effets sont nocifs en provoquant des pertes fonctionnelles, par exemple dans le cas d'hémoragies des vaisseaux rétiniens qui pouvent conduire à la cécité ou dans le cerveau à des pertes de fonctions motrices ou autres.

En conséquence, l'invention concerne des compositions pharmaceutiques comprenant au moins un RGTA tant dans des domaines d'applications chez l'homme que chez l'animal. Ces compositions sont bénéfiques tant dans les domaines médicales, vétérinaires et cosmétologiques que dans les domaines alimentaires comme des agents de préservation d'aliments ou de nutriments contenant naturellement des antioxydants ou auxquels ont été ajoutés des agents antioxydants comme de la SOD animale ou végétale.

Les qualités protectices de la SOD endogène ou apporté d'une manière exogène sont renforcées par les RGTA. Ainsi,les RGTA peuvent être administrés dans tous les cas où l'effet bénéfique de la SOD a été décrit.

Pour le traitement de maladies dans lesquelles la SOD en apport exogène a été montrée comme efficace, l'effet thérapeutique résultant d'une action protectrice de la SOD endogène peut alors agir plus efficacement en présence de RGTA. Les RGTA limitent ou évitent des apports exogènes en agissant indirectement comme agents anti oxidants. Ils résultent de ces propriétés les effets thérapeutiques suivants :

- Une protection contre les agressions la souffrance ou la dégénérescence des tissus nerveux. Par exemple, dans le stress (Shahen et col., Effects of various stressors on the level of lipid peroxide antioxidants and Na+, K+ - ATPases activity in brain, Experentia, 1996, 52, 336-339) ou encore dans la dégénérescence neuronale et les maladies neurodégénératives associés aux accidents vasculaires, traumatiques, ou à des pathologies comme le Parkinson, dans lesquelles les activités de protection de la SOD endogène permettent une lutte plus efficace (Bostantjopoulos et col., Super oxide dismutase activity in early and advanced Parkinson's Disease, Funct. Neurol., 1997, 12, 63-68).

- Le vieillissement par un effet anti-apotose (Fernandez Novoa et coll., Methods Find Exper Clin Pharmacol, 1997, 9, 99-106).

- Une protection antioxydante dans les ischémies des membres. Dans cette application le RGTA peut être administré seul. La protection assurée par un traitement avec la SOD seule serait bien évidemment renforcé par l'administration de RGTA. Il apparait au vue des propriétés décrites que les RGTA seraient bénéfiques administrés seuls et/ou en association avec la SOD dans les nombreuses applications décrivant les effets de la SOD (D'Agnillo et Chang, Reduction of hydroxyl radical generation in a rat hindlimb model of ischemia - reperfusion injury using cross linked hemoglobin - superoxide dismutase - catalase, Artif. Cells Blood Subsiti Immobil Biotechnol, 1997, 25, 163-80).

- L'administration de RGTA seuls ou en association avec de la SOD sera bénéfique contre les souffrances du coeur, du cerveau, du système nerveux central comme dans le cas des lésions de la moelle épinière (Nakauchi et col., Effects of lecithinized super oxide dismutase on rat spinal cord injury, J. Neurotrauma, 1996, 13, 573-82) ou encore de la rétine.

- Dans le traitement des insuffisances respiratoires associées à la fatigue du diaphragme (Supinski et col., Effect of free radical scavengers on diaphragmeatic fatigue, Am. J. Respir. Crit. Care Med., 1997, 155, 622). Ceci pourrait-être notamment intéressant chez les malades atteints de dystrophie musculaires.

- Tous les tissus notamment ceux pour lesquels le taux de SOD active endogène est plus élevé (cellules endothéliales vasculaires, dans le foie, le rein, le muscle cardiac et squelettique, le pancréas, les cellules épithéliales des muqueuses de l'intestin, du colon, de la trachée, de l'oesophage, des tissus conjonctifs, du cartilage).

- Le traitement des effets délétères associés au diabète comme dans les rétinopathies diabétiques (Szabo et col., Direct measurement of free radicals in ischemic/reperfused diabetic rat retina, Clin. Neurosci., 1997, 4, 240-5).

- Le traitement des lépreux (Serum, zinc, copper, magnesium, proteins and super oxide dismutase in leprosy patients on multidrug therapy-a follow up study, Indian J. Lepr. 1996, 68, 325-33).

- Dans le traitement du choc endotoxique (Hepatic response to the oxydative stress induced by E. Coli endotoxin, Mol. Cell. Biochem. 1996, 159, 115-121).

- Dans le traitement des lésions induites par le stress causé par la présence d'agents pathogènes de toute nature, comme notamment les Virus dont le virus du sida ou les agents induisant des pathologies ESTT tels le prion.

- Dans le traitement préventif et/ou curatif contre les irradiations. Ainsi les effets adverses de la radiothérapie peuvent être réduits par un traitement préventif et/ou curatif de RGTA. L'utilisation des RGTA permet de réduire la dose de radiothérapie dans les conditions de traitement des cancers (Prevention of radioinduced cystisi by orgotein; a random ranomized study, Anticancer Res., 1996, 16, 2025-8) ou de prévenir les effets clastogéniques induits par l'irradiation. Les effets d'hyperthermie associés à la radiothérapie peuvent être diminués par l'utilisation de RGTA de la même manière que la SOD (Kandasamy et col., Involvement of superoxide dismutase and gluthatione peroxidase in attenuation of radiation-induced hyperthermia by interleukin 1 alpha in rats, Brain res., 1993, 606, 106-10).

- Dans la protection contre les effets induits par les rayons ultraviolets, par exemple sur la rétine. (Oguni et coll., Chronical retinal effects by ultraviolet irradiation with special référence to superoxide dismutase, Histol. Histopathol, 1996, 11, 695-702), et le traitement des Uvéïtes (Koch et coll., Effects of different antioxidants on lens-induced uveitis, Ger. J. Ophthalmol., 1996 5; 1185-8).Le traitement par le RGTA peut être envisagé seul ou en association avec la SOD et son utilisation peut être aussi bien médicale que cosmétologique.

- Dans le traitement de l'hypertension. En effet, l'activité de la SOD endogène est diminuée chez les sujets hypertendus (Jun Ke yan et Catalano, Increase superoxide anion production in humans, a possible mechanism for the pathogenesis of hypertension, J. Hum. Hypertens, 1996, 10, 305-309). Un apport de RGTA aura pour effet d'augmenter cette activité et de réduire l'hypertension.

- Dans le traitement des maladies inflammatoires comme l'arthrite.

Les RGTA sont utiles également pour la conservation des organes ou tissus ainsi que dans le maintien des fluides biologiques ce qui permet d'envisager les applications suivantes :

- Dans les fluides biologiques comme le sang et ses constituants cellulaires, par exemple en hémodialyse.
- Pour la préservation des organes, dans le cas de greffes ou de traitements *ex vivo* ou en reperfusion (Razak et col., Crosslinked hemoglobine-superoxide dismutase-catalase scavenges free radicals in a rat model of intestinal ischemia-reperfusion injury, Artif. cells Blood substiti immobil. Biotechnol. 1997, 25, 181-192). L'addition de RGTA à des solutions de conservation d'organes et administrés en reperfusion permet une préservation de ces organes.

L'invention concerne des compositions pharmaceutiques comprenant au moins un polymère défini précédemment et destiné au traitement et/ou à la prévention des lésions et souffrances tissulaires, telles que celles rencontrées en traumatologie, nécessitant des chirurgies réparatrices ou plastiques des planchers cutanés ou plus profonds comme ceux des muscles, des os, du cerveau, du coeur, des viscères, etc..., dans les maladies dégénératives associées ou non à des fibroses, dans les pertes de masse tissulaire comme l'ostéoporose ou les myopathies, dans les domaines cardio vasculaires, neurologiques, en dermatologie, etc....

Une propriété remarquable des RGTA est leur capacité à se fixer sur les tissus lésés, ce qui permet d'utiliser les RGTA comme vecteur de ciblage dans un objectif thérapeutique et/ou de diagnostic. En conséquence, les polymères de l'invention peuvent être couplés à des molécules, représentées précédemment par le groupement Z, douées d'activités thérapeutiques ou à des molécules permettant de faciliter le repérage du tissu lésé.

D'autres avantages et caractéristiques de l'invention apparaîtront des exemples qui suivent concernant d'une part la préparation de polymères de l'invention dont le squelette polymérique est de type polyester ou de nature polysaccharidique, et d'autre part, les propriétés des polymères de l'invention.

A) PRÉPARATION DES POLYMÈRES DE L'INVENTION.

EXEMPLE 1 : Synthèse de dérivés de poly (acide - $\beta$ - malique) à partir d'un squelette non polysaccharidique.

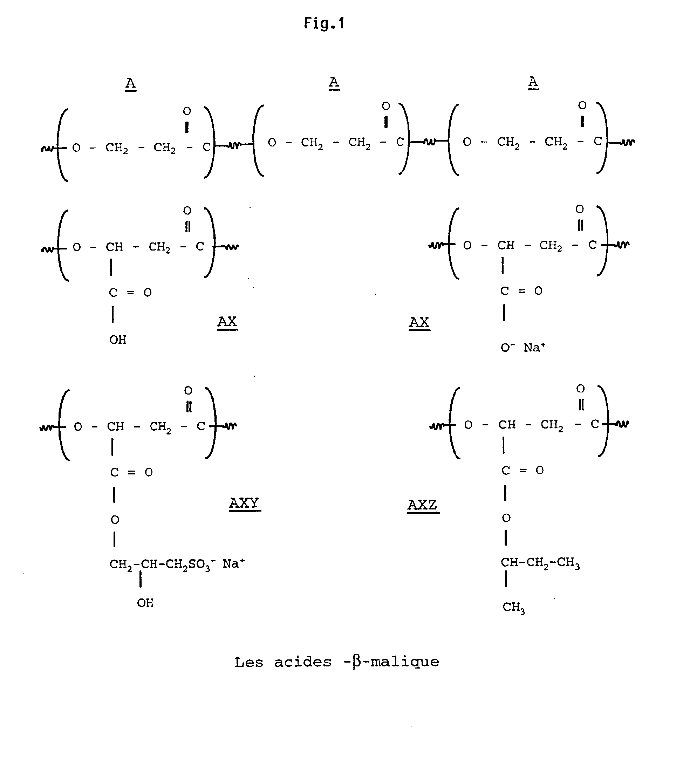

Dans cet exemple, le polymère est un copolymère d'acides - $\beta$ - malique de formule générale (II) dont les motifs A, substitués par X et/ou Y et/ou Z sont représentés à la figure 1. Dans la figure 1 :

A est -(O-CH$_2$-CH$_2$-CO)-
X est -COOH ou -COO-Na$^+$
Y est -CO-CH$_2$-CHOH-CH$_2$-SO$_3$H ou -CO-CH2-CHOH-CH$_2$-SO$^3$-Na$^+$
Z est -CO-OCH$_3$-CH(CH$_2$-CH$_3$)-CH$_3$

x, y et z, correspondant aux pourcentages des groupements X, Y et Z, indiqués dans le tableau I ci-dessous en fonction des différents polymères synthétisés.

Tableau I

| Référence | Type de polymère | Groupements carboxyliques = X | Groupements Soufrés = Y | Groupements hydrophobes = Z |
|---|---|---|---|---|
| RGTA 2010 | Pcoo- | 100% | 0% | 0% |
| RGTA 2011 | P1S | 60% | 10% | 10% |
| RGTA 2012 | P2S | 75% | 11% | 12% |

Pcoo$^-$ correspond à un polymère ne comportant exclusivement que des groupements X carboxyliques ou carboxylates.

PIS et P2S correspondent à des polymères comportant en plus des groupements X, des groupements Y sulfonatés et des groupements hydrophobes butyles Z .

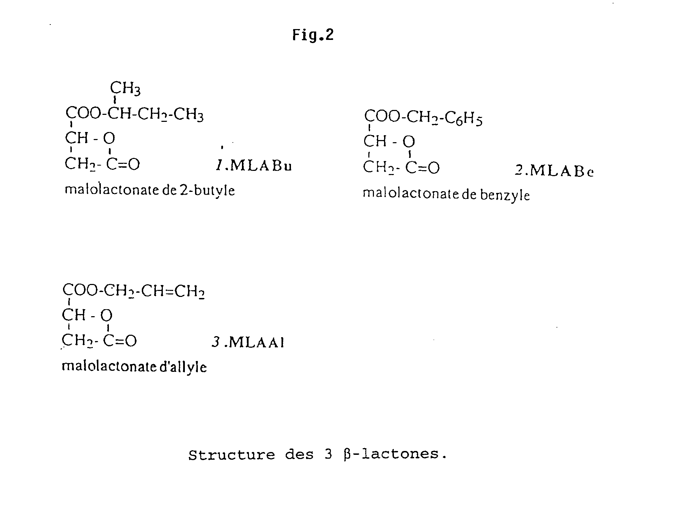

La synthèse de ce polymère passe par la préparation de $\beta$-lactones $\beta$-substituées et est suivie d'une polymérisation anionique par ouverture de cycle. Les trois $\beta$-lactone synthétisées sont représentées à la figure 2 en annexe.

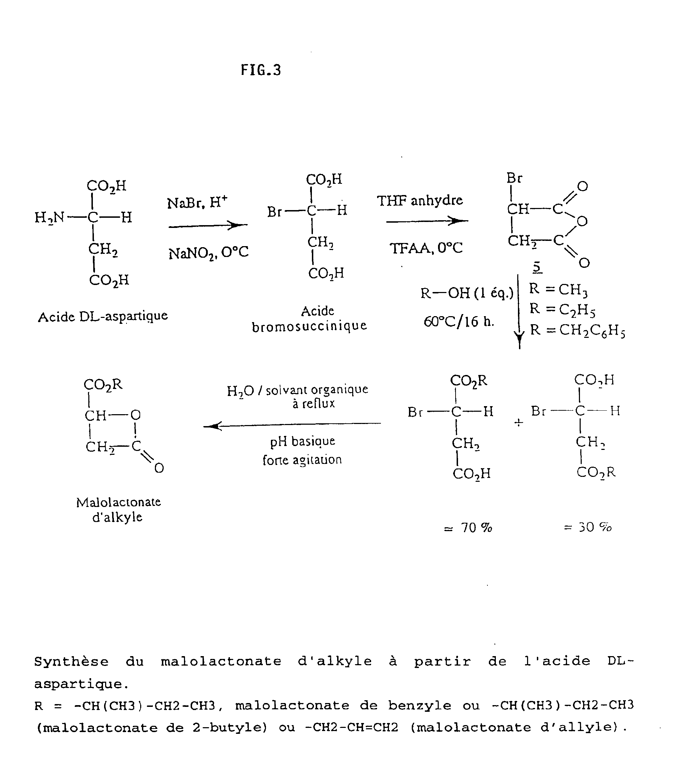

Les monomères sont obtenus à partir de l'acide DL-aspartique en quatre étapes avant la polymérisation comme montré à la figure 3 en annexe.

La synthèse du malolactonate d'alkyle se fait à partir de l'acide DL-aspartique où R représente -CH$_2$-C$_6$H$_5$ (malolactonate de benzyle) ou -CH(CH$_3$)-CH$_2$-CH$_3$ (malolactonate de 2-butyle) ou encore -CH$_2$-CH=CH$_2$ (malolactonate d'allyle).

I - <u>Synthèse des monomères (voies de l'acide aspartique).</u>

1) <u>Synthèse de l'acide (2R,S) 2-bromosuccinique.</u>

La synthèse de l'acide (2R,S) bromosuccinique est obtenue après une réaction de diazotation de l'acide DL-aspartique. Pour cette réaction, le carbone portant le groupement amine subit une double inversion de configuration (Guérin et coll., Optically active poly(β-malic acid), 1985, Polymer Bulletin, 14: 187).

100 g (0,75 moles) d'acide DL-aspartique et 415 g (4,04 moles; 5,5 éq.) de bromure de sodium sont dissous dans 1620 millilitres d'acide sulfurique 2N dans un bain de glace. 62 g (0,9 moles; 1,2 éq.) de nitrite de sodium sont additionnés par petites quantités, sous agitation. 30 minutes après la fin de cette addition, le milieu réactionnel est neutralisé par 8 g (0,13 moles; 0,18 éq.) d'urée pendant 30 minutes à température ambiante. L'acide bromosuccinique est extrait par 1 litre d'acétate d'éthyle et la phase aqueuse est lavée avec 1 litre d'acétate d'éthyle. Les phases organiques sont séchées sur sulfate de magnésium, filtrées sur büchner puis l'acétate d'éthyle est éliminé au rotavapor.

L'acide bromosuccinique est alors recristallisé 4 fois dans l'acétonitrile, filtré sur büchner puis séché 4 h à 45°C sous vide dans un dessiccateur.

Caractéristiques : PM = 197; m = 52,8 g; Rendement de 36%; Tf=168°C; Aspect : poudre blanche

2) <u>Synthèse des monoesters.</u>

La synthèse des monoesters passe par la préparation préalable de l'anhydride sans racémisation des centres chiraux. L'anhydride est synthétisé à partir de l'acide (2R,S) 2-bromosuccinique sous l'action d'un agent déshydratant, l'anhydride trifluoroacétique (TFAA) en conditions anhydres et sous azote. L'étape suivante est l'ouverture de l'anhydride par un alcool qui conduit à 2 monoesters dont un seul sera lactonisable. Le choix de l'alcool est fait selon la nature de la lactone souhaitée.

a) <u>Synthèse du bromosuccinate de benzyle.</u>

50 g (0,25 moles) d'acide bromosuccinique sont dégazés sous courant d'azote pendant 2 h. Dans un bain de glace, 125 millilitres de tétrahydrofurane (THF) anhydre puis 43,4 millilitres (0,30 moles; 1,2 éq.) d'anhydride trifluoroacétique (TFAA) sont ajoutés goutte à goutte par une ampoule à brome. La solution est laissée 2 h à température ambiante sous agitation, puis le THF, le TFA formé et le TFAA en excès sont éliminés au rotavapor. L'anhydride bromosuccinique obtenu est mis à dégazer 2 h.

27,7 millilitres (0,25 moles; 1 éq.) d'alcool benzylique sont ensuite ajoutés. La solution est agitée à 40°C sous atmosphère inerte pendant 12 h. Le mélange de monoesters ainsi obtenus est dissout dans 150 millilitres d'éther. La phase éthérée est lavée 3 fois avec 100 millilitres d'eau, séchée sur sulfate de magnésium et filtrée sur büchner. Caractéristiques : PM = 287; m = 71,3 g; Rendement de 95%; Aspect : huile jaune pâle

b) <u>Synthèse du bromosuccinate d'allyle.</u>

Le même protocole que précédemment est mis en oeuvre, mais 17,86 millilitres (0,25 moles; 1 éq.) d'alcool allylique sont ajoutés. Le milieu réactionnel est agité 22 h à 60°C sous atmosphère inerte au lieu de 12 h à 40°C. Les monoesters sont purifiés de façon identique.

Caractéristiques : PM = 237; m = 17,6 g; Rendement de 72,8%; Aspect : huile visqueuse

c) <u>Synthèse du bromosuccinate de 2-butyle.</u>

Même protocole que ci-dessus en 2)b) mais avec 18,8 g (1 éq.) de (RS)-2-butanol préalablement distillé. Le milieu réactionnel est agité pendant 12 heures à 60°C sous atmosphère inerte.

Caractéristiques : PM = 253; m = 45 g; Rendement de 90%; Aspect : huile jaune orangée

3) <u>Synthèse des lactones.</u>

La réaction de lactonisation a lieu sur le seul monoester lactonisable et présente une inversion de configuration.

Elle est effectuée directement sur le mélange de monoesters après neutralisation à pH 7,2 par de la soude 2N. La réaction a lieu dans un milieu biphasique dichlorométhane/eau. La lactone est purifiée par chromatographie sur colonne de silice. La nature de l'éluant est différent selon la nature de la lactone. Cette lactone est ensuite distillée sur une colonne appropriée.

a) Synthèse du malolactonate de benzoyle.

71 g (dont 70% de monoester lactonisable soit 0,173 moles) de mélange de monoesters sont dissous dans 300 millilitres d'éther et 250 millilitres d'eau dans un ballon. Une solution d'hydroxyde de sodium 2N est ajoutée goutte à goutte jusqu'à pH 7,2, puis 450 millilitres de dichlorométhane sont additionnés. Le ballon est surmonté d'un système réfrigérant et le système biphasique est fortement agité pendant 3 h à 40°C.

Après décantation, la phase organique est lavée 2 fois avec 250 millilitres d'eau puis 2 fois avec 250 millilitres de saumure, séchée sur sulfate de magnésium et filtrée. Le solvant est éliminé au rotavapor. Cette lactone est purifiée sur colonne de silice (éluant : dichlorométhane/éther de pétrole 8/2) et distillée 3 fois sous vide.

Caractéristiques : PM=206; m=20,7 g avant purification soit un rdt =40,5%; m=3,41 g après purification soit un rdt=6,7%; Te=116-118°C sous $3.10^{-2}$ mbars; IR (n, cm$^{-1}$): $n_{(CO lactone)}$ =1825cm$^{-1}$; $n_{(CO ester)}$ =1740cm$^{-1}$; Aspect : huile incolore.

b) Synthèse du malolactonate d'allyle.

Selon le même protocole que ci-dessus en 3)a) mais le pH de la phase aqueuse est de 7,8 au lieu de 7,2 et la solution est agitée 5 h au lieu de 3 h. La lactone est purifiée sur colonne de silice (éluant : éther de pétrole/éther éthylique 4/6) et est distillée 3 fois sous vide.

Caractéristiques : PM = 156 m = 15,3 g avant purification soit un rendement de 53,4%; m=4 g après purification soit un rdt=13%; Te = 62-65°C sous $3.10^{-2}$ mbars; IR (n, cm$^{-1}$): $n_{(CO lactone)}$ =1825cm $n_{(CO ester)}$ = 1740cm$^{-1}$; Aspect : liquide incolore.

c) Synthèse du malolactonate de 2-butyle.

Selon le même protocole que ci-dessus en 3)a). La lactone est purifiée sur colonne de silice (éluant : dichlorométhane/éther de pétrole 8/2) et est distillée 3 fois sous vide.

Caractéristiques : PM = 172; m = 26,7 g avant purification soit un Rendement de 55%; m = 14,4 g après purification soit un Rendement de 28%; Te = 80-82°C sous 3.10-2 mbars; IR(n, cm$^{-1}$): n(CO lactone)=1825cm$^{-1}$; n(CO ester)= 1740cm$^{-1}$; Aspect : huile visqueuse incolore.

II - Synthèse des polymères.

Les lactones ont été polymérisées en présence de benzoate de tétraméthyl ammonium (10-3 éq.) à 37°C pendant 15 jours. Le suivi de la polymérisation a été fait par analyse infrarouge en observant la disparition de la bande lactonique à 1850 cm$^{-1}$.

1) Synthèse du poly(malate de benzyle-co-malate de butyle-co-malate d'allyle).

5 g (24,2 mmoles) de malolactonate de benzyle, 1,4 g (9,3 mmoles) de malolactonate d'allyle et 0,7 g (4,1 mmoles) de malolactonate de butyle sont dégazés pendant 2 h et transférés par canule dans un ballon contenant 471 millilitres d'une solution d'amorceur (benzoate de tétraéthylammonium:10$^{-3}$ éq.; 37,72.10$^{-6}$ moles) à 80.10$^{-3}$ M, préalablement dégazée 2 h sous courant d'azote. La copolymérisation est conduite à 37°C pendant 15 jours sous atmosphère inerte et sous agitation. Le polymère est alors dissous dans un minimum de chloroforme. Les chaînes sont terminées par addition d'une goutte d'acide chlorhydrique concentré et le polymère est précipité à l'éthanol puis séché sous vide à 40°C pendant 48h.

Caractéristiques : m = 4,37 g ; Rendement de 61,5%; Tv = -5°C; IR (n, cm-1): n (C=O)=1748 cm-1; SEC (THF, standards polystyrène):; Mn = 6600; Mw = 9200; Ip = 1,4; MSEC = 10000; S 134 (CH=); 168 (C=O chaîne latérale); 170 C=O chaîne principale); Aspect : polymère vitreux transparent

2) Epoxydation du poly(malate de benzyle-co-malate de butyle-co-malate d'allyle).

1,12 g (7,91 mmoles d'unités insaturées) de polymère sont dissous dans 3 millilitres de dichlorométhane anhydre dans un ballon. Une solution contenant 466,34 milligrammes (4,69 mmoles; 6 éq.) d'acide métachloroperbenzoïque (MCPBA) dans 2 millilitres de dichlorométhane est ajoutée par canule. Le mélange est agité 24 heures à température ambiante, puis est filtré. Le polymère est alors précipité à l'éthanol et séché dans un dessicateur sous vide.

Caractéristiques : m = 4 g ; Rendement de précipitation de 92%; Rendement de la réaction d'époxydation = 100%;

La masse molaire ne varie pas lors de l'époxydation car le MCPBA n'induit pas une modification de la longueur de la chaîne.

3) Hydrocrénolyse du poly(malate de benzyle-co-malate de butyle-co-malate d'allyle).

4 g du polymère sont dissous dans 5 millilitres de dioxanne fraîchement distillé sur sodium dans un ballon. 800 milligrammes (20% en poids) de palladium sur charbon actif sont ajoutés et l'hydrogénolyse est mise en route. Quand le volume d'hydrogène consommé n'augmente plus (24 h après le début de la réaction), l'hydrogénolyse est arrêtée. La solution est filtrée sur célite et le dioxanne est éliminé au rotavapor.

Caractéristiques . m = 2 g; Rendement d'hydrogénolyse de 100%;

4) Sulfonation du poly(malate de benzyle-co-malate de butyle-co-malate d'allyle).

4 g de polymère (soit $5,64.10^{-3}$ moles d'époxyde) sont dissous dans 20 millilitres d'eau. 2,14 g de bisulfite de sodium (2 éq., $11,28.10^{-3}$ moles) sont ajoutés. Le pH de la solution est ajusté à 7,4 (Housse-Ferrari, "Préparation et caractérisation de silices poreuses modifiées par des polymères et des copolymères de N,N' diméthylacrymide", Thèse de l'université Paris VI, 30 janvier 1990). La solution est laissée sous agitation pendant 7 h dans la glace puis est ultrafiltrée 24 h à 4°C contre de l'eau et lyophilisée.

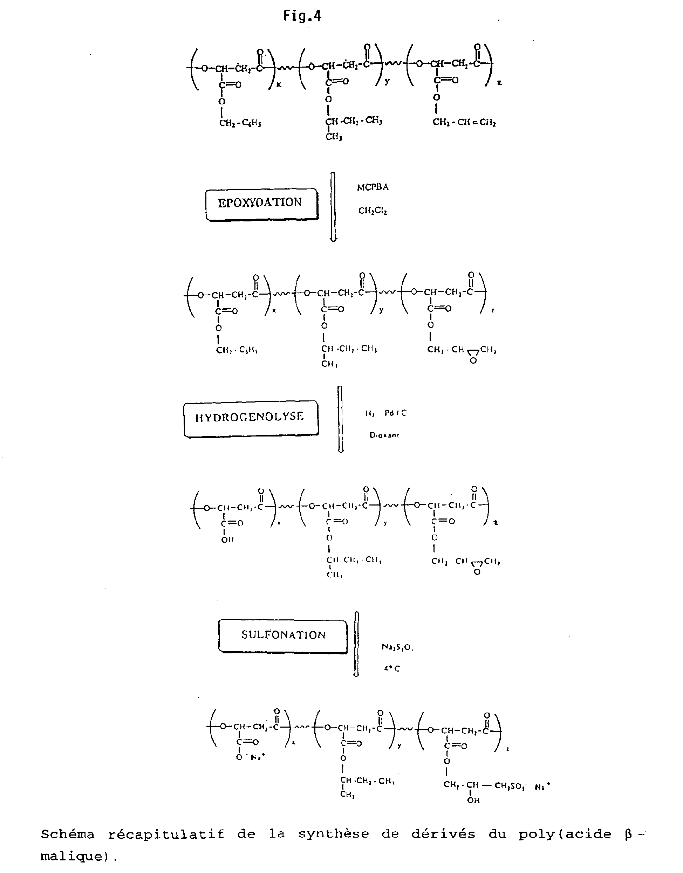

La figure 4 en annexe récapitule les étapes de synthèse des dérivés du poly(acide - $\beta$ - malique).

EXEMPLE 2 : Synthèse de polymères polysaccharidiaues constitués de motifs de glucose substitués.

I - Synthèse de Carboxyméthylesulfates de Dextran désignés CMDS.

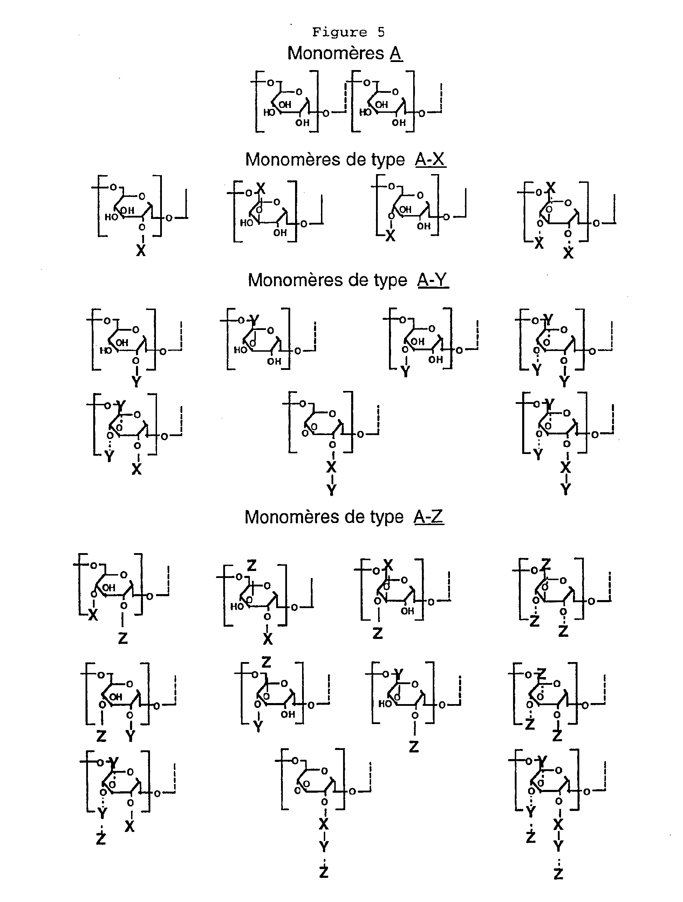

Dans cet exemple, le polymère de l'invention est constitué de dextrane substitué dont les motifs de glucose A substitués par X et/ou Y et où Z est rien sont représentés dans la figure 5 en annexe. Les différents types de greffage sont représentés à la figure 5, dans laquelle :

A est un monomère de glucose sur lequel les X, Y et Z sont greffés par l'intermédiaire des fonctions hydroxyles en position 2 et/ou position 3 et/ou position 4 et/ou par l'intermédiaire des groupements X pour Y et/ou Z, et/ou par l'intermédiaire des groupements Y pour Z,

X est -$CH_2COOH$ ou -$CH_2COO$-$Na^+$

Y est -$SO_3H$ ou $SO_3$-$Na^+$

Z est un groupement variable dont quelques exemples sont rapportés plus loin.

Les polymères de type CMDS dans lesquels Z = rien contiennent plusieurs types de monomères. Les premiers types de monomères substitués sont des carboxyméthyl glucose de type A-X substitués en position 2 et/ou 3 et/ou 4 (motifs présentés figure 5). L'addition du groupement Y = (motifs A-Y représentés à la figure 5) correspond à une O-sulfatation et devient, avec R = rien et R'= $H^+$ ou $Na^+$- Y = O-$SO_3^-$ $H^+/Na^+$. Si Y est fixé sur X, R de Y devient $CH_2$-CO et la fonctionnalité de X ($COO^-$) perdue, n'est plus considérée comme existant. Elle devient alors partie de Y et rentre dans la mesure des pourcentages de substitution des groupements actifs Y.

Les différents monomères constituants le polymère CMDS sont donc soit du glucose non substitué, soit du carboxyméthyl glucose soit du glucose sulfate soit du carboxyméthyl glucose sulfate. Les différentes formes isomériques sont schématisées figure 5. Les polymères corespondent donc à toutes les combinaisons possibles de ces différentes formes monomériques et se définissent par les taux résiduels de groupements X et Y libres.

Les monomères ainsi obtenus sont soit du glucose sulfate en position 2, 3 et/ou 4 et/ou du carboxyméthyl glucose sulfate. Le sulfate est fixé soit sur le glucose soit sur le groupe carboxylique.

Ainsi, dans la figure 5, dans laquelle les liaisons des groupements schématisés par un pointillé représentent des monomères où toutes les combinaisons circulaires peuvent être envisagées.

1) Synthèse de dextrane carboxyméthyl sulfate désigné $Cm_nDS_m$.

a) Carboxyméthylation.

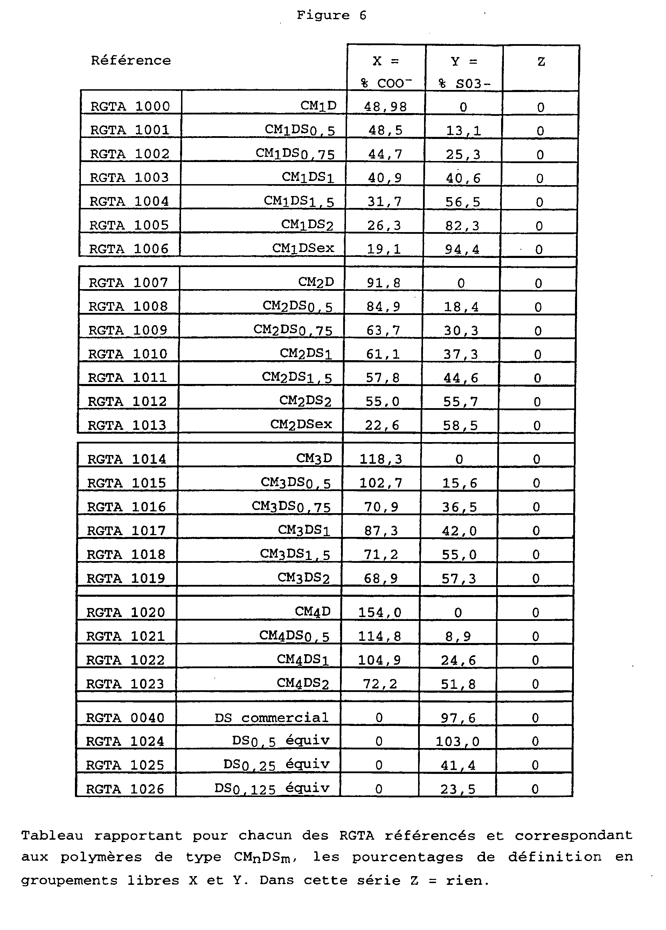

La première étape de carboxyméthylation du dextran est réalisée selon le protocole décrit dans Mauzac et collaborateurs (Mauzac et coll., Anticoagulant activities of dextran derivatives Part I : Synthesis and characterization, 1984, Biomaterials 6/61-63). Il s'agit d'une étherification des fonctions hydroxyles des résidus glucose du dextran en vue de l'obtention d'un carboxyméthyldextran. Cette réaction peut être reproduite plusieurs fois et conduit à des produits dénommés $CM_nD$ où n représente le nombre d'étapes de carboxyméthylation. Les différents produits appelés $CM_nD$ se caractérisent par un % de COOH croissant. Ce procédé permet donc d'obtenir différents taux de carboxyméthylation du dextrane comme indiqué dans le tableau de la figure 6.

Ainsi, dans un ballon réfrigéré de 250 millilitres, du Dextran T40 (37,37 g, 9,34 $10^{-4}$ mol), provenant de la société Sigma et de poids moléculaire 40 000 D, est solubilisé (182 millilitres d'eau distillée) à 4°C. Une solution de soude (74 g, 1,85 mol dans 124 millilitres d'eau distillée), également refroidie à 4°C, est versée lentement dans la solution de Dextran tout en maintenant la température de 4°C constante. De l'acide monochloroacétique (76,2 g, 0,806 mol) réduit en fine poudre, est additionné lentement en maintenant la même température de réaction. Cependant la température du milieu s'élève en fin de réaction de 4°C à 21°C en quelques minutes. Le mélange est ensuite amené à 50°C dans un bain d'huile thermostaté pendant 40 mn. Pendant le chauffage, le milieu réactionnel acquiert une coloration jaune. Après refroidissement, le pH est neutralisé à 7,2 avec de l'acide acétique glacial. (Takakura, 1990). Le carboxyméthyldextran est recueilli par précipitation dans de l'éthanol absolu froid (5 à 6 fois le volume réactionnel). Il est, ensuite, séché dans une étuve à vide. Le polymère $CM_1D$ est purifié par ultrafiltration puis lyophilisé (masse = 56 g brut).

La présence des ions carboxyliques est quantifiée par dosage acido-basique en retour avec l'utilisation de l'acide nitrique. La base utilisée est la soude 1 N. Le résultat s'exprime en % de groupements COOH. % COOH du $CM_1D$ = 48,98 %

Ce pourcentage signifie que statistiquement environ une unité glucose sur deux a été carboxyméthylée.

En pratique, cette réaction peut être réalisée plusieurs fois pour atteindre les taux de substitution souhaités. Ainsi, le même protocole a été appliqué pour la synthèse d'un $CM_2D$ à partir du $CM_1D$ et d'un $CM_3D$ à partir du $CM_2D$ : % COOH du $CM_2D$ = 91,8 % et % COOH du $CM_3D$ = 118,3 %.

b) Sulfatation.

La réaction de sulfatation des fonctions hydroxyles résiduelles après les étapes de carboxyméthylation est effectuée avec de l'acide chlorosulfonique. Elle aboutit à des composés dénommés $CM_nDS_m$ donnés dans le tableau de la figure 6 en annexe, où m correspond à des équivalents d'acide chlorosulfonique tels que définis dans l'exemple ci-dessous.

Exemple de sulfatation du $CM_1D$ :

500 milligrammes (PM=7,8 mmol/g) du carboxyméthyl$_1$dextran ($CM_1D$) sont dispersés dans 40 millilitres de dichlorométhane sec). Le nombre de résidus hydroxyles restés libres et susceptibles de réagir dans une réaction de sulfatation est de nOH = $4.10^{-3}$ mole. La réaction a été réalisée en présence d'un équivalent d'acide chlorosulfonique (nClSO3H = 4. $10^{-3}$ mole) soit approximativement = 0,5g ou un volume de 0,3 millilitres, la densité de la solution d'acide chlorosulfonique étant de 1,75). Les 0,3 millilitres d'acide chlorosulfonique sont dilués dans 4 millilitres de dichlorométhane déshydraté. Le $CM_1DS_1$ ainsi obtenu est récupéré par filtration du milieu réactionnel sous vide sur fritté.

La même réaction peut être réalisée en présence de 0,5 ou 1,5 ou 2 ou 3 équivalents d'acide chlorosulfonique ou d'un excès de façon à greffer des quantités croissantes de groupements O-sulfates. Les polymères RGTA ainsi obtenus sont appelés $CM_nDS_m$, avec n = 1, 2, etc... et m = 0.5, 1, 2, etc....

Sur le même principe et en vue de comparer ces polymères à d'autres molécules sulfatées, nous avons considéré comme produits de comparaison avec les $CM_nDS_m$ soit des dextranes sulfatés du commerce (produit Pharmacia Biotech, code 17-0270-01), soit des dextranes sulfatés à partir de dextran $T_{40}$ en présence de m équivalents d'acide chlorosulfonique, c'est à dire dans des conditions réactionnelles comparables à celles permettant d'obtenir les $CM_nDS_m$.

Les résultats de différents dosages des groupements X par titration et Y par analyse élémentaire du taux d'atome de soufre permettent de préciser les valeurs correspondantes x et y pour chacun des composés synthétisés $CM_nDS_m$. L'ensemble de ces données est rapporté dans le tableau de la figure 6 en annexe. Cette figure précise par ailleurs ce pourcentage pour du sulfate de dextran commercial et pour les dextranes sulfatés dans les mêmes conditions que les $CM_nDS_m$.

2) Synthèse des polymères de dextran carboxyméthyle-phényl sulfonate noté $CM_2DPhS$ et d'un dérivé Dextran carboxyméthyl sulfate phényl sulfonate désigné CM2DPhSS.

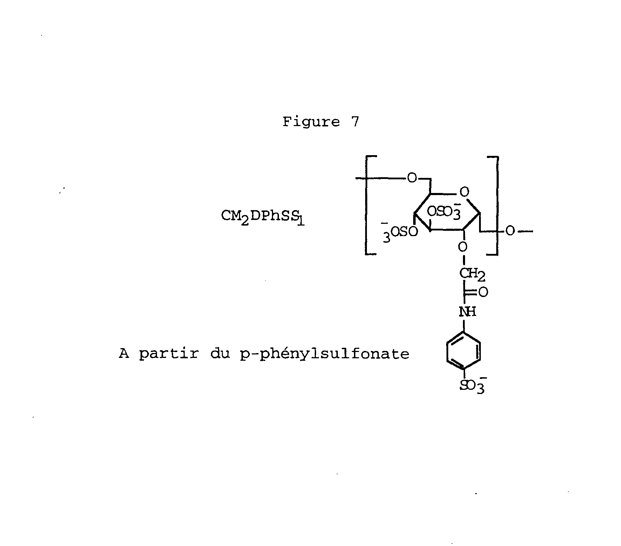

Ces polymères sont constitués d'un enchaînement de motifs représentés à la figure 7 en annexe, et correspondent à ceux de la figure 4 ou Z n'était rien. Ces polymères sont constitués d'un enchaînement de motifs de type A-X, A-Y et A-Z tels que représentés à la figure 6.

Dans cet exemple, le groupement Z est du phényl sulfonate noté PhS. La figure 7 représente un monomère A-Z dans le cas où Z a été additionné sur un radical carboxyl greffé en position 2 du glucose d'origine, lui même sulfaté en position 3 et 4.

Le polymère $CM_2D$ (2 g; 3,90 mmol) est dissous dans 13 millilitres d'eau distillée. Le pH de la solution est ajusté à 3,5 avec de l'HCl 3 M. L'agent couplant EEDQ (1,93 g, 2 équi.) est dissous dans 16 millilitres d'éthanol (0,12 g d'EEDQ/ millilitre) à 40°C. La solution d'EEDQ est ajoutée progressivement au polymère et le mélange réactionnel est agité fortement pendant 30 min. Le sel d'acide phénylsulfanilique (NH2PhSO3Na, 3,045 g, 2équi) est ensuite ajouté par petites quantités. Le pH de la réaction est ajusté à 9 avec de la soude. Le mélange est agité à température ambiante pendant 4 heures, puis neutralisé avec HCl dilué. Le produit $CM_2DPhS$ est alors ultrafiltré, évaporé sous vide puis lyophilisé (1,66 g).

Les analyses élémentaires et acide-bas du CM2DPhS donnent : % C = 33,9; % H = 5,28; % N = 0,3; % S = 0,67; % COOH = 81 %

Les analyses élémentaires et titrimétriques acide-base du polymère $CM_2DPhSS*_1$ donnent % C = 23,16; % H = 3,72; % N = 0,27; % S = 9,60; % COOH = 52 %

## 3) Synthèse d'un dérivé Carboxymethyl Dextran N et O Sulfaté.

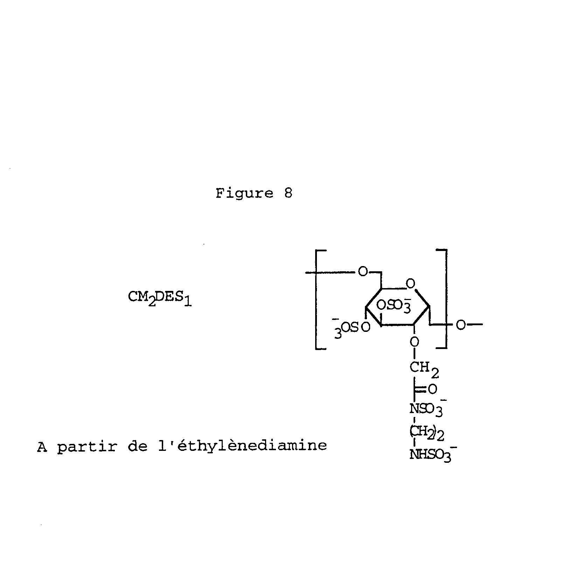

Ces polymères sont constitués d'un enchaînement de motifs de type A-X, A-Y et A-Z tels que représentés à la figure 6, dans lesquels un des motifs caractéristiques A-Z est présenté à la figure 8 en annexe. Dans cet exemple le groupement Z qui est de l'ethylènediamine noté DE est greffé sur le radical carboxyl du carbone 2 du monomère glucose d'origine, lui même sulfaté en position 3 et 4.

Le même protocole que précédemment a été appliqué sur le $CM_2D$ avec dans un premier temps, l'addition d'un groupement Z d'éthylènediamine noté DE pour aboutir au produit désigné $CM_2DE$.

Les analyses élémentaires et titrimétriques acide-base du polymère $CM_2DE$ donnent : % C = 37,67; % H = 6,25; % N =5,35; % COOH = 19 %.

Le protocole de sulfatation a été réalisé sur une fraction de ce polymère en utilisant 1 équivalent d'acide chlorosulfonique. Les produits obtenus correspondent à du $CM_2DES_1$ représenté à la figure 7 en annexe. Dans ce cas, selon la formule générale des RGTA, Z est du diethylamine.

Les analyses élémentaires et titrimétriques acide-base du polymère $CM_2DDES_1$ donnent : % C = 36,83; % H = 5,82; % N = 4,67; % S = 1,82; % COOH = 10,7 %

Ces synthèses permettent de greffer des groupements N-sulfate en plus des groupements O-sulfate des CMDS. Ces différents polymères permettent d'apprécier les rôles spécifiques des groupements N-sulfate et O-sulfate des $CM_2DES$ par rapport aux groupements phényl sulfonate notés PhS des composés $CM_2DPhS$, aux composés sulfatés et sulfonatés des $CM_2DPhSS$ ou aux composés. Dans ce type de polymères, il apparaît que les groupements sulfates sont essentiellement liés aux propriétés des RGTA.

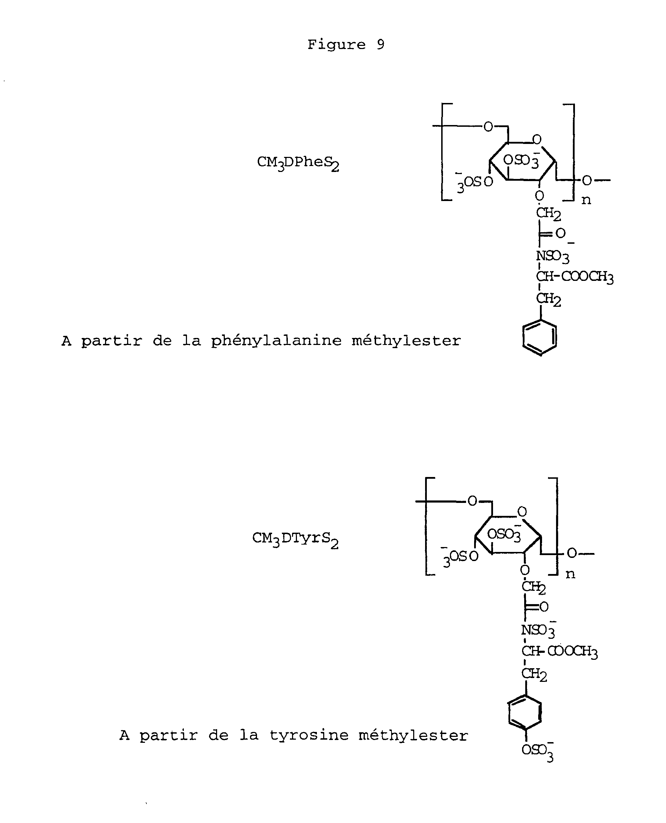

## 4) Synthèse des polymères de dextran carboxyméthyle-phénylalanine sulfate désigné $CM_3DPheS$ et de dextran carboxvméthyle tyrosine sulfate désigné $CM_3DTyrS$ représentés à la figure 9 en annexe.

Ces polymères sont constitués d'un enchaînement de motifs de type A-X, A-Y et A-Z tels que représentés à la figure 6 dans lesquels les motifs caractéristiques sont représentés à la figure 9 en annexe. Dans cet exemple, les groupements Z sont respectivement soit de la phénylalanine pour Phe soit de la tyrosine pour Tyr. Ils ont été additionnés sur un radical carboxyl greffé en position 2 du glucose d'origine lui même sulfaté en position 3 et 4.

Dans ces polymères, Z est un acide aminé avec soit de la phénylalanine (Phe) ou de la tyrosine (Tyr) et Y est un groupe -OSO3⁻.

Ces synthèses ont été réalisées pour apprécier l'importance des structures aromatiques de ces deux acides aminés Phe et Tyr en remplacement de la benzylamine notée B telle que connue dans le CMDBS de l'art antérieur, mais dont le relarguage dans les applications *in vivo* peut être préjudiciable en générant des risques de tumorigénicité.

Le polymère $CM_3D$ (% COOH = 136 %, 0,6 g, 3,014 mmol) est dissous dans 6 millilitres d'eau distillée. Le pH de la solution est ajusté à 3,5 avec HCl 3M. l'agent couplant EEDQ (745 milligrammes, 1 équi.), est dissous dans 6,2 millilitres d'éthanol à 40°C. La solution d'EEDQ est ajoutée au polymère goutte à goutte et le mélange réactionnel est agité vigoureusement pendant 30 min. à température ambiante. La phénylalanine méthylester (1,3 g, 2 équi.) est ajouté très lentement au mélange et le pH est ramené de 5 à 9 avec de la soude. La réaction est maintenue à température ambiante pendant 4 heures. La solution est ensuite neutralisée avec HCl dilué. Le polymère $CM_3DPhe$ est alors ultrafiltré, évaporé sous vide puis lyophilisé (524 milligrammes).

Les analyses élémentaires et titrimétriques acide-base du polymère $CM_3DPhe$ donnent : % C = 36,9; % H - 5,03; % N = 0,44; % COOH = 101,05 %.

Le même protocole a été réalisé sur le $CM_3D$ avec la tyrosine méthylester $CM_3DTyr$.

Les analyses élémentaires et titrimétriques acide-base du polymère CM$_3$DTyr donnent : % C = 34,41; % H = 5,12; % N = 0,28; % COOH = 101,76 %.

Le protocole de sulfatation a été réalisé sur ces deux polymères en utilisant 2 équivalents d'acide chlorosulfonique.

Les analyses élémentaires et titrimétriques acide-base du polymère CM$_3$DPheS*$_2$ donnent : % C = 18,08; % H = 2,89; % N = 0,30; % S = 14,71; % COOH = 28,79 %.

Les analyses élémentaires et titrimétriques acide-base du polymère CM$_3$DTyrS$_2$ donnent : % C = 17,99; % H = 3,13; % N = 0,3; % S = 14,35; % COOH = 19,85 %.

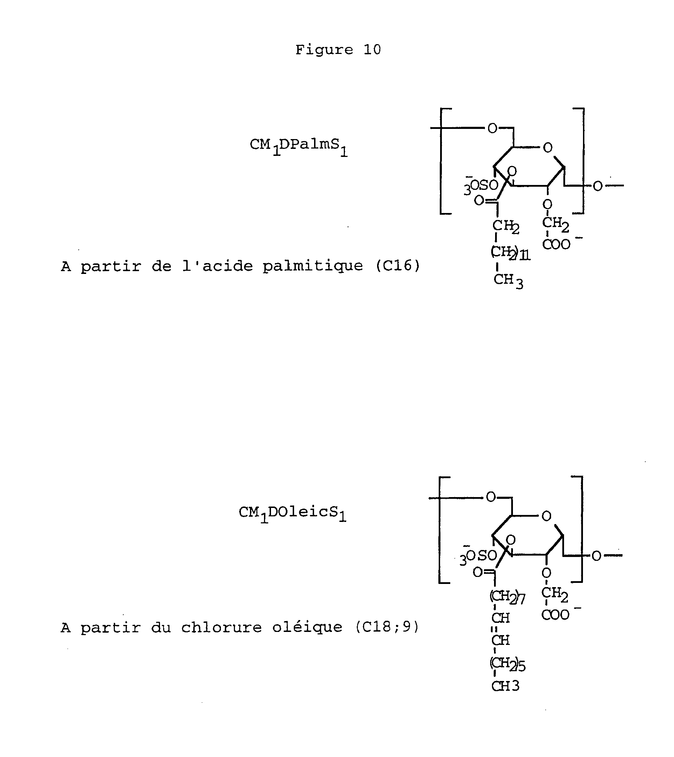

5) Synthèse des polymères lipidiques : exemple avec le dextran carboxyméthyle - acide oléique Sulfaté (CM$_1$DoléicS) et le dextran carboxyméthyle - acide palmitique Sulfaté (CM$_1$DpalmS) représentés à la figure 10 en annexe.

Ces polymères sont constitués d'un enchaînement de motifs de type A-X, A-Y et A-Z tels que représentés à la figure 6 dans lesquels les motifs caractéristiques sont représentés à la figure 10 en annexe. Dans cet exemple, les groupements Z qui sont respectivement soit de l'acide oléique pour oléic soit de l'acide palmitique pour palm ont été additionnés sur l'hydroxyle du carbone 3 d'un carboxyméthyl glucose en position 2 sulfaté en position 4.

Ces composés répondent à la formule (I) dans laquelle Y = -OSO3$^-$ et Z est de l'acide oléique ou palmitique. Ces acides gras ont été greffés pour apprécier l'importance de la balance hydrophobicité / hydrophilie des polymères en plus du rôle propre de ces acides gras.

Au polymère CM$_1$D (1 g, 2,43 mmol) dissous dans du DMSO (16 millilitres) est ajouté de la triéthylamine (0,8 millilitres) et goutte à goutte du chlorure oléique (1,6 millilitres). Le mélange réactionnel est agité à température ambiante pendant 2 heures. Le polymère est précipité dans 120 millilitres d'acétate d'éthyle, centrifugé puis séché sous vide. Le précipité est dissous dans 20 millilitres d'acétate de sodium 2M et le sel formé est précipité dans l'éthanol (160 millilitres), filtré, dissous dans 20 millilitres d'eau distillée puis enfin dialysé contre l'eau. Après dialyse (24 h.), la solution évaporée sous vide fournit le dextran lipidique CM1Doléic (751 milligrammes).

Analyses élémentaires du produit CM$_1$Doléic : % C = 34,25 et % H = 5,91

Le même protocole a été réalisé avec le chlorure palmitique polymère CM$_1$Dpalm.

Les analyses élémentaires du polymère CM$_1$Dpalm donnent : % C = 35,13, % H = 5,96

Le protocole de sulfatation a été réalisé sur ces deux polymères en utilisant 1 équivalent d'acide chlorosulfonique.

Les analyses élémentaires du polymère CM$_1$DoléicS*$_1$ donnent : % C = 28,28; % H = 5,04; % S = 5,26.

Analyses élémentaires du produit CM$_1$DpalmS*$_1$ donnent : % C = 29,17 % ;H = 4,88; % S = 5,14.

Les différents exemples évoqués selon le greffage d'un groupement Z aboutissent à des composés dont la définition de quelques uns d'entre eux sont présentées dans le tableau II ci dessous.

Tableau II

| Référence | Groupements | X | Y | Z |
|---|---|---|---|---|
| RGTA 1110 | CM$_2$DPhSS1 | 52,1 | 43,8 | 8,9 |
| RGTA 1111 | CM$_2$DES$_1$ | 10,7 | 42,4 | 21,2 |
| RGTA 1112 | CM$_2$DPheS2 | 28,9 | 56,2 | 17,9 |
| RGTA 1113 | CM$_3$DTyrS2 | 19,8 | 65,9 | 28,9 |
| RGTA 1114 | CM$_1$DPalmS1 | 39,8 | 47,5 | 3,8 |
| RGTA 1115 | CM$_1$DOléiS1 | 36,0 | 43,9 | 2,2 |

Le tableau II ci-dessus indique les pourcentages de substitution des polymères comportant un groupement Z.

6) Etapes de purification des différents dérivés de dextranes des exemples ci-dessus.

a) Dialyse à l'équilibre.

A la suite de chaque étape de synthèse les polymères sont recueillis sous forme solide (précipitation ou filtration suivie d'une lyophilisation). Les polymères sont ensuite resolubilisés dans le volume minimum d'eau distillée puis introduits dans un boudin de dialyse (Spectrapor) de seuil de coupure 6000 à 8000 g.mol-1. La dialyse est effectuée contre de l'eau bidistillée (MilliQ) dans un rapport de 1 volume de produit pour 50 volumes d'eau pendant 4 à 5 jours avec deux changements d'eau par jour.

b) Chromatographie.

A l'étape précédente est associée un chromatographie HPLC sur tamis moléculaire (Colonne TSK) de façon à établir la masse molaire des polymères purifiés.

c) Ultrafiltration tangentielle.

Après la dialyse, le contenu des boudins est ultrafiltré dans une cellule d'ultrafiltration (Pellikon, Millipore) sur une membrane de cellulose de seuil de coupure 10000 g.mol-1. La qualité de la purification est suivie à l'aide d'une cellule de conductimétrie. Lorqu' en sortie de cellule, la conductivité de l'eau éliminée est revenue à la conductivité de l'eau distillée pure ( $2\mu S$ ), la purification est arrêtée et la solution est concentrée avant lyophilisation.

7) Détermination des pourcentages de substitution.

Dans les deux groupes d'exemples détaillés ci-dessus, les pourcentages de substitution des groupements X, Y et éventuellement Z sont établis de la façon suivante.

a) Polymères poly acide - $\beta$ - malique (Exemples 1).

Pour ces polymères, les pourcentages de substitution sont définis *a priori* en fonction de la proportion des différents monoesters mis à polymériser.

b) Polymères de dextrane (Exemples 2).

Deux cas doivent être envisagés pour ces polymères obtenus à partir du dextrane selon la définition du groupe Z.
Le premier correspond au cas des $CM_nDS_m$ où Z = rien et le second, dépend de la nature chimique de Z.
Sur chaque résidu glucose, 3 fonctions hydroxyles sont susceptibles de réagir. Une masse molaire relative de 54 g.mol-1 est attribuée à chaque fonction hydroxyle, soit le tiers de la masse moléculaire de 162 g.mol-1 d'un résidu constitutif du dextran. Il est admis que chaque hydroxyle possède la même réactivité et que les substitutions affectent d'abord une fois chaque unité glucose avant une éventuelle seconde substitution sur le même résidu.
Un dextran T 40 de 40 000 g.mol$^{-1}$ contient donc 247 résidus glucose de masse molaire 162 g.mol$^{-1}$.
Les taux de substitutions atteints lors des carboxyméthylations sont déterminés par dosage acide-base avec un titrimètre automatique (Tacussel). Ce dosage détermine une valeur $X_1$ correspondant au nombre de moles d'acide fixées par gramme de polymère.
Ainsi, lorsqu'un hydroxyle est substitué, il apparaît sur le glucose un motif : -OCH2COONa. Chacune de ces sous unités substituées a une masse moléculaire relative de 240 g.mol$^{-1}$.
Après la sulfatation, il apparaît plusieurs motifs.
Les taux de groupements carboxyliques libres déterminés par dosage acide base donnent une valeur X2 toujours inférieure à la valeur initiale $X_1$. La différence $X_1$-$X_2$ correspond à des motifs -OCH2COO-SO3Na. Chacune de ces sous unités substituées a une masse moléculaire de 320 g.mol$^{-1}$.
L'analyse par RMN permet de montrer que le S correspond à une sulfatation des hydroxyles libres des résidus glucose en plus de la réaction précédente. Dans ce cas, il apparait un motif -OSO3Na. Chacune de ces sous unités de glucose sulfaté a une masse moléculaire relative de 200 g.mol$^{-1}$. Les microanalyses donnent le taux de S en pourcentage de la masse du polymère.
Il est ainsi possible, en fin de synthèse, en connaissant les pourcentages de radicaux carboxyles libres $X_1$ et $X_2$ déterminés respectivement avant et après l'étape de sulfatation, de considérer que le polymère contient :

- a résidus glucose non substitué de masse 162 g.
- $X_2$ résidus carboxyliques libres de masse 240 g.
- $X_1$-$X_2$ résidus carboxyliques sulfatés de masse 320 g.
- Y résidus glucose sulfatés de masse 200 g. A partir de ces données il est possible d'établir le pourcentage de substitution des groupements X et Y.

Ainsi, le pourcentage de soufre donné par les résultats de microanalyse (S%) permet de connaître le nombre d'atomes de soufre ($\sum_S$) greffés sur le polymère. Ce nombre d'atomes est de $\sum_S$ = (S% x MM) / 32 x 100, où 32 est la masse atomique de S et MM la masse molaire du polymère synthétisé.

Il est posible d'en tirer le pourcentage Y de radicaux SO3⁻ comme étant égal à : (100 x S% x MM)/247 x 3200

Dans le deuxième cas, où le groupement Z greffé est par exemple de la tyrosine, le même raisonnement s'applique avec la valeur de l'azote donnée par les résultats de l'analyse élémentaire.

B) PROPRIETES DES POLYMERES DE L'INVENTION.

Les propriétés communes aux RGTA et aux HBGFPP.

1) Ils sont dépourvus d'activité anticoagulante significative c'est à dire présenter une activité inférieure à 50 UI/milligrammes comparée à celle de l'héparine dont l'activité est de l'ordre de 150 à 170 UI/milligrammes.

2) Ils stabilisent et potentialisent les facteurs de croissance présentant une affinité pour l'héparine et en particulier à tire d'exemple le FGF1 et/ou le FGF2 et/ou le TGF β.

3) Ils protégent ces facteurs contre des agents protéolytiques comme la trypsine.

4) Ils inhibent les activités protéasiques impliquées dans le processus inflammatoire comme par exemple élastase leucocytaire et/ou la plasmine.

5) Ils exercent un effet cicatrisant dans au moins l'un des modèles présentés dans les brevets suscités, à savoir, les muscles, les nerfs, ou le tractus digestif.

Les nouvelles propriétés des RGTA.

6) Ils protégent et potentialisent les activités enzymatiques impliquées dans la lutte contre le stress oxydatif comme par exemple la super oxyde dismutase ou SOD. De par cette propriété ils agissent comme agents anti oxydants et peuvent être utilisés seuls ou associés à la SOD dans des indications thérapeutiques et/ou cosmétologiques de la SOD ou comme agent protecteur antioxydant notamment dans la protection des aliments ou comme nutrient.

7) Ils inhibent l'activité des enzymes comme la Calpaïne

8) Ils inhibent l'activité des enzymes comme l'héparitinase ou l'héparinase.

9) Ils augmentent la survie de cellules soumises à des radiations ionisantes et régulent la sécrétion, sur le plan quantitatif comme qualitatif, des constituants de leur matrice comme les collagènes par exemple.

10) Ils agissent comme des agents antifibrotiques en modulant la croissance des cellules mésenchymateuses comme les cellules musculaires lisses, les fibroblastes ou les cellules hépatiques et la qualité du type de collagène qu'elles sécrètent.

11) Ils présentent une dégradabilité lente critère permettant de les différencier des héparanes sulfates qui sont des produits naturellement dégradés par l'héparinase ou l'héparitinase.

12) Ils ne contiennent pas et ne libèrent pas après dégradation de produits connus pour leur toxicité comme par exemple peut le faire le CMDBS avec les groupements greffés Z = benzylamine, ce qui exclut donc le CMDBS.

13) Ils présentent *in vivo* des capacités de protection et de régénération tissulaire dans les différents modèles suivants :

a) lésion de plaies cutanées,

b) régénération de tissus osseux comme les os longs avec l'exemple du fémur,

c) protection contre la perte de tissu osseux et de régulation de son remaniement comme dans le cas de l'ostéoporose ou des parodonties. De fait ce sont des agents régulateurs de l'homéostasie tissulaire et des masses tissulaires comme la masse osseuse ou musculaire.

d) régénération hépatique ou de protection contre la dégénérescence du système nerveux central et périphérique,

e) protection contre les effets délétères des ischémies quelque soit leur localisation.

EXEMPLE 3 : Mesure des activités anti-coagulantes des polymères des exemples 1 et 2.

Les tests de coagulation sont réalisés selon la technique du Temps de Céphaline Activée ou T.C.A. (Biggs, 1972, in Human blood coagulation, Oxford Blackwell Scientific Publications). Cent microlitres d'une solution de polymère à différentes concentrations en tampon d'Owen Koller sont incubés 5 minutes à 37°C avec 100 microlitres de plasma pauvre en plaquettes et 100 microlitres d'une solution de céphaline de cerveau de lapin. 100 microlitres de chlorure de calcium 0,25 M sont ajoutés et le temps d'apparition du caillot est repéré par chronométrage.

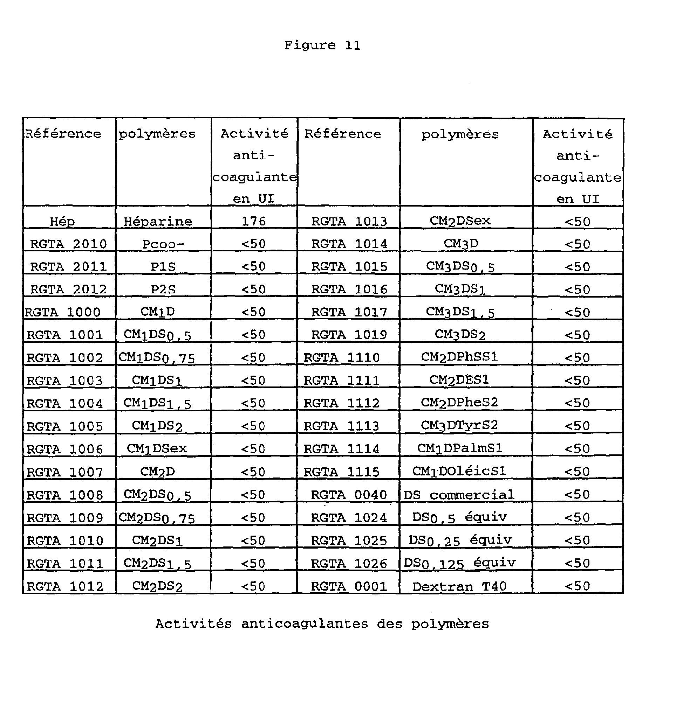

Comme le montre la figure 11, les polymères des exemples 1 et 2 ne présentent pas d'activité anticoagulante supérieure à 50 UI/milligrammes, en particulier par rapport à l'héparine, pris comme contrôle positif. Il convient de remarquer que les valeurs des activités anticoagulantes des produits donnés ici en exemple sont toutes inférieures à 10 UI/milligrammes de produits.

EXEMPLE 4 : Stabilisation et potentialisation des polymères des exemples 1 et 2 sur des facteurs de croissance présentant une affinité pour l'héparine et en particulier à tire d'exemple le FGF1 et/ou le FGF2 et/ou le TGF β.

Cet exemple considère l'effet des polymères sur la stabilisation du FGF1 et l'effet sur la potentialisation du FGF1 et du FGF2. Ces effets sont évalués sur la croissance des cellules 3T3 BALB/c ou CCL39. Les conditions utilisées pour ces expérimentations sont celles décrites dans l'art antérieur.

1) Effets des polymères de l'invention sur la stabilisation des FGF1 ou FGF2.

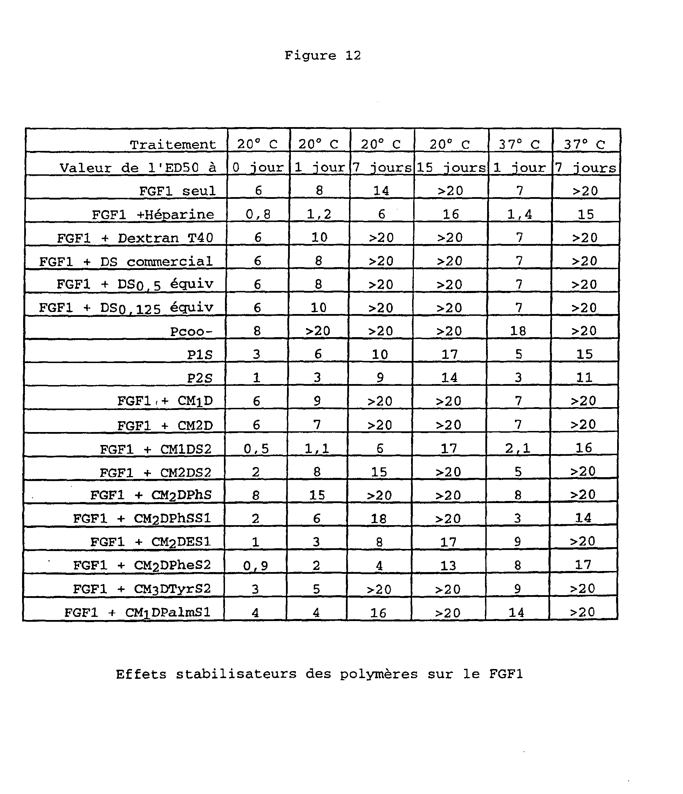

La figure 12 en annexe représente les effets des RGTA contre des dégradations thermiques à 20° C et à 37° C en fonction du temps d'incubation de FGF2 conservé seul ou en présence des produits testés. L'ED5O représente la concentration en mcrogramme/millilitre de FGF1, ici de 6 nanogrammes/millilitre, qu'il faut inoculer à une culture de cellules fibroblastiques, les cellules CCL 39, pour obtenir 50 % du taux maximal d'incorporation de thymidine tritiée.
Les résultats obtenus montrent que l'ensemble des polymères testés exercent à 20° C comme à 37° C, de effets protecteurs comparables à ceux de l'héparine, et parfois avec une efficacité plus importante.

22) Effets des polymères de l'invention sur la potentialisation des FGF1 ou FGF2.

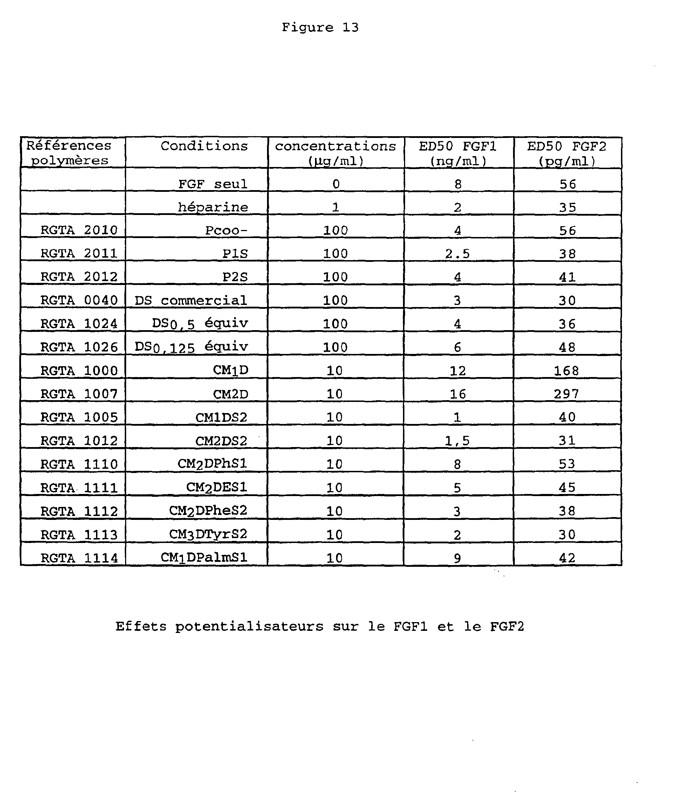

Le protocole est le même que celui précédemment décrit avec une quantité variable de FGF associée ou non à une quantité constante de polymère. La concentration de polymère retenue correspond à celle qui potentialise au maximum l'effet mitogène du FGF. Ces tests sont faits sur les cellules 3T3 pour le FGF1 et le FGF2. Les contrôles sont les mêmes que ceux cités précédemment à l'exception d'une étude systématique pour chaque test de la valeur de l'ED50.
Les molécules de ces deux familles de polymères testés potentialisent les actions du FGF1 et du FGF2 puisque les valeurs de l'ED50 sont obtenues pour des valeurs de FGF inférieures ou comparables à celle obtenue en présence d'héparine (figure 13).

EXEMPLE 5 Protection des facteurs contre des agents protéolytiques comme la trypsine.

La trypsine est une enzyme protéolytique à large spectre d'action qui est utilisée dans les tests in vitro et qui est une des enzymes fonctionnelle primordiale dans les processus digestifs.
Le test de protection contre la trypsine se déroule ainsi. Un nanogramme/millilitre final de FGF2 iodé ou 5 microgrammes/millilitre final de trypsine sont incubés dans un premier temps 15 minutes à 37°C avec différentes concentrations de polymères (0,5 à 500 microgrammes/millilitre) dans un tampon Tris HCl 100 mM, 0,18 M NaCl, brij 0,03% pH 7,6. 5 microgrammes/millilitre final de trypsine ou 1 nanogramme/millilitre final de FGF2 iodé sont alors ajoutés respectivement. Le volume réactionnel total est de 30 μl. La réaction enzymatique est arrêtée après 2 h d'incubation à 37°C, par addition de tampon Laemmli et un chauffage de 5 minutes à 90°C (Laemmli U.K. Cleavage of structural proteins during assembly of the head of the bacteriophage T4, Nature, 1970, 227:680-685).
Chaque échantillon est déposé sur un gel de polyacrylamide de 15%. La migration s'effectue pendant 1 h à 200 V dans la glace. Les gels sont séchés 2 h à 80°C sous vide et exposés à -80°C en présence d'un film autoradiographique. L'intensité des bandes est mesurée par analyse d'image et le pourcentage de FGF2 protégé par rapport au FGF2 dégradé calculé

Protection du FGF1 et du TGF β

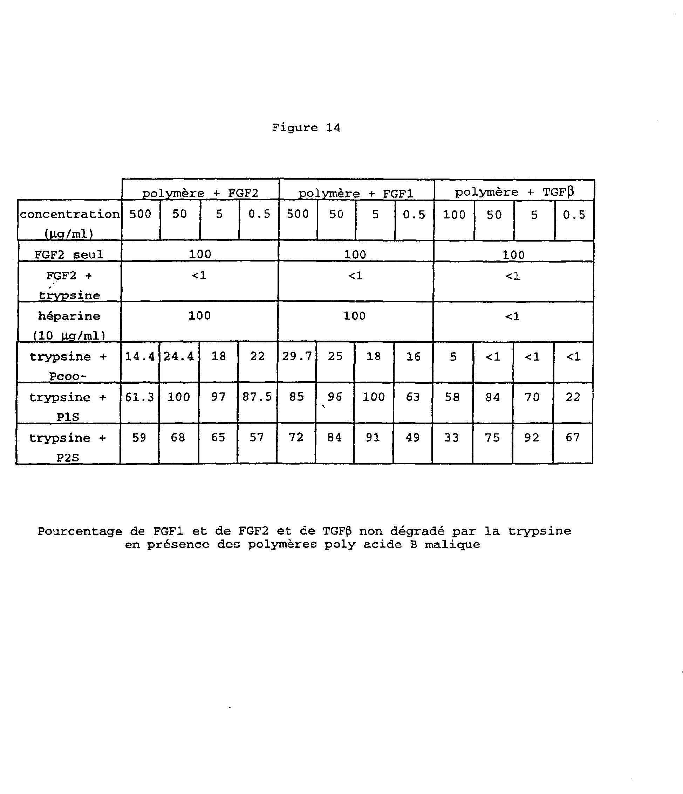

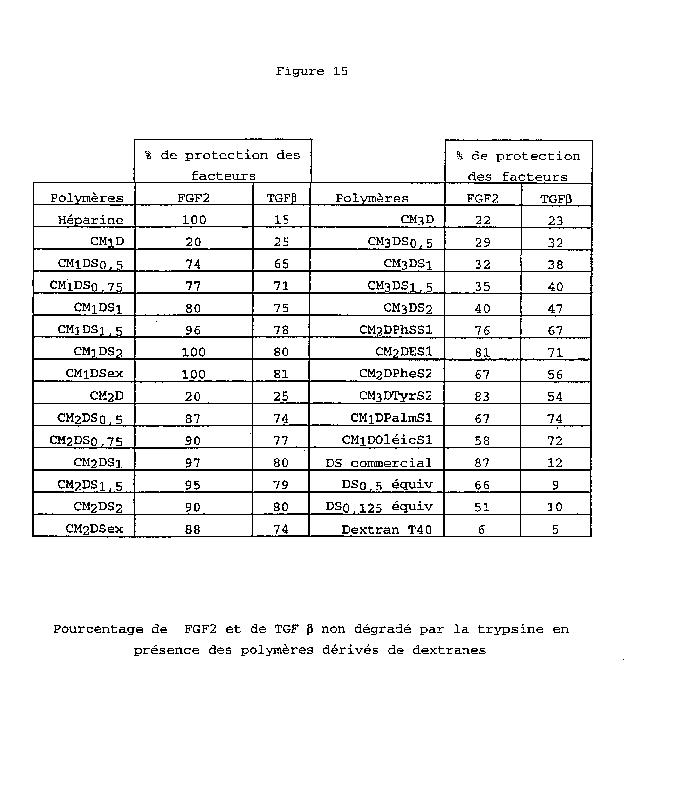

La figure 14 en annexe présente les effets protecteurs exprimés en % de protection des polymères poly acide β malique sur le FGF1, le FGF2 et le TGFβ, vis à vis d'une attaque par la trypsine. La figure 15 présente les effets protecteurs exprimés en % de protection des polymères dérivés de dextrane sur le FGF2 et le TGFβ, vis à vis d'une attaque par la trypsine.
Ces polymères exercent pour la plupart d'entre eux un effet protecteur comparable à celui de l'héparine pris comme référence positive.

EXEMPLE 6 : Inhibition des activités protéasiques impliquées dans le processus inflammatoire comme par exemple l'élastase leucocytaire ou la plasmine.

L'élastase leucocytaire et la plasmine sont des protéases clés dans l'installation et le déroulement de la phase inflammatoire. Ces tests visent à établir si les polymères protègent les facteurs de croissance des dégradations par l'élastase leucocytaire humaine.

Les tests de protection contre l'élastase leucocytaire se déroulent ainsi. Le protocole utilisé est le même que celui utilisé avec la trypsine avec 30 nanogrammes/millilitre final d'élastase et 0,5 à 500 microgrammes/millilitre de polymères. Le tampon est du Tris HCl 100 mM, NaCl 0,18 M, 0,03% brij pH8. L'intensité des bandes est évaluée par analyse d'image et le pourcentage de FGF2 non dégradé est calculé. Le contrôle positif pour ces tests est l'héparine. Le dextran T40 et le dextran sulfate ont été pris comme références internes.

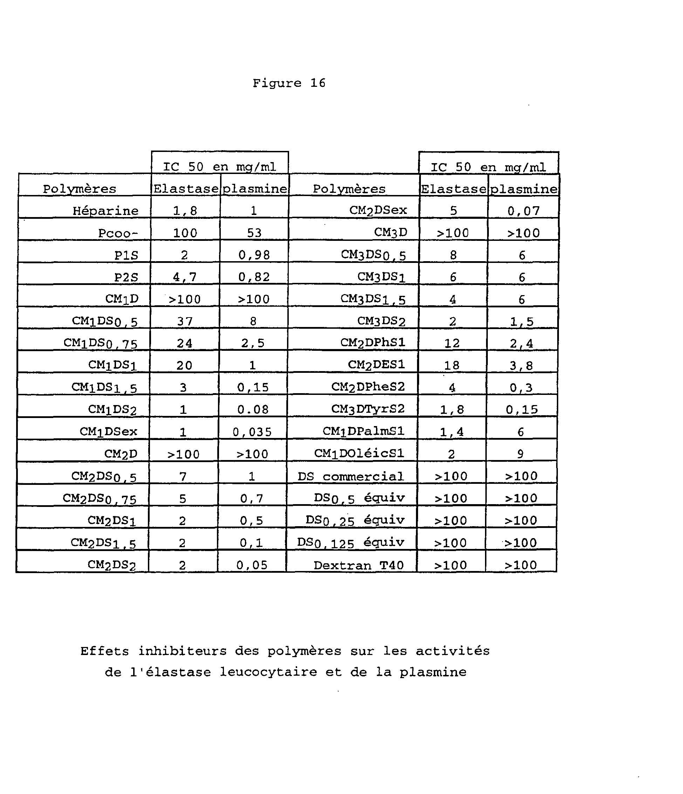

La figure 16 rapporte les effets inhibiteurs des différents polymères exprimés par les valeurs des IC50.

EXEMPLE 7 Exemple des effets des RGTA sur la régénération, la protection et la restauration fonctionnelle des tissus cas de la régénération du muscle squelettique.

Le modèle retenu pour apprécier le plus efficacement les propriétés cicatrisantes des RGTA a été celui de l'écrasement musculaire tel que défini et présenté dans le brevet Français n° 2 718 026.

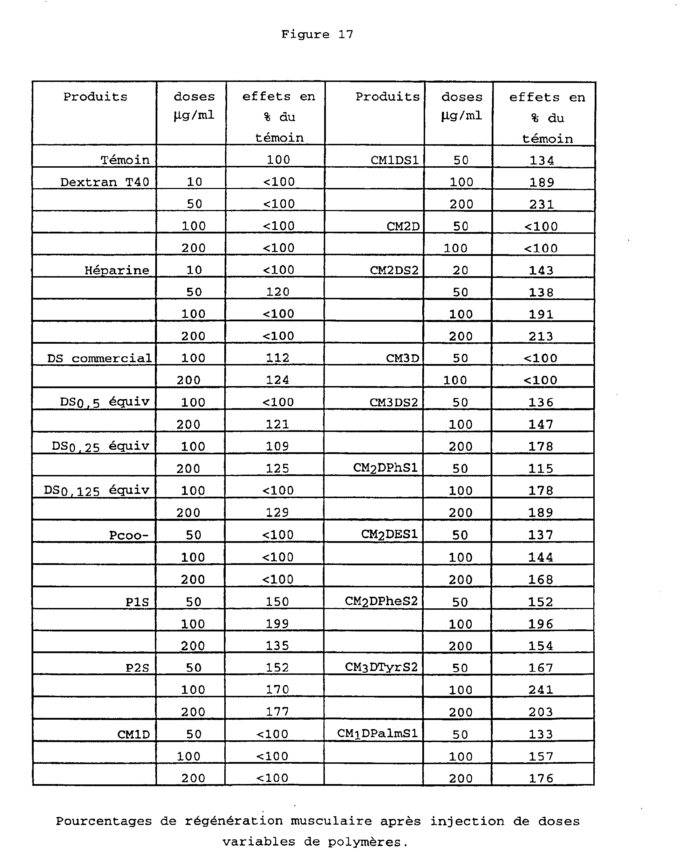

Après écrasement du muscle EDL (Extensor Digitorum Longus) de la patte arrière du rat et injection dans le muscle écrasé d'une solution de sérum physiologique contenant ou non les substances à tester, les muscles ainsi traités sont récupérés 8 jours après l'opération. L'analyse du poids associée à une étude histologique permettent de quantifier les effets des polymères sur la régénération musculaire. Les résultats sont exprimés en % par rapport aux caractéristiques d'un muscle n'ayant reçu qu'une injection de sérum physiologique sans polymère dans les mêmes conditions expérimentales. La figure 17 présente les résultats obtenus qui démontrent qu'aussi bien les nouveaux polymères dérivés du squelette de dextrane que les copolymères d'acide $\beta$ malique excercent les effets révendiqués.

Il est important de noter que ces effets sur la régénération musculaire sont obtenus aussi bien par injection in situ des polymères que par injection intra veineuse, intra artérielle ou intra musculaire à partir du moment où les doses injectées en fonction des voies d'administration sont adaptées.

EXEMPLE 8 : Effets des RGTA sur la régénération de l'os plat.

Le modèle retenu pour évaluer les propriétés cicatrisantes des RGTA est celui déjà connu de l'art antérieur et décrit par Blanquaert F, et al. Bone. 1995; 17(6): 499-506).

Ce modèle consiste à pratiquer une trépanation circulaire de 5 millimètres de diamètre dans la calvaria d'un rat adulte. Le défaut est comblé par un tampon de collagène prédécooupé aux mêmes dimensions et imbibé ou non dans une solution contenant des RGTA. Dans l'exemple présenté ici, les polymères étudiés sont les polymères de type CMS (RGTA 1005 et 1012) et les copolymères d'acide $\beta$-malique (RGTA 2011). Le tableau III ci-dessous présente les pourcentages de comblement osseux établis par analyse d'image des radiographies prises 35 jours après le traitement.

Tableau III

| Type de traitement | % de comblement osseux |
| --- | --- |
| contrôle | 18 +/- 4,8 |
| RGTA 2011 | 56 +/- 7,0 |
| RGTA 1005 | 54 +/- 6,9 |
| RGTA 1012 | 72 +/- 8,9 |

Les deux types de polymères stimulent donc la régénération osseuse d'autant que dans ces conditions de cicatrisation la suture sagittale se reforme *ad integrum.*

Exemple 9 Protection et potentialisation des activités enzymatiques impliquées dans la lutte contre le stress oxydatif comme par exemples la super oxyde dismutase ou SOD.

La production d'ions $O_2^-$ et celle de peroxyde d'hydrogène ($H_2O_2$) représentent des radicaux qui exercent des effets cytotoxiques particulièrement destructeurs. Les SOD ou super oxydismutase sont des enzymes engagées dans la détoxification de ces radicaux. Ce sont des agents qui préservent l'organisme du stress oxydatif.

Les RGTA présentent plusieurs types d'activité vis à vis de la SOD :

- Ils exercent un effet potentialisateur sur l'activité catalytique de la SOD à pH neutre et un effet protecteur et potentialisateur à des pH acide et basique.
- Ils présentent la propriété de protéger les SOD vis à vis des dégradations enzymatiques comme par exemple la trypsine et d'autre part vis à vis de traitements thermiques.
- Sur des modèles de cultures de monocytes activés, ils stimulent les activités catalytique des SOD endogènes et permettent de diminuer la production des ions superoxydes.

Dans tous les cas, les dosages de l'activité SOD sont réalisés selon la technique de Pick, (Freund M et Pick E, The mechanism of action of lymphokines. IX. The enzymatic basis of hydrogen peroxide production by lymphokine-activated macrophages. J Immunol 1986 15:137(4):1312-1318). Cette technique repose sur le dosage des ions $O_2$- par réduction du cytochrome c. De la MnSOD (Sigma ref. S 8151) à 30 U/millilitre est mise en solution en présence de différents RGTA à la concentration de 10 microgrammes/millilitre. Ces mélanges subissent différents traitements.

L'activité SOD est évaluée en présence de différentes concentrations de polymères dans des conditions réactionnelles normales.

Soit, les mélanges de SOD et de polymères sont soumis à température ambiante à l'action de la trypsine (même conditions que pour les tests de protection de l'exemple 5) soit ils sont soumis à un traitement thermique à 60°C pendant 30 minutes. L'activité catalytique résiduelle de la SOD de ces mélanges est alors évaluée par les techniques enzymatiques classiques ou sur système cellulaire.

Les échantillons ainsi traités sont incubés dans une suspension de monocytes ($2,5\ 10^6$ cellules/millilitre) stimulés par 200 nM de PMA. Cette condition induit la production d'anion superoxyde par ces monocytes activés en macrophages. La stimulation par le PMA induit une augmentation de la production d'ions superoxyde normalement produits à un niveau basal en l'absence d'activation. L'adjonction de MnSOD active utilisée comme contrôle positif, diminue la quantité d'ions superoxydes produits.

Dans ces conditions, plus la production d'ions superoxydes sera faible, plus l'activité catalytique résiduelle de la SOD contenue dans les mélanges sera élevée. Ces tests permettent donc d'apprécier les effets protecteurs et potentialisateurs des polymères sur l'activité SOD exogène comme endogène.

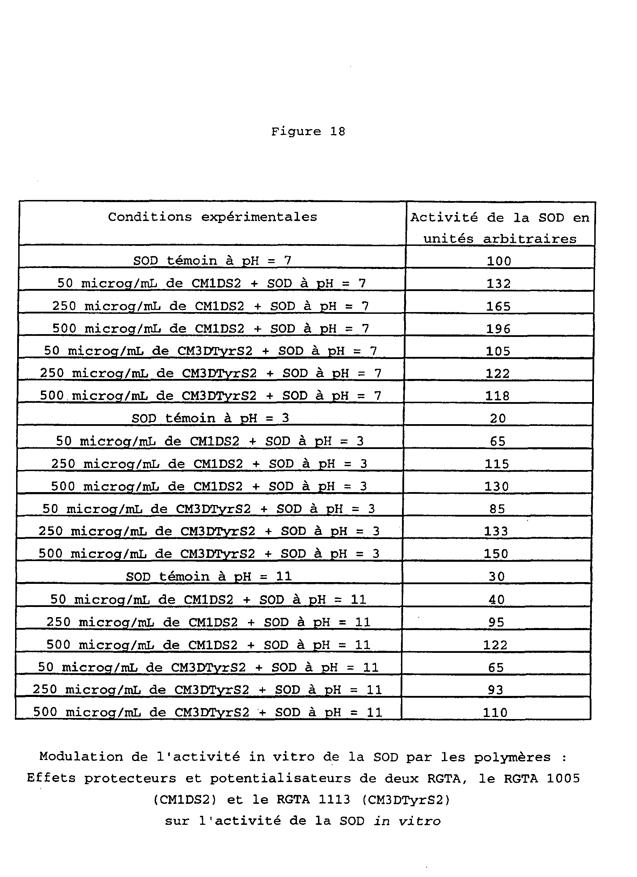

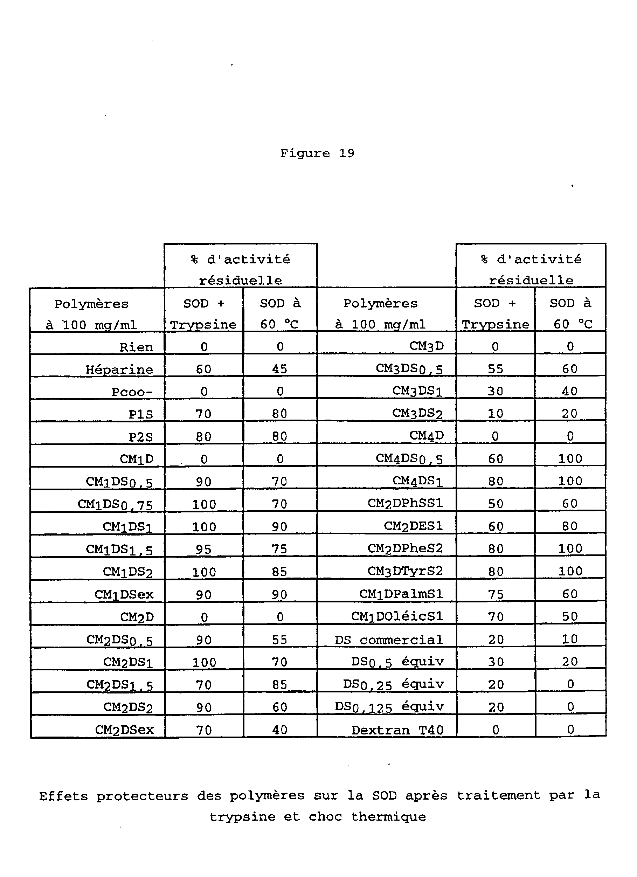

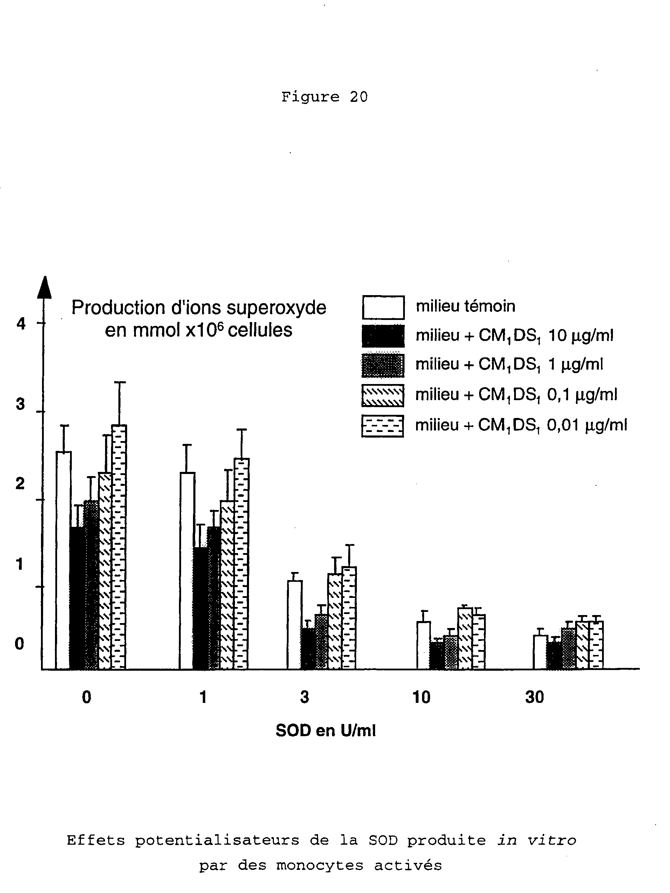

La figure 18 illustre les effets protecteurs et potentialisateurs des RGTA sur l'activité catalytique de la SOD in vitro à diférents pH, la figure 19 les effets protecteurs des RGTA sur la SOD soumise à une attaque par la trypsine ou après un choc thermique et la figure 20 les effets potentialisateurs de l'activité SOD sur la production d'ions superoxydes par des macrophages activés.

Conclusion::Les polymères exercent donc des effets potentialisateurs et protecteurs aussi bien dans des systèmes acellulaires que cellulaires. L'adjonction des différents polymères module donc l'activité catalytique de SOD ajoutée de façon exogène comme de SOD produite de façon endogène par les cellules.

EXEMPLE 10 Inhibition de l'activité des enzymes comme la Calpaïne par les RGTA.

1) Introduction.

La calpaïne 1 (Sigma P 4533) est utilisée dans cet exemple. Ainsi, 0.78 unité d'enzyme (57,2 nM) sont incubées dans 1 millilitre de tampon 50 mM Tris-HCl pH 7.4, 0.05% Brij, 2mM CaCl2, 2 mMDT et 0.15M NaCl. Les solutions de RGTA à tester sont ajoutées pendant 5 minutes à 27°C. Le substrat (250 micromolaire de N-succinyl-leu-tyr-amido-7méthyl coumarin, de chez Sigma (S 1153) est ensuite ajouté dans une cuve en quartz dans laquelle le mélange décrit ci-dessus est ajouté. Les mesures sont ensuite réalisées toutes les 3 à 5 minutes par excitation à 380 nm et détection en fluorescence à 460 nm de la transformation du substrat en 7 amino-4methyl coumarin (selon le protocole décrit par Sasaki, Kikuchi, Yumoto N, Yoshimura et Murachi T, 1984.dans J. Biol. Chem 259,(20) p 12489-12494.

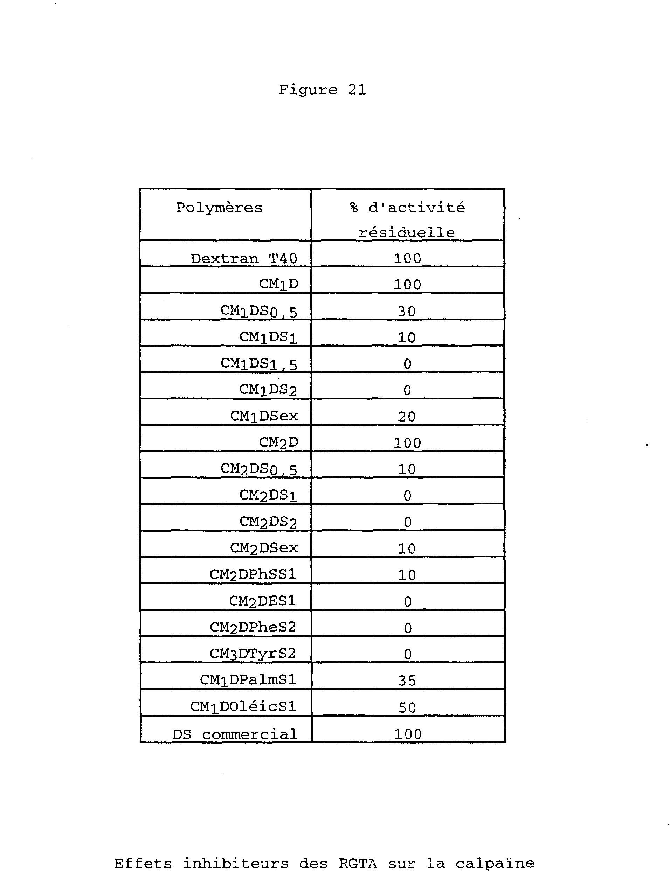

Les résultats obtenus sont résumés dans la figure 21.

2) Conclusion.

Des résultats obtenus dans l'inhibition de l'activité de la calpaïne par les RGTA, nous pouvons prédire que les RGTA ont une activité de protection contre les lésions induites par les ischémies cérébrales comme le laissent prévoir les publications de Markgraf C G et col. (Stroke, 1998, 29, 152-158) ou encore Saido et al, (Neuroscience. Letters, 1997 16;227:75-78) qui décrivent l'effet d'inhibiteurs des calpaïnes comme le MDL28170 ou encore la protéine Calpastatine dans le traitement des lésions post ischémique du cortex ou de l'hippocampe. L'adminsitration par voie locale ou intraveineuse de RGTA aura l'effet inhibiteur des calpaïnes et favorisera la réparation du tissus nerveux lésé notamment par manque d'apport en oxygène comme c'est le cas des ischémies.

EXEMPLE 11 : Inhibition de l'activité des enzymes comme l'héparitinase par les RGTA.

1) activité inhibitrice des héparinase et héparitinases.

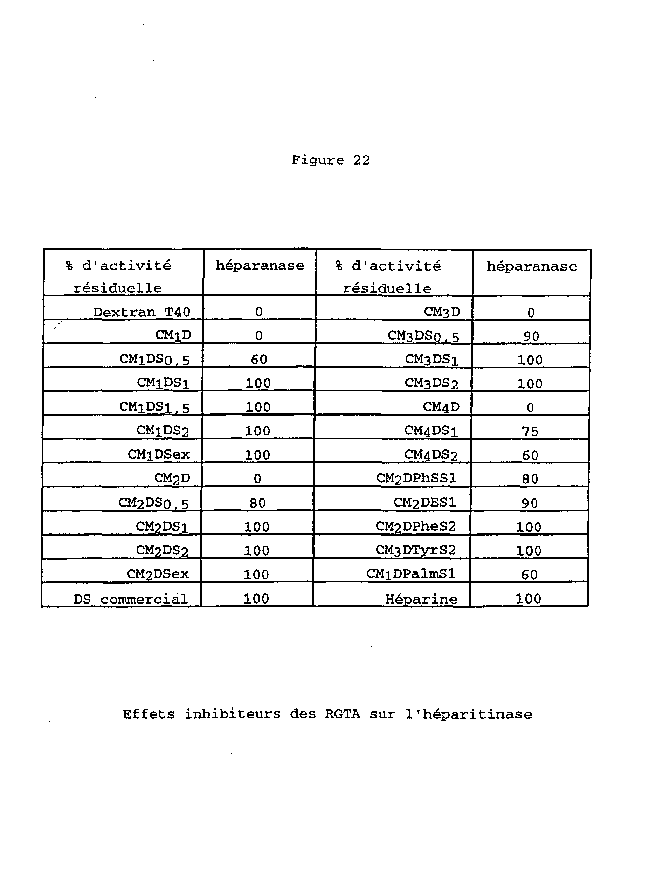

La mesure de l'inhibition par les RGTA des activités héparinase ou héparitinase est réalisée en utilisant comme substrat les héparanes sulfates radiomarqué au soufre 35 et présents dans une matrice extracellulaire synthétisés par des cellule endothéliales cultivés en présence de Na2 35SO4 pendant 7 jours. Le protocole utilisé est celui décrit par Ishai-Michaeli R et coll. (Importance of size and sulfation of heparin in release of basic fibroblast growth factor from the vascular endothelium and extracellular matrix. Biochemistry 1992;31(7):2080-2088). La matrice extracellulaire obtenue après élimination des cellules endothéliales est ensuite incubée 24h à 37°C en présence ou en absence d'héparanase et 0.5 microgramme /ml de RGTA.. Afin de mesurer la dégradation des héparanes sulfates radio marqués le milieu d'incubation est collecté et déposé sur une colonne de Sepharose 6B selon le protocole décrit dans Ishai-Michaeli (Biochemistry 1992;31(7):2080-2088). La figure 22 illustre les résultats obtenus. Les héparanes sulfates de haut poids moléculaire correspondant au matériel non dégradé par l'enzyme héparanase sont élues en premier (fractions 3 à 20). Le traitement à l'héparanase induit la disparition de ce pic et l'apparition d'un pic correspondant à des fractions de petit poids moléculaire d'héparanes sulfate dégradé. (fractions 20 à 40). Dans l'exemple donné l'effet du RGTA 1005 à 50 microgramme/ml induit 50% d'inhibition et 100 microgrammes/ml 100% d'inhibition de l'activité héparanase.

EXEMPLE 12 : Effets des RGTA sur la protection des cellules musculaires lisses intestinales humaines soumises à des radiations ionisantes; effets sur leur survie et sur leurs effets antifibrotiques évalués à travers la quantité et la qualité des collagènes sécrétés.

La formation d'un tissu fibreux ou fibrotique est une étape physiologique essentielle, associée aux processus de réparation et de remodelage tissulaire. Le tissu fibrotique est un tissu de comblement, normalement transitoire, visant à conserver l'intégrité structurale et fonctionnelle des tissus et organes. Il se caractérise par la richesse en collagènes de la matrice extra-cellulaire.

Lorsque cette situation persiste ou se développe, elle correspond à une pathologie illustrant un dérèglement de l'homéostasie structurale et fonctionnelle des tissus et se traduit par une accumulation anormalement élevée de matrice extra-cellulaire qui engendre une fibrose. Une fibrose, quelle que soit son origine se caractérise par :

la présence d'un infiltrat inflammatoire permanent, l'existence d'un déséquilibre dans la balance entre un état prolifératif et quiescent des cellules conjonctives ou mésenchymateuses comme les fibroblastes ou les cellules musculaires lisses, la destruction progressive du tissu envahi qui se renouvelle peu ou de façon défectueuse, l'existence d'un déséquilibre de la balance entre la synthèse et la dégradation de la matrice extra-cellulaire.

Une fibrose peut être induite soit à la suite d'un traumatisme d'origines variées (infectieux, mécanique, toxique, etc...) soit à la suite de radiations ionisantes (notamment par des rayons y). Il s'agit dans ce cas des fibroses radioinduites comme c'est fréquemment le cas chez les patients ayant subi une radiothérapie.

Les collagènes sont les composants majeurs de la matrice extra-cellulaire des tissus normaux comme des tissus fibrotiques. Ils sont essentiellement synthétisés par les cellules mésenchymateuses comme les fibroblastes et les cellules musculaires lisses. Dans un tissu fibreux, le collagène total est quantitativement augmenté, en raison de modifications affectant aussi bien, sur le plan quantitatif que qualitatif, la synthèse et/ou la dégradation des collagènes, c'est à dire la dynamique du remodelage. Les fibroses se caractérisent par une augmentation du collagène de type III, et ceci préférentiellement lors de fibroses radioinduites. Cette augmentation de collagène de type III est associée à une augmentation mais dans une moindre importance du rapport entre le collagène de type III et le collagène de type I. Un autre collagène, le collagène de type V, est associé à la qualité de l'organisation des fibres de collagènes dans la matrice c'est à dire la fibrillogenèse. Dans les tissus fibrotiques, l'effondrement du taux de collagène de type V est une des origines de la déstructuration des fibres de collagène de la matrice extracelulaire.

Le modèle cellulaire considéré dans cet exemple est celui des cellules HISM, Human Intestinal Smooth Muscle cells, (American Type Culture Condition, Rockville, Maryland ATCC CRL 192), issues de la muscularis propria de jéjunum humain (Graham M.,Diegelmann R., Elson C., Bitar K. and Ehrlich H. Proc. Soc. Exp. Biol. Med. 176 (1984) 503.). Cette lignée de cellules musculaires lisses intestinales humaines a été utilisée pour apprécier les effets des radiations sur la survie cellulaire et l'induction de phénomènes fibrotiques analysés à travers la quantité et la qualité des types de collagène sécrétées par des cellules normales ou en situation d'inflammation ou de processus de réparation par une fibrose.

Les cellules sont cultivées en milieu DMEM contenant 1 g/l de glucose, 1% de L-glutamine, 1% pénicilline-streptomycine et 10% de sérum de veau foetal et conservées dans un incubateur à 37°C sous une atmosphère saturée à 5% de $CO_2$ et 95 % d'humidité relative, dans des flasques 75 cm$^2$. Les cellules HISM sont ensemencées sur plaques 24 puits à fond plat à raison de 20 000 cellules par puits. Le volume de chaque puits est complété à 2 milliL de milieu.

Plusieurs cinétiques de croissance sont réalisées en présence ou non de RGTA à différentes concentrations allant de 0,4 à 400 microgrammes/milliL.

L'irradiation est effectuée à partir d'une source d'irradiation de 60 Cobalt dans un incubateur où les plaques de culture sont disposées. Deux boites sont soumises simultanément à cette irradiation dont la source est verticale et dont les doses sont évaluées en fonction de la surface irradiée. Le débit de l'irradiateur est de 1 Gy/mn. Les doses absorbées sont de 10 Gy pour un temps d'irradiation par boîte de 10 minutes.

Différents protocoles sont utilisés pour apprécier le rôle des RGTA selon qu'ils sont ajoutés au milieu de culture avant, pendant ou après l'irradiation c'est à dire sur un plan préventif, curatif ou les deux à la fois. Le tableau IV ci-dessous précise les différents protocoles.

Tableau IV

| Séquence | RGTA | irradiation | RGTA |
|---|---|---|---|
| Témoin | - | - | - |
| Irradiation | - | + | - |
| Curatif | - | + | + |
| Préventif | + | + | - |
| Préventif et curatif | + | + | + |

L'effet préventif est évalué par adjonction de RGTA (+) à la dose de 400 microgrammes/mL dans le milieu de culture, 48 heures avant l'irradiation. L'effet curatif est apprécié par adjonction de RGTA 2 heures après l'irradiation aux mêmes doses que pour l'effet préventif. Pour les effets cumulés préventifs et curatifs, les cellules sont continuellement cultivées en présence des mêmes doses de RGTA.

72 heures après l'irradiation, les cellules sont incubées dans un milieu dépourvu de sérum en présence de proline tritiée (10 microCi/mL) et d'acide ascorbique (50 microgrammes/mL) pendant 24 heures. Le surnageant est alors prélevé et la couche cellulaire récupérée à l'aide d'un robber-policeman dans un volume final de 18 mL. Les milieux de culture et les couches cellulaires récupérées sont extensivement dialysées (seuil de coupure de 6 à 8 kDa)contre de l'eau courante (24 heures à 4°C) pour éliminer les petites molécules des macromolécules. Après dialyse, des fractions aliquotes sont prélevées et hydrolysées (HCl 6M, 105°C, 24 h) pour déterminer la radioactivité de l'hydroxy($^3$H)proline, marqueur spécifique des collagènes A ce stade, une fraction aliquote est utilisée pour quantifier par comptage de la radioactivité, la synthèse totale de collagènes.

Le volume restant est à nouveau dialysé en présence de pepsine et de collagène I contre de l'acide acétique 0,5 M pendant 24 h à 4°C. La pepsine va digérer les contaminants non collagèniques qui seront éliminés dans le dialysat sous forme de peptides. Du collagène de type I est ajouté afin d'augmenter la proportion de collagène qui est faible dans chaque échantillon et de tendre vers un rapport enzyme/substrat de 1/5. Les conditions de réaction sont les suivantes : 1 mL de solution de pepsine (0,5 M) + 1 mL de solution de collagène I (0,5 M) + 0,514 mL d'acide acétique pour avoir une concentration finale en acide acétique de 0,5M, dans un échantillon de 18 mL. Chaque dialysat ainsi obtenu est lyophilisé à froid (-50°C) et conservé à -20°C jusqu'à utilisation.

Les différentes chaînes α de collagène sont séparées par électrophorèse sur gel de polyacrylamide en présence de SDS (Sodium Dodécyl Sulfate) selon la méthode de Leammli, après réduction avec du β mercaptoéthanol. La radioactivité incorporée dans chaque collagène est déterminée par hydrolyse des chaînes α issues du découpage des bandes de gel correspondant à chaque chaîne de collagène d'un type précis. Celles-ci sont ensuite dissoutes dans de l'eau oxygénée à 60°C et la radioactivité contenue dans les différentes bandes dissoutes est comptée au compteur β à scintillation liquide ou par autoradiographie directe des gels.

La séparation électrophorétique est réalisée avec 5 microlitres de collagène V et 50 microlitres de chaque échantillon réduit.

Ces gels permettent plusieurs types d'exploitation :

- La détermination des différents types de collagène par analyse de la radioactivité incorporée.
- La quantification de ces différents types de collagène par analyse densitométrique après autoradiographie.

Les fractions aliquotes prélevées après la dialyse contre de l'eau courante sont hydrolysées (HCl 6M, 105°C, 24 h) en ampoules scellées. L'acide acétique est ensuite évaporé, puis le contenu de l'ampoule est resuspendu dans 1 ml d'eau distillée. La proline et l'hydroxyproline sont séparés par la méthode de Rojkind et Gonzales (Rojkind M et Gonzalez E, An improved method for determining specific radioactivies of proline-14C and hydroxyproline-14C in collagen and in noncollagenous proteins.Analytical Biochemistry 1974;57(1):1-7). Le principe consiste à oxyder la proline et l'hydroxyproline par la chloramine T. La proline est transformée en pyrroline carboxylate soluble dans le toluène alors que l'hydroxyproline est transformée en carboxylate soluble dans l'eau. Après traitement, les deux fractions proline et hydroxyproline sont récupérées pour chaque échantillon et quantifiées par mesure de la radioactivité.

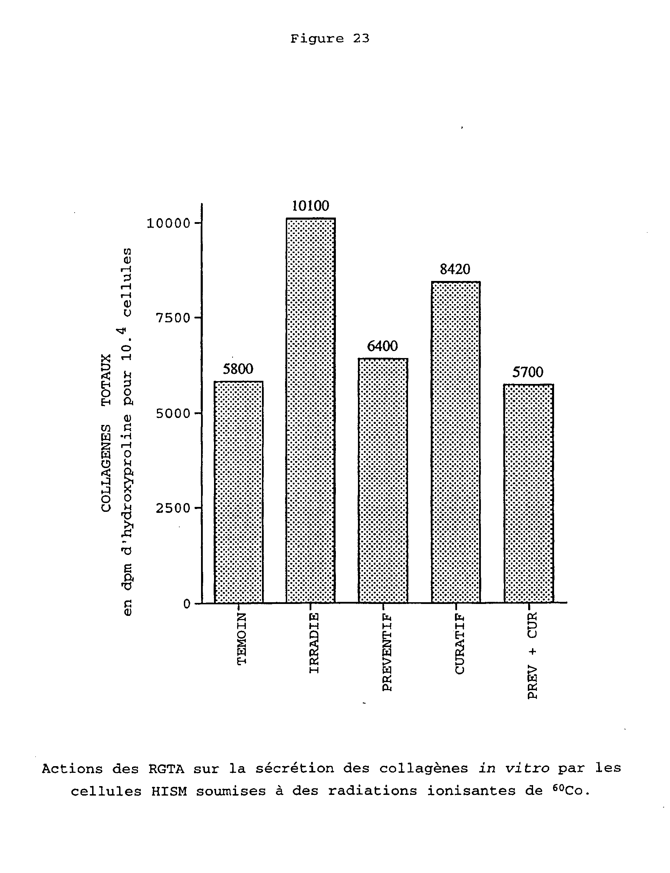

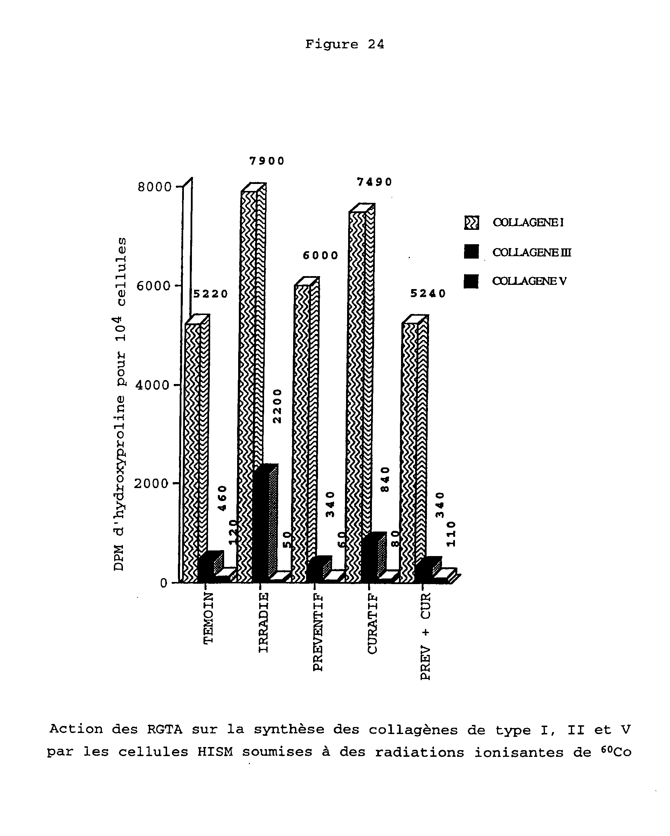

En conditions normales, les cellules HISM se caractérisent par la quantité de collagène synthétisé (figure 23) et surtout, sur le plan qualitatif, par le phénotype des collagènes sécrétés (figure 24).

Dans des conditions normales, le collagène majoritaire est le collagène de type I. Les collagènes de type III et de type V sont minoritaires. La quantité de collagène de type V est associée à la qualité de l'organisation de la fibrillogenèse.

Le collagène de type III est un "collagène d'alerte", synthétisé en théorie de façon transitoire dans le cas d'une réaction à un stress mais de façon permanente dans le cas d'une fibrose. La quantité proportionnelle de collagène de type III par rapport au collagène de type I augmente de façon importante dans les situations de réponse à une lésion tissulaire, un stress ou une agression comme par exemple des radiations ionisantes. Le collagène de type III devient prépondérant dans les matrices des tissus présentant une réaction fibrotique aiguë comme chronique. Ce collagène peut être considéré comme un composant signalétique de la réaction fibrotique.

Lorsque les cellules HISM sont cultivées dans des conditions témoins c'est à dire sans RGTA, elle synthétisent ces trois types de collagènes (figure 24). L'irradiation modifie la quantité (figure 23) et la qualité (figure 24) des collagènes élaborés. La synthèse globale de collagène augmente de près de 50 % (figure 23). La sécrétion ce collagène de type I et surtout de type II augmente considérablement (respectivement de 50 % et de près de 500 %). A l'inverse, la synthèse de collagène de type V diminue de plus de 50 %.

La présence de différents RGTA (RGTA 1005 et RGTA 1025) restaure presque à l'identique le comportement témoin des cellules malgré leur exposition à l'irradiation. La figure 23 montre que la synthèse globale de collagène revient à des valeurs normales en particulier pour les conditions de traitements préventifs ou cumulés (Prév + Cur). La figure 24 confirme ce retour à une homéostasie de référence en particulier dans le cas des traitements cumulés. Les traitements curatifs comme préventifs exercent les mêmes types d'effets, surtout en ce qui concerne les valeurs des rapports de sécrétion des collagènes de type I et de type III qui retrouvent des valeurs comparables à celles des cellules témoins. Ces RGTA restaurent le fonctionnement des cellules HISM vis à vis des collagènes.

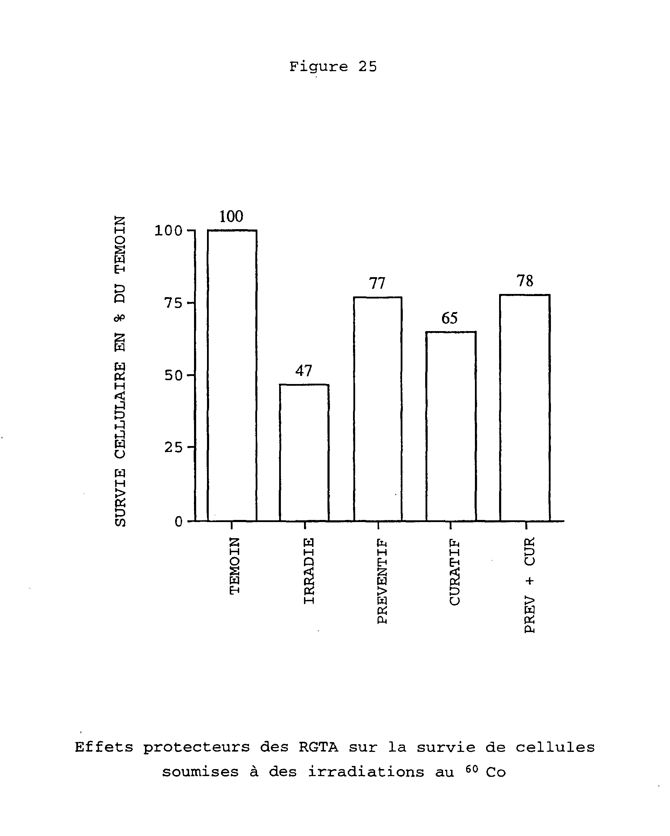

Ils agissent également sur la survie des cellules (figure 25). L'irradiation provoque en effet une mortalité cellulaire importante de plus de 50 % de la population irradiée. En présence des polymères, un effet protecteur net est enregistré puisque la mortalité est réduite à environ 25 %, l'effet le plus marqué étant obtenu pour les traitements cumulés préventif + curatif.

EXEMPLE 13 : Action comme agents antifibrotiques en modulant la croissance des cellules mésenchymateuses comme les cellules musculaires lisses, les fibroblastes ou les cellules hépatiques et la qualité du type de collagène qu'elles sécrètent.

Comme exposé dans l'exemple 12 précédent, la fibrose traduit une altération de la croissance des cellules mésenchymateuses associée à une modification de la quantité et de la qualité des collagènes synthétisés.

Ce exemple utilise un autre modèle cellulaire que sont les cellules musculaires lisses d'aorte de porc. Ces cellules sont obtenues selon la méthode des explants à partir d'aortes. L'aorte est composée de 3 couches cellulaires : l'adventice (couche externe) qui contient les cellules fibroblatiques, la média (couche centrale) qui contient les cellules musculaires lisses (CML) et l'intima (couche interne) qui contient les cellules endothéliales.

La média, après prélèvement est dilacérée en petits morceaux. Les explants sont mis en culture en flasque 25 cm$^2$ contenant du DMEM 1 g/L de glucose, avec rouge de phénol, additionné de 20% de sérum de veau foetal (SVF), 1% de L-glutamine et 1% de pénicilline-streptomycine. Les cellules se détache de ces explants et vont coloniser le milieu (stade P0). Au bout de 2 semaines de mise en culture, les cellules sont congelées à une densité cellulaire de 2 millions de cellules dans du DMSO 10 % et DMEM 20% SVF.

Les cinétiques de croissance sont effectuées en évaluant le nombre de cellules par puits à l'aide du compteur automatique de particules (Coulter Counter ZM, Coultronics) étalonné préalablement par une évaluation du diamètre des cellules à l'aide d'une cellule de Malassez. Chaque comptage est réalisé sur une moyenne de 4 puits à partir desquels les cellules sont détachées à l'aide de 500 microL/puits d'une solution de trypsine-EDTA (10mM).

Les CML sont ensemencées dans les mêmes conditions d'expérimentation que les cellules HISM. Les cinétiques de croissance sont réalisées en présence ou non de polymères des séries RGTA 1000 à 1025 pour les polymères ou Z est rien, et quand Z existe, pour les séries des RGTA 1110 à 1115 ou d'héparine à titre de contrôle, aux différentes concentrations allant de 0,4 à 400 mg/mL.

Le pourcentage d'inhibition de la prolifération est calculé selon la formule suivante :

$$I\% = 100 \times (1 - \text{croissance nette avec polymères})/\text{croissance nette témoin}$$

où la croissance nette représente la différence entre la quantité de cellules dénombrées lorsqu'elles sont à confluence avec le nombre de cellules ensemencées en début de mise en culture.

Le témoin correspond aux cultures de CML en absence de polymères. L'héparine et les RGTA représentent les différents effecteurs testés, susceptibles de modifier l'activité biologique de la cellule.

Dans les mêmes conditions que les cellules HISM, la synthèse et le typage des collagène par les CML a été réalisé.

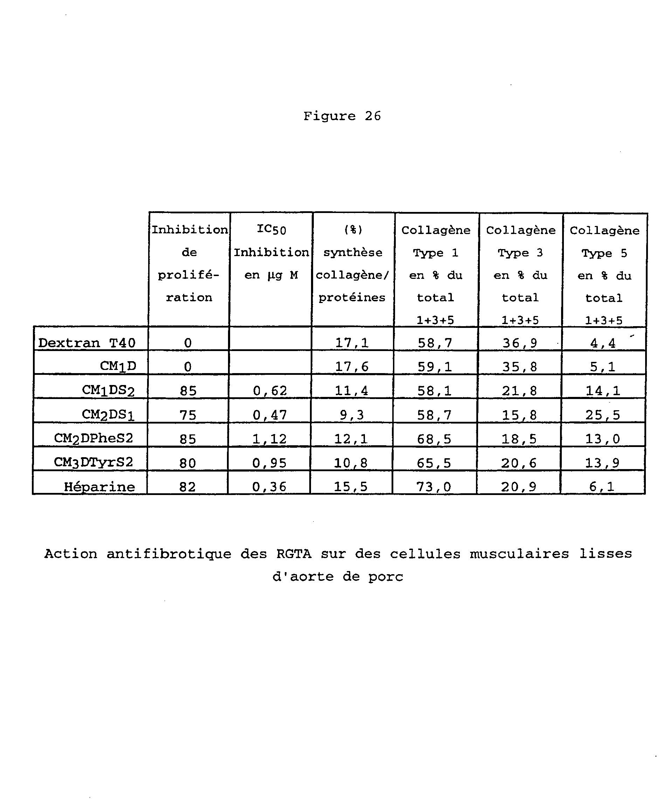

La figure 26 présente les résultats obtenus. Les RGTA exercent un effet inhibiteur sur la prolifération des cellules musculaires lisses comparable à celui de l'héparine utilisée comme molécule de référence contrôle comme l'illustrent les valeurs des deux premières colonnes. Cependant et à la différence de l'héparine, ces polymères rétablissent le phénotype de sécrétion des collagènes. En effet le taux de synthèse global des collagènes est significativement diminué en présence des RGTA 1005, 1012 et 1013. Sur le plan qualitatif, ces mêmes polymères à l'inverse de l'héparine diminue le taux de synthèse de collagènes de type I et de type III alors qu'ils augmentent la sécrétion de collagène de type V.

Ces effets confirment les propriétés antifibrotiques des polymères de l'invention.

EXEMPLE 14 : Effets des RGTA sur la régénération de la peau de rat.

Cet exemple illustre l'effet des RGTA sur la cicatrisation cutanée profonde après suture chez le rat.

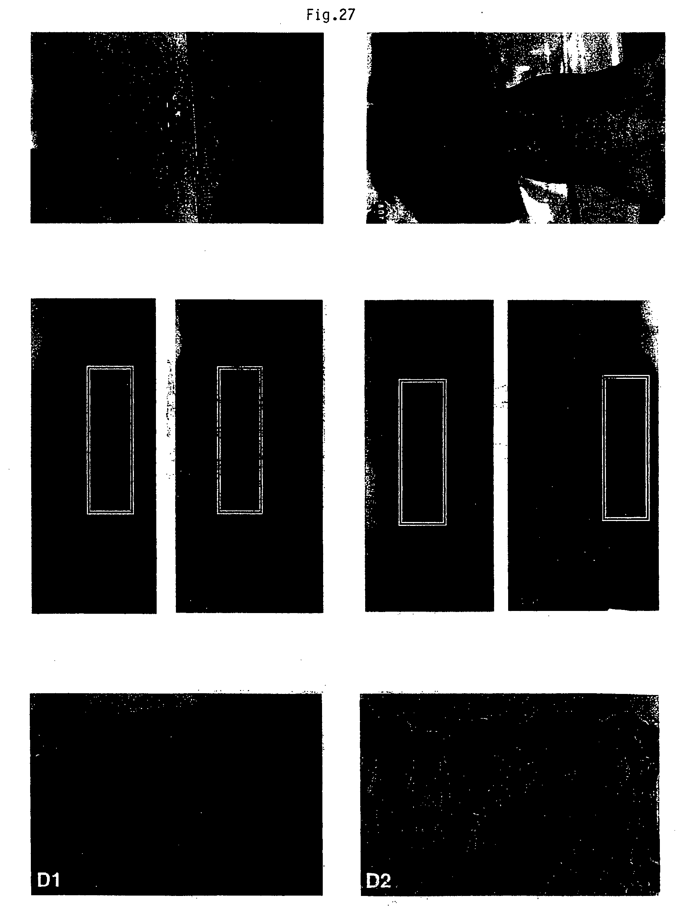

Des rats mâles Hairless de 250 grammes sont anesthésiés par une injection IM de Kétamine et de Largactil. Deux excisions de 3 cm sont pratiquées latéralement par rapport à l'axe de symétrie dorsal des animaux de chaque coté de la colonne vertébrale (figure 27). Par rapport aux animaux témoins non traités par le RGTA 1012, les animaux traités reçoivent en intra musculaire une injection d'une solution de RGTA 1012 à 1 milligrammes par kilogramme 30 minutes avant l'excision et 100 microlitres d'une solution à 100 microgrammes par millilitre en application topique sur la plaie juste avant la suture. La suture est effectuée en un seul plan par un surjet intradermique au prolène 2-0. Les rats traités et contrôles sont macrophotographiés aux jours 7, 21 et 60 après l'opération. A chacun de ces délais, trois rats de chaque série expérimentale sont euthanasiés en vue d'une étude histologique des prélèvements cutané cicatriciels.

La figure 27 montre les effets des RGTA sur la cicatrisation cutanée :

- Figure 27-A : Incision cutanée profonde réalisée jusqu'au plancher musculaire sousjacent de 3 cm de long de part et d'autre de la colonne vertébrale.
- Figure 27-B : Vue dorsale de l'animal après suture des berges de la plaie par surjet
- Figures 27-C : Aspect des cicatrices après dix jours après le traitement. C1 et C2 correspondent à un animal contrôle, traité par du sérum physiologique sans RGTA 1012. En C1 les fils n'ont pas été enlevé au contraire de la photographie C2. La cicatrice est visible et présente encore des croutes de sang coagulé en particulier au iveau des traces des aiguilles utilisées pour la suture. C3 et C4 correspondent à un animal traité par du sérum physiologique contenant du RGTA 1012. Dans le même temps les cicatrices ne sont plus visibles. Les fils de la suture sont visibles sur la figure C3 alors qu'ils ont été enlevés sur la figure C4. Sur cette photographie seul un fin liséré marque l'incision d'origine.
- Figures 27-D : Analyse histologique. D1 et D2 présentent les coupes histologiques de la peau ayant été incisée 60 jours au préalable. D1 correspond à un animal contrôle, traité par du sérum physiologique sans RGTA 1012, la figure D2 à à un animal traité par du sérum physiologique contenant le RGTA 1012. En D1, le derme sous jacent à un épithélium encore imparfaitement mature n'a pas retrouvé une structure identique à celle d'une peau normale. Le remodelage est très partiel. A l'inverse, en D2 la peau a retrouvé une structure et une organisation identique à celle d'une peau qui n'aurait jamais été lésée. La seule trace d'une cicatrice est visible dans la profondeur du derme qui laise encore détéctée une zone incomplètement remodelée. Dans ce dernier cas, il y a absence totale de cicatrice externe.

Les photographies de la figure 27 montrent au bout de 7 jours la disparition de cicatrice superficielle que les fils aient (C4) ou non (C3) été enlevés alors que les animaux contrôle laissent apparaitre une cicatrice dans les deux conditions précitées (C1 et C2).

Une analyse histologique pratiquée à 60 jours montre que les peaux des animaux non traités (D1) présentent un derme absolument pas mature alors que les animaux traités par les RGTA ne laissent apparaître qu'une légère trace dermique en profondeur. L'histologie de la peau des animaux traités (D2) est d'un aspect comparable à une peau d'un animal témoin non soumis à un quelconque processus cicatriciel. Dans ce modèle, les RGTA non seulement accélèrent la vitesse de cicatrisation du plancher cutané mais aussi et surtout permettent un remaniement du tissu cicatriciel qui aboutit à un tissu régénéré dans lequel aucune trace de fibrose n'est détectable. Ils apparaissent avec cet exemple comme des régulateurs particulièrement puissants de l'homéostasie tissulaire.

<u>Exemple 15 Effets de protection contre l'ischémie par les RGTA.</u>

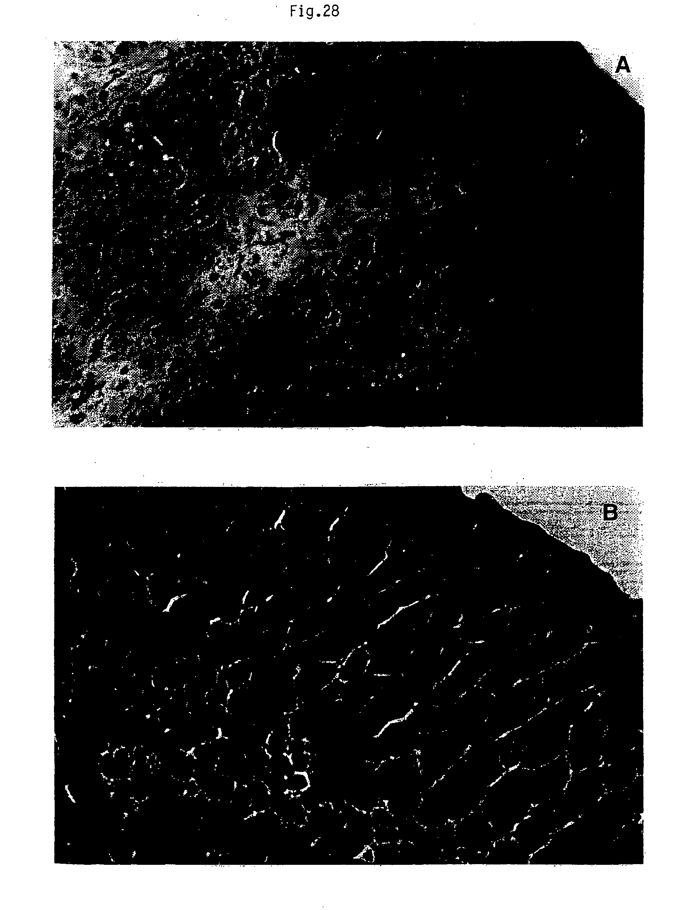

Cet exemple illustré avec le polymère RGTA 1005 démontre les effets de protection des RGTA contre la souffrance tissulaire qui permettent de conserver 80 % de la masse d'un organe en comparaison des organes non traités (figure 28). Ces effets de protection des RGTA contre les effets délétères provoqués par le stress induit par un manque d'apport d'oxygène résultant d'une ischémie des muscles sont présentés dans l'expérimentation décrite ci-dessous.

Le modèle utilisé est inspiré du modèle décrit par Hansen-Smith, F.M., Carlson, B.M., & Irwin, K.L. (1980, Revascularization of the freely grafted Extensor digitorum Longus muscle in the rat. Am. J. Anat. 158, 65-82). La procédure expérimentale consiste à sectionner le tronc neurovasculaire des muscles EDL (Extensor Digitorum Longus) sur les deux pattes arrières de rats adultes Wistar (350g) au niveau de son entrée dans le muscle et à compléter l'ischémie par une ligature des deux tendons. Une injection de 100 microlitres d'une solution de RGTA 1005 à 50 microgrammes par millilitre dans du sérum physiologique est alors effectuée directement dans un muscle EDL. Dans l'autre muscle controlatéral, le même volume de sérum physiologique sans RGTA est injecté.

7 jours après l'injection, les muscles sont retirés et examinés après préparation histologique au microscope. Dans chaque groupe de muscle, traité et non traité, un ensemble de paramètres tels le diamètre moyen du muscle, l'épaisseur de l'épimysium, de la zone périphérique, le diamètre moyen de la zone ischémiée a été mesuré à l'aide d'un objectif x10 et d'une échelle micrométrique. Le nombre de couches de fibres musculaires ayant survécu à l'ischémie dans la zone périphérique a été compté sur trente champs différents pris au hasard et observés à l'aide d'un objectif x20.

La figure 28 montre les effets protecteurs des RGTA (RGTA 1005) contre la souffrance tissulaire dans un modèle d'ischémie musculaire chez le rat. La figure 27-A et la figure 27-B montrent respectivement deux coupes histologiques de muscles de rats contrôle (27-A) et traité (27-B) par une solution de RGTA 1005. Chez le contrôle, l'ischémié a provoqué la dégénérescence des fibres musculaires à l'exception d'une couronne de fibres périphériques au contact de l'épimysium. L'administration de RGTA 1005 limite de façon considérable la dégénérescence des fibres musculaires situées en profondeur tout comme elle diminue de façon très significative la réaction inflammatoire et les dégradations qu'elle provoque. Le RGTA 10015 préserve donc les cellules des effets délétères provoqués par l'ischémie.

On observe sur la figure 28 qu'après une semaine, le diamètre moyen n'est pas modifié après traitement au RGTA (5,2 +/-0.3mm) par rapport au diamètre (5,1+/-0,2mm) d'un muscle témoin non lésé. La réaction inflammatoire dans l'épimysium est diminuée dans les muscles traités au RGTA d'une manière très significative puisque les épaisseurs des épimysiums sont de 10 +/- 5 micromètres avec traitement par le RGTA en comparaison de 85 +/- 10 micromètres sans traitement avec du RGTA ($p < 0.01$). La zone centrale ischémiée des muscles EDL non traités au RGTA présente un diamètre moyen de 4,4000 +/- 100 micromètres dans laquelle les fibres musculaires ont complètement disparu. Cette zone est entourée d'une zone périphérique de 270 +/- 50 micromètres contenant une moyenne de 3.2 +/- 0.5 couches de fibres musculaires. Le traitement au RGTA est caractérisé par une nette diminution de l'importance de la zone centrale dégénérée dont le diamètre moyen est de 3600 micromètres +/-200 ($p < 0.05$) et d'une augmentation de la zone périphérique dont l'épaisseur est de 700+/- 40 ($p < 0.05$) micromètres et qui contient 8.3 +/-1.8 $p < 0.01$) couches de fibre.

<u>EXEMPLE 16 : Effets des RGTA sur la régénération des os longs.</u>

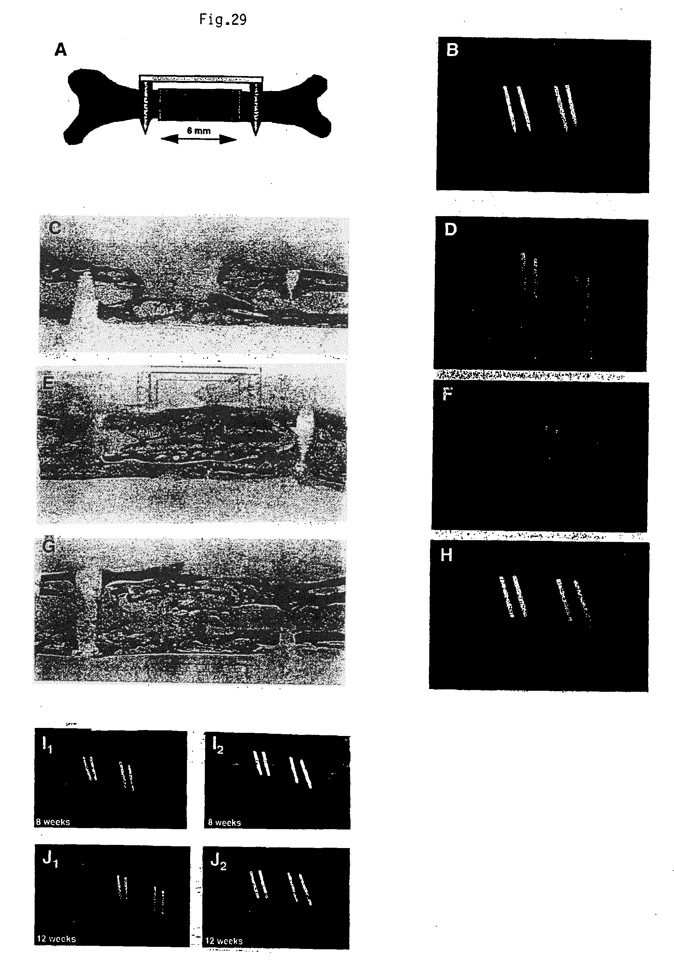

Cet exemple illustre la reconstruction d'un défaut osseux, pratiqué dans le tube diaphysaire d'un fémur de rat, à l'identique avec au bout de 8 semaines et mieux à 12 semaines une reconstitution d'un canal médullaire identique à celui d'origine et de corticales matures comme dans l'os non fracturé d'origine (figure 29).

Des rats mâles Wistar (Ico: WI (IOPS AF/Han), Iffa Credo) de 275 à 325 grammes sont utilisés. L'étude est réalisée en accord avec les recommandations de la CEE sur le travail sur animal (décret 87-848 - 04/19/1987). Sous anesthésie, par injection de pentobarbital de sodium, le fémur est abordé latéralement. Les tissus musculaires et périostiques sont désolidarisés du tube diaphysaire. Une plaque en polyéthylène de haute densité est fixée à la surface du fémur par des broches de Kirschner. Un défaut segmentaire de 5 millimètres est pratiqué au milieu de la diaphyse fémorale. Un implant et inséré à la place du défaut osseux avant suture des tissus. Cet implant correspond à une matrice osseuse allogénique déminéralisée préparée avec des diaphyses fémorales d'autres rats selon la procédure décrite par F. Blanquaert et coll. (1995, Bone 17:499-506), imprégnée ou non de RGTA 1012 par incubation dans une solution saline comprenant 100 microgrammes par millilitre de ce produit. Les animaux sont ensuite maintenus en cage sans contrainte déambulatoire. Les fémurs des animaux sont radiographiés toutes les deux semaines pendant 12 semaines avant d'être euthanasiés. Les fémurs sont alors prélevés et soumis aux traitements nécessaires à une étude histologique. Les radiographies sont étudiées en particulier au niveau de leur densitométrie par analyse d'images.

La figure 29 montre les effets des RGTA sur la régénération des os longs et rapporte plus particulièrement les études histologiques et radiographiques de fémurs de rat traités ou non par du RGTA 1015.

La figure 28-A présente le modèle de défaut pratiqué dans la diaphyse fémorale.

Les figures 28-B, 28-D, 28-F et 28-H représentent des radiographies de fémur de différents groupes expérimentaux. Les figures 28-C, 28-E et 28-G représentent des coupes histologiques des pièces opératoires correspondant respectivement aux spécimens présentés en 28-D, 28-F et 28-H.

Sur les figures 28-C et 28-D, on observe que le défaut fémoral n'a reçu aucun traitement particulier. Il n'y a pas eu en 12 semaines de phénomène cicatriciel et le fût osseux n'est pas reconstitué.

Sur les figures 28-E et 28-F, on observe que le défaut osseux a été comblé par de la matrice osseuse déminéralisée. Dans ce cas, le comblement est effectué mais aucun remaniement n'est observable. La structure de comblement ne présente pas l'organisation d'un os long traditionnel.

Sur les figures 28-G et 28-H, on observe que le défaut osseux a été comblé par de la matrice osseuse déminéralisée imprégnée dans une solution de RGTA 1015. Dans ce cas, la structure des tissus de comblement correspond à celle d'un os normal. L'os compact cortical est en relation avec la zone non lésée dans laquelle on peut voir l'empreinte des vis de fixation. Cette partie délimite une cavité remplie de moelle osseuse en continuité avec la cavité medullaire d'origine.

Les figures 28-I1, 28-I2, 28-J1 et 28-J2 montrent l'effet du RGTA 1015 sur la vitesse de reformation de l'os long. Les radiographies I1 et I2 sont prises à 8 semaines, les radiographies J1 et J2 à 12 semaines. I1 et J1 correspondent au traitement présenté en 28-E et 28-F où le défaut osseux a été seulement comblé par de la matrice osseuse déminéralisée san. RGTA. I2 et J2 correspondent au traitement présenté en 28-G et 28-H où le défaut osseux a été comblé par de la matrice osseuse déminéralisée imprégnée dans une solution de RGTA 1015. Ces radiographies montrent une accélération de la cicatrisation et surtoute de la maturation des os reformés surtout en ce qui concerne une corticalisation nette (I2 et J2) non détectable en I1 et J1. Le RGTA 1015 agit comme un agent de régénération qui permet de reconstituer, dans un délai accéléré, une structure osseuse d'os long identique à la structure de l'os d'origine.

Ainsi donc et de façon étonnante, le RGTA 1012, imprégné dans la matrice osseuse déminéralisée en comparaison d'une matrice imprégnée dans du sérum physiologique sans le polymère induit une accélération tout à fait significative sur les processus de remodelage et de maturation osseuse. Cet effet se manifeste par l'apparition de nouvelles corticales après seulement 8 semaines alors que dans les conditions contrôles ce phénomène ne se déroule pas. Après 12 semaines, six des sept animaux traités par l'association avec du RGTA 1012 présentent à l'étude radiologique les évidences d'une union complète du défaut avec la reformation de corticales épaisses et délimitées tandis que sans le $CM_1DS_2$ seule l'union est observée avec des images radiologiques d'os immature sans corticalisation. Une étude quantitative en analyse d'image des radiographies confirme ces observations. Cette étude précise par ailleurs que le profil des os traités par le RGTA 1012 est comparable à celui d'un os normal, à la seule différence que le matériel osseux est d'une densité relativement plus faible dans les délais expérimentaux considérés de 12 semaines. La projection de la densité de l'os néoformé dans le défaut d'origine révèle que le traitement par le polymère RGTA 1012 induit un remodelage tissulaire à travers la reformation de corticale et d'un canal médullaire.

Les études histologiques corrèlent et confirment les résultats établis par analyse d'image des données radiologiques. Ces études histologiques démontrent la présence de nouvelles corticales constituée d'os compact avec encore quelques régions de moelle et la présence d'un canal médullaire en continuité avec le fut diaphysaire d'origine, rempli de nouvelle moelle osseuse. Les fémurs traités sans RGTA 1012 ne montrent aucune figure de maturation. L'os reconstitué ne présente pas d'organisation particulière. Il est constitué d'un mélange hétérogène d'os compact et de tissu médullaire sans délimitation particulière du territoire.

Ces résultats illustre les effets ostéoinducteurs des polymères de l'invention sur leur capacité à induire non seulement la réparation des os longs mais aussi et surtout leur potentiel à réguler l'homéostasie des tissus régénérés au niveau de leur masse comme de leur remodelage.

Les propriétés des RGTA comme agent régulateur de l'homéostasie des tissus osseux, c'est à dire sur la masse tissulaire, sa fonctionnalité et son remodelage sont confirmées par l'exemple 17 ci-après.

EXEMPLE 17 : Effets des RGTA, sur le remaniement et la protection de la masse osseuse dans un modèle de parodontie aigue chez le hamster.

Le modèle utilisé dans cet exemple concerne le remaniement osseux de la mandibule de Hamster.

Une parodontie est induite chez des Hamsters dorés de Syrie (centre d'élevage Dépré, références HSM 41/50) après deux mois d'une alimentation hyperglucidique. Le régime administré se compose de saccharose (56 %), lait en poudre écrémé (28 %), farine complète de blé (6 %), levure de bière (4 %, luzerne en poudre (3 %), poudre de foie (1 %) et chlorure de sodium (2 %).

Les animaux sont répartis en groupes expérimentaux. 14 animaux constituent les groupes témoins qui reçoivent une alimentation normale à base de biscuit et de croquette. 24 animaux constituent les groupes expérimentaux soumis au régime hyperglucidique. Ils développent une parodontie chronique établie au bout de deux mois. Les groupes expérimentaux reçoivent après ce délai, chaque semaine pendant 3 semaines, une injection intramusculaire de RGTA 1005 à différentes doses comprises entre 0,1 et 15 milligrammes par kilogramme sous un volume de 0,5 millilitres de sérum physiologique tamponné. Les groupes contrôles reçoivent une injection de sérum physiologique sans RGTA 1005 (SHAM). Le poids des animaux est relevé chaque semaine. Un mois après chaque type de traitement, les animaux sont sacrifiés, les mandibules prélevées et préparées pour une étude histologiques. Les inclusions des pièces opératoires sont réalisées dans du méthyl métacrylate stabilisé.

Dans ce modèle de parodontite, seuls 200 micromètres sont exploitables en hauteur pour chaque hémimandibule. Le système est standardisé de façon étudier toujours la même séquence de coupes à une profondeur définie et repérée par le tissu osseux compris entre les racines des deux premières molaires du coté lingual.

La maladie parodontale, au niveau de ces molaires se manifeste par l'apparition d'une poche parodontale qui délimite un volume rempli de plaque bactérienne. Cette maladie entraîne une destruction importante de l'os parodontal qui se caractérise par une importante résorption ostéoclastique et une réduction des surfaces osseuse en apposition c'est à dire à une phase d'ostéosynthèse.

La résorption osseuse est quantifiée en mesurant les zones de contact entre l'os et les ostéoclastes (Oc). Ce sont des cellules géantes colorées en bleu par le bleu de toluidine. L'apposition quant à elle se caractérise par une bande de tissu ostéoïde, identifiée par une coloration bleu atténuée recouverte d'ostéoblastes. A l'inverse des OC, les ostéoblastes sont des cellules de petite taille, de forme cubique et mononuclées. Les quantifications de ces phénomènes se font à l'aide d'une station d'imagerie assistée d'un logiciel de traitement d'images.

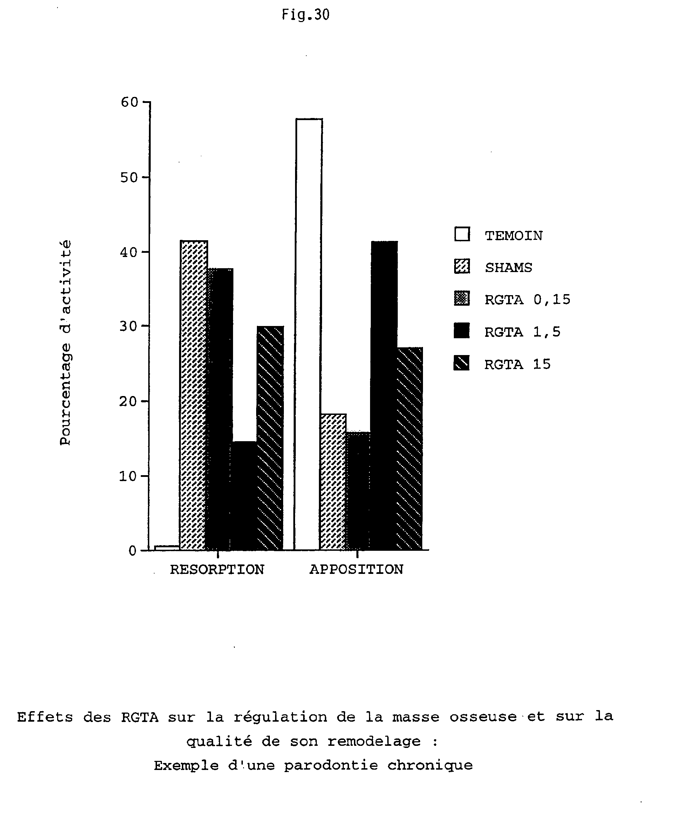

La figure 30 présente les quantifications obtenues dans les différentes conditions expérimentales. Chez les animaux témoins qui n'ont pas développé la maladie, l'activité de la résorption est quasiment nulle alors que celle de l'apposition est importante. Chez les animaux non traités (SHAM), la maladie est fortement développée et se caractérise par une forte résorption et un faible taux d'apposition. Chez les animaux traités par du $CM_1DS_2$, et en particulier à la dose de 1,5 milligrammes par kilogramme, le taux de résorption est fortement diminué et est associé à une augmentation très importante du taux d'apposition qui atteint près de 80 % du taux établi chez les témoins.

Ces effets sont obtenus par injection intramusculaire. Ils montrent que les RGTA régulent la masse du tissu osseux en rééquilibrant la balance du remodelage entre la résorption et l'apposition.

EXEMPLE 18 : Données pharmacocinétiques montrant les propriétés des RGTA pour vectoriser des molécules vers des tissus lésés.

Cet exemple illustre la capacité que présentent les polymères de l'invention à se fixer de façon spécifique et à se concentrer au niveau des sites de souffrance tissulaire.

Dans le modèle de la régénération du muscle soumis à un écrasement, décrit dans l'exemple 7, une lésion est pratiquée sur l'EDL de la patte gauche des animaux. Chaque animal reçoit une injection intraveineuse de 2 $10^6$ cpm de RGTA 1012 radiomarqué au tritium par les soins de la société SibTech, Inc. (NY, USA). L'activité spécifique de ce traceur est de 20 milliCurie par milligramme.

A différents temps post-opératoires, les muscles EDL lésés des pattes gauches et ceux non lésés des pattes droites sont prélevés et congelés dans l'azote liquide. La réalisation de coupes histologiques en congélation permet grâce à un beta imager (Société Biospace) de mesurer la quantité de produit radiomarqué fixé au niveau des coupes des tissus étudiés. Les résultats rapportés dans le tableau V ci-dessous montre que les muscles lésés concentrent dès 24 heures une quantité de produit radioactif environ 5 fois plus élevée que les muscles non lésés dont le niveau de marquage ne diffère pas du bruit de fond de l'appareil.

Tableau V

| | cpm de RGTA 1012 fixé au niveau des tissus | | |
|---|---|---|---|
| Temps post-opératoires | 24 heures | 48 heures | 96 heures |
| Tissu musculaire lésé | 11800+/-890 | 10150+/-1020 | 9000+/-790 |
| Tissu musculaire témoin controlatéral | 2060+/-530 | 1980+/-390 | 1870+/-640 |
| Bruit de fond de l'enregistrement | 1800+/-160 | 1780+/-210 | 1950+/-180 |

Ces résultats démontrent les capacités d'autociblage des polymères de l'invention qui se concentrent spécifiquement au niveau des tissus présentant une souffrance ou une lésion. Une propriété particulièrement intéressante de ces polymères réside donc également dans leur capacité de vectorisation d'un principe médicamenteux ou de diagnostic.

# EP 1 117 695 B1

<u>Exemple 19 Effets du RGTA en parodontie et sur la masse osseuse.</u>

Dans le domaine de la parodontie, une étude macroscopique de la perte d'os alvéolaire a été menée sur la parodontite du hamster.

Après une période d'induction de la maladie de 2 mois, des animaux (n=20) ont été traités par voie IM pendant 2 autres mois, sans agir sur la cause initiale de la maladie, c'est à dire la composante bactérienne. D'autres animaux sont restés non traités (n=20). Ces 2 groupes ont été comparés à des hamsters sains (pas de parodontite) (n=12).

A la fin de la période expérimentale, les maxillaires supérieurs ont été décharnés pour mettre en évidence la perte osseuse. La zone de la 1ère molaire a été photographiée de façon standardisée. Sur chaque photo, une ligne de référence a été tracée, elle correspond à la jonction émail-cément. Puis a été tracée une seconde ligne qui épouse les contours de la crête osseuse. Ces deux lignes ont été réunies en avant et en arrière de la 1ère molaire. Cette surface a été mesurée.

Il faut remarquer que chez les témoins, il y a une zone de dénudation de la racine qui correspond :

- à une zone d'insertion fibreuse, physiologique, qui ancre la gencive sur la racine,
- à une perte de hauteur d'os qui se produit en rapport avec le vieillissement des animaux.

Cette dénudation représente chez nos animaux 0,96 mm$^2$.

Chez les animaux atteints, la perte d'os est de 1,34 mm$^2$, ce qui inclut la zone physiologique initiale (qui se détruit au cours de la parodontite) et une partie de la perte due au vieillissement. Néanmoins, nous pouvons considérer que la maladie a provoqué une perte d'os de l'ordre de 0,38 mm$^2$ (différence : p<0,0001).

Chez les traités, il y a toujours une zone incompressible (idem témoins). La dénudation de la racine représente 1,02 mm$^2$ chez ces animaux. Il y a donc un déficit net par rapport aux témoins de 0,06 mm$^2$ (différence non significative), et une amélioration de 0,32 mm$^2$ par rapport aux non traités (p = 0,0005).

## Revendications

1. Polymère biocompatible constitué par un enchaînement de motifs identiques ou différents, de formule générale (II) suivante :

$$A_a\, X_x\, Y_y\, Z_z$$

dans laquelle :

- A est un monomère de glucose;
- X est $CH_2COOH$ ou $CH_2COO^-Na^+$,
- Y est $-SO_3H$ ou $-SO_3^-Na^+$,
- Z représente des groupements chimiques fonctionnels à l'exception de la benzylamine et de la benzylamine sulfonate, identiques ou différents et différents de X et Y, choisis parmi les acides aminés, les acides gras, les alcools gras, les céramides, des dérivés de ceux-ci, les séquences nucléotidiques d'adressage, les agents thérapeutiques et les agents de diagnostic,

les groupements X et Y étant fixés sur le monomère A,

- a représente le nombre de monomères A, la masse desdits polymères de formule AaXxYy est supérieure à 5 000 daltons
- x représente le taux de substitution de l'ensemble des monomères A par les groupements X, lequel est compris entre 20 et 150%,
- y représente le taux de substitution de l'ensemble des monomères A par les groupements Y, qui est compris entre 30 et 150%,
- z représente le taux de substitution de l'ensemble des monomères A par des groupements Z, où $0\% < z \leq 50\%$.

2. Polymère biocompatible selon la revendication 1, dans lequel le groupement Z est choisi parmi un anti-inflammatoire, un anti-microbien, un antibiotique, une enzyme, un facteur de croissance.

3. Polymère biocompatible selon l'une quelconque des revendications précédentes, dans lequel les groupements Z sont fixés par covalence directement sur les monomères A ou fixés par covalence sur les groupements X et/ou Y.

4. Polymère biocompatible selon l'une quelconque des revendications 1 à 3, dans lequel les groupements Z sont conjugués aux polymères par des liaisons autres que covalentes.

**5.** Polymère biocompatible selon la revendication 4, dans lequel les groupements Z sont conjugués aux polymères par des interactions ioniques ou hydrophiles.

**6.** Composition pharmaceutique ou de diagnostic, comprenant au moins un polymère selon l'une quelconque des revendications 1 à 5 associé dans ladite composition avec un véhicule pharmaceutiquement acceptable.

**7.** Composition pharmaceutique ou de diagnostic selon la revendication 6, dans laquelle le dosage permet une administration de 0,001 à 1 milligramme dudit polymère par centimètre carré ou de 0,005 à 1 milligramme de polymère par centimètre cube de tissu à traiter.

**8.** Composition pharmaceutique ou de diagnostic selon la revendication 6, contenant entre 0,01 et 10 milligrammes dudit polymère par millilitre de solution physiologique de dissolution.

**9.** Composition pharmaceutique ou de diagnostic selon la revendication 6, dans laquelle le dosage permet une administration par voie systémique, intra-péritonéale, intra-oculaire, dans le liquide céphalorachidien, dans le liquide intracochléaire ou dans tous fluides péri ou intra tissulaires, de 0,1 et 100 milligrammes dudit polymère par kilogramme de poids d'homme ou d'animal à traiter.

**10.** Composition pharmaceutique ou de diagnostic selon la revendication 6, dans laquelle le dosage permet une administration par voie intramusculaire, comprise entre 0,2 et 500 milligrammes dudit polymère par kilogramme de poids d'homme ou d'animal à traiter.

**11.** Composition pharmaceutique ou de diagnostic selon la revendication 6, dans laquelle le dosage permet une administration per os comprise entre 1 et 5000 milligrammes dudit polymère par kilogramme de poids d'homme ou d'animal à traiter.

**12.** Composition pharmaceutique selon l'une quelconque des revendications 6 à 11, présentant une activité anticoagulante inférieure à 50 UI/mg.

**13.** Utilisation d'un polymère biocompatible selon l'une quelconque des revendications 1 à 5 comme médicament présentant une activité réparatrice et/ou cicatrisante des tissus musculaires, nerveux et du tractus digestif et de l'os long.

**14.** Utilisation selon la revendication 13, dans laquelle ledit médicament est destiné à la cicatrisation des tissus musculaires, nerveux et du tractus digestif.

**15.** Utilisation selon la revendication 13, dans laquelle ledit médicament est destiné à la cicatrisation cutanée.

**16.** Utilisation selon la revendication 13, dans laquelle ledit médicament est destiné à la cicatrisation de la cornée.

**17.** Utilisation selon la revendication 13, dans laquelle ledit médicament est destiné à la cicatrisation de l'os plat.

**18.** Utilisation selon la revendication 13, dans laquelle ledit médicament est destiné à la cicatrisation de l'os long.

**Patentansprüche**

**1.** Biokompatibles Polymer, bestehend aus einer Aneinanderreihung von gleichen oder unterschiedlichen Motiven, der folgenden allgemeinen Formel (II):

$$A_a\, X_x\, Y_y\, Z_z,$$

wobei:

- A ein Glucose-Monomer ist,
- X $CH_2COOH$ oder $CH_2COO^-Na^+$ ist,
- Y $-SO_3H$ oder $-SO_3^-Na^+$ ist,

- Z für chemische funktionelle Gruppen mit Ausnahme von Benzylamin und Benzylaminsulfonat steht, die gleich oder unterschiedlich sowie verschieden von X und Y sind, ausgewählt aus Aminosäuren, Fettsäuren, Fettalkoholen, Ceramiden, deren Derivaten, Zielsteuerungs-Nukleotidsequenzen, therapeutischen Mitteln und diagnostischen Mitteln,

wobei die Gruppen X und Y an das Monomer A gebunden sind,

- a für die Anzahl der Monomere A steht, wobei die Masse der Polymere der Formel AaXxYy größer als 5000 Dalton ist
- x für den Substitutionsgrad der gesamten Monomere A durch die Gruppen X steht, der zwischen 20 und 150% beträgt,
- y für den Substitutionsgrad der gesamten Monomere A durch die Gruppen Y steht, der zwischen 30 und 150% beträgt,
- z für den Substitutionsgrad der gesamten Monomere A durch die Gruppen Z mit 0% < z ≤ 50% steht.

2. Biokompatibles Polymer nach Anspruch 1, wobei die Gruppe Z aus einem Entzündungshemmer, einem Mikrobizid, einem Antibiotikum, einem Enzym, einem Wachstumsfaktor ausgewählt wird.

3. Biokompatibles Polymer nach einem der vorhergehenden Ansprüche, wobei die Gruppen Z kovalent direkt an die Monomere A gebunden sind oder kovalent an die Gruppen X und/oder Y gebunden sind.

4. Biokompatibles Polymer nach einem der Ansprüche 1 bis 3, wobei die Gruppen Z an die Polymere über andere als kovalente Bindungen konjugiert sind.

5. Biokompatibles Polymer nach Anspruch 4, wobei die Gruppen Z an die Polymere über ionische oder hydrophile Wechselwirkungen konjugiert sind.

6. Pharmazeutische oder diagnostische Zusammensetzung, umfassend mindestens ein Polymer nach einem der Ansprüche 1 bis 5, das in der Zusammensetzung mit einem pharmazeutisch annehmbaren Vehikel vereinigt ist.

7. Pharmazeutische oder diagnostische Zusammensetzung nach Anspruch 6, wobei die Dosierung eine Verabreichung von 0,001 bis 1 Milligramm des Polymers pro Quadratzentimeter oder von 0,005 bis 1 Milligramm Polymer pro Kubikzentimeter des zu behandelnden Gewebes gestattet.

8. Pharmazeutische oder diagnostische Zusammensetzung nach Anspruch 6, die zwischen 0,01 und 10 Milligramm des Polymers pro Milliliter physiologisches Lösungsmittel enthält.

9. Pharmazeutische oder diagnostische Zusammensetzung nach Anspruch 6, wobei die Dosierung eine Verabreichung auf systemischem, intraperitonealem, intraokularem Weg, in die Zerebrospinalflüssigkeit, in die intracochleäre Flüssigkeit oder in alle Gewebe-umgebenden oder innerhalb von Geweben befindlichen Flüssigkeiten von 0,1 und 100 Milligramm des Polymers pro Kilogramm Gewicht des zu behandelnden Menschen oder Tieres gestattet.

10. Pharmazeutische oder diagnostische Zusammensetzung nach Anspruch 6, wobei die Dosierung eine Verabreichung auf intramuskulärem Weg von zwischen 0,2 und 500 Milligramm des Polymers pro Kilogramm Gewicht des zu behandelnden Menschen oder Tieres gestattet.

11. Pharmazeutische oder diagnostische Zusammensetzung nach Anspruch 6, wobei die Dosierung eine Verabreichung per os von zwischen 1 und 5000 Milligramm des Polymers pro Kilogramm Gewicht des zu behandelnden Menschen oder Tieres gestattet.

12. Pharmazeutische Zusammensetzung nach einem der Ansprüche 6 bis 11, die eine antikoagulierende Aktivität unter 50 IU/mg aufweist.

13. Verwendung eines biokompatiblen Polymers nach einem der Ansprüche 1 bis 5 als Arzneimittel mit einer restaurativen und/oder wundheilenden Aktivität auf Muskel-, Nerven- und Verdauungstraktgewebe sowie Röhrenknochen.

14. Verwendung nach Anspruch 13, wobei das Arzneimittel für die Wundheilung von Muskel-, Nerven- und Verdauungstraktgeweben bestimmt ist.

15. Verwendung nach Anspruch 13, wobei das Arzneimittel für die Wundheilung der Haut bestimmt ist.

**16.** Verwendung nach Anspruch 13, wobei das Arzneimittel für die Wundheilung der Hornhaut bestimmt ist.

**17.** Verwendung nach Anspruch 13, wobei das Arzneimittel für die Wundheilung flacher Knochen bestimmt ist.

**18.** Verwendung nach Anspruch 13, wobei das Arzneimittel für die Wundheilung von Röhrenknochen bestimmt ist.


**Claims**

**1.** Biocompatible polymer constituted by a sequence of identical or different components of the following general formula (II)

$$AaXxYyZz$$

wherein:

A is a glucose monomer,
X is $CH_2COOH$ or $CH_2COO^-Na^+$,
Y is $-SO_3H$ or $-SO_3^-Na^+$,
Z represents functional chemical groups, except benzylamine and sulfonate benzylamine, which are different from X and Y, selected from amino acids, fatty acids, fatty alcohols, ceramides or derivatives thereof, nucleotide addressing sequences, therapeutic agents and diagnostic agents,
the groups X, Y being fixed on the monomer A ,
a represents the number of monomers A , the mass of said polymers of formula AaXxYy being greater than 5,000 Daltons,
x represents a substitution rate of all the monomers A by X groups, which is between 20 and 150%, and
y represents a substitution rate of all the monomers A by Y groups, which is between 30 and 150%,
Z represents the substitution rate of the all monomers A by Z groups, wherein $0 < z \leq 50\%$.

**2.** A biocompatible polymer according to claim 1, wherein the group Z is selected from an anti-inflammatory, antimicrobial, antibiotic, enzyme and a growth factor.

**3.** A biocompatible polymer according to any of the preceding claims, wherein the groups Z are covalently bonded directly to the monomers A or covalently bonded to the X and/or Y groups.

**4.** A biocompatible polymer according to any of claim 1 to 3, wherein the groups Z are conjugated to the polymers by bonds other than covalent bonds.

**5.** A biocompatible polymer according to any of claim 1 to 4, wherein the groups Z are conjugated to the polymers by ionic or hydrophilic interaction.

**6.** Pharmaceutical or diagnostic composition comprising a polymer according any of claim 1 to 5 associated with an acceptable pharmaceutical vehicle.

**7.** Pharmaceutical or diagnostic composition according to claim 6, wherein the dosage allow an administration of 0,001 to 1 milligrams of said polymer per square centimeter or of 0,005 to 1 milligrams of polymer per cubic centimeters.

**8.** Pharmaceutical or diagnostic composition according to claim 6 containing between 0,01 and 1 milligrams of said polymer per milliliter of the dilution physiological solution.

**9.** Pharmaceutical or diagnostic composition according to claim 6, wherein the dosage allows an administration via the systemic route, via the intraperitoneal route, via the intraocular route, into the cerebrospinal fluid, into the intracochlear fluid or into any peritissular or intratissular fluids, of between 0,1 and 100 milligrams of said polymer per kilogram of weight of the human or animal to be treated.

**10.** Pharmaceutical or diagnostic composition according to claim 6, wherein the dosage allows administration via the intramuscular route, of between 0,2 and 500 milligrams of said polymer per kilogram of weight of the human or animal to be treated.

11. Pharmaceutical or diagnostic composition according to claim 6, wherein the dosage allow oral administration of between about 1 and about 5000 milligrams of polymer per kilogram of weight of the human or animal to be treated.

12. Pharmaceutical or diagnostic composition according any of claims 6 to 11, having an anticoagulant activity lower than 50 IU/milligram.

13. Use of a compatible biopolymer according to any of claim 1 to 5 as a medicament presenting a repairing activity and /or cicatricial of muscular, nervous tissues and digestive tract and long bone.

14. Use of a compatible biopolymer according to claim 13 wherein said drug is intended to muscular, nervous tissues and digestive tract cicatrization.

15. Use of a compatible biopolymer according to claim 13 wherein said drug is intended to cutaneous cicatrization.

16. Use of a compatible biopolymer according to claim 13 wherein said drug is intended to cornea cicatrization.

17. Use of a compatible biopolymer according to claim 13 wherein said drug is intended to flat bone cicatrization.

18. Use of a compatible biopolymer according to claim 13 wherein said drug is intended to long bone cicatrization.

Fig.1

Les acides -β-malique

**Fig.2**

$$CH_3$$
$$COO-CH-CH_2-CH_3$$
$$CH - O$$
$$CH_2- C=O \qquad 1.MLABu$$

malolactonate de 2-butyle

$$COO-CH_2-C_6H_5$$
$$CH - O$$
$$CH_2- C=O \qquad 2.MLABe$$

malolactonate de benzyle

$$COO-CH_2-CH=CH_2$$
$$CH - O$$
$$CH_2- C=O \qquad 3.MLAAl$$

malolactonate d'allyle

Structure des 3 β-lactones.

FIG.3

Synthèse du malolactonate d'alkyle à partir de l'acide DL-aspartique.

R = -CH(CH3)-CH2-CH3, malolactonate de benzyle ou -CH(CH3)-CH2-CH3 (malolactonate de 2-butyle) ou -CH2-CH=CH2 (malolactonate d'allyle).

Fig.4

Schéma récapitulatif de la synthèse de dérivés du poly(acide β - malique).

Figure 5

Monomères A

Monomères de type A-X

Monomères de type A-Y

Monomères de type A-Z

37

Figure 6

| Référence | | X =<br>% COO⁻ | Y =<br>% SO3- | Z |
|---|---|---|---|---|
| RGTA 1000 | $CM_1D$ | 48,98 | 0 | 0 |
| RGTA 1001 | $CM_1DS_{0,5}$ | 48,5 | 13,1 | 0 |
| RGTA 1002 | $CM_1DS_{0,75}$ | 44,7 | 25,3 | 0 |
| RGTA 1003 | $CM_1DS_1$ | 40,9 | 40,6 | 0 |
| RGTA 1004 | $CM_1DS_{1,5}$ | 31,7 | 56,5 | 0 |
| RGTA 1005 | $CM_1DS_2$ | 26,3 | 82,3 | 0 |
| RGTA 1006 | $CM_1DSex$ | 19,1 | 94,4 | 0 |
| RGTA 1007 | $CM_2D$ | 91,8 | 0 | 0 |
| RGTA 1008 | $CM_2DS_{0,5}$ | 84,9 | 18,4 | 0 |
| RGTA 1009 | $CM_2DS_{0,75}$ | 63,7 | 30,3 | 0 |
| RGTA 1010 | $CM_2DS_1$ | 61,1 | 37,3 | 0 |
| RGTA 1011 | $CM_2DS_{1,5}$ | 57,8 | 44,6 | 0 |
| RGTA 1012 | $CM_2DS_2$ | 55,0 | 55,7 | 0 |
| RGTA 1013 | $CM_2DSex$ | 22,6 | 58,5 | 0 |
| RGTA 1014 | $CM_3D$ | 118,3 | 0 | 0 |
| RGTA 1015 | $CM_3DS_{0,5}$ | 102,7 | 15,6 | 0 |
| RGTA 1016 | $CM_3DS_{0,75}$ | 70,9 | 36,5 | 0 |
| RGTA 1017 | $CM_3DS_1$ | 87,3 | 42,0 | 0 |
| RGTA 1018 | $CM_3DS_{1,5}$ | 71,2 | 55,0 | 0 |
| RGTA 1019 | $CM_3DS_2$ | 68,9 | 57,3 | 0 |
| RGTA 1020 | $CM_4D$ | 154,0 | 0 | 0 |
| RGTA 1021 | $CM_4DS_{0,5}$ | 114,8 | 8,9 | 0 |
| RGTA 1022 | $CM_4DS_1$ | 104,9 | 24,6 | 0 |
| RGTA 1023 | $CM_4DS_2$ | 72,2 | 51,8 | 0 |
| RGTA 0040 | DS commercial | 0 | 97,6 | 0 |
| RGTA 1024 | $DS_{0,5}$ équiv | 0 | 103,0 | 0 |
| RGTA 1025 | $DS_{0,25}$ équiv | 0 | 41,4 | 0 |
| RGTA 1026 | $DS_{0,125}$ équiv | 0 | 23,5 | 0 |

Tableau rapportant pour chacun des RGTA référencés et correspondant aux polymères de type $CM_nDS_m$, les pourcentages de définition en groupements libres X et Y. Dans cette série Z = rien.

Figure 7

CM$_2$DPhSS$_1$

A partir du p-phénylsulfonate

Figure 8

$CM_2DES_1$

A partir de l'éthylènediamine

Figure 9

CM3DPheS2

A partir de la phénylalanine méthylester

CM3DTyrS2

A partir de la tyrosine méthylester

Figure 10

CM$_1$DPalmS$_1$

A partir de l'acide palmitique (C16)

CM$_1$DOleicS$_1$

A partir du chlorure oléique (C18;9)

Figure 11

| Référence | polymères | Activité anti-coagulante en UI | Référence | polymères | Activité anti-coagulante en UI |
|---|---|---|---|---|---|
| Hép | Héparine | 176 | RGTA 1013 | $CM_2DSex$ | <50 |
| RGTA 2010 | Pcoo- | <50 | RGTA 1014 | $CM_3D$ | <50 |
| RGTA 2011 | P1S | <50 | RGTA 1015 | $CM_3DS_{0,5}$ | <50 |
| RGTA 2012 | P2S | <50 | RGTA 1016 | $CM_3DS_1$ | <50 |
| RGTA 1000 | $CM_1D$ | <50 | RGTA 1017 | $CM_3DS_{1,5}$ | <50 |
| RGTA 1001 | $CM_1DS_{0,5}$ | <50 | RGTA 1019 | $CM_3DS_2$ | <50 |
| RGTA 1002 | $CM_1DS_{0,75}$ | <50 | RGTA 1110 | $CM_2DPhSS1$ | <50 |
| RGTA 1003 | $CM_1DS_1$ | <50 | RGTA 1111 | $CM_2DES1$ | <50 |
| RGTA 1004 | $CM_1DS_{1,5}$ | <50 | RGTA 1112 | $CM_2DPheS2$ | <50 |
| RGTA 1005 | $CM_1DS_2$ | <50 | RGTA 1113 | $CM_3DTyrS2$ | <50 |
| RGTA 1006 | $CM_1DSex$ | <50 | RGTA 1114 | $CM_1DPalmS1$ | <50 |
| RGTA 1007 | $CM_2D$ | <50 | RGTA 1115 | $CM_1DOléicS1$ | <50 |
| RGTA 1008 | $CM_2DS_{0,5}$ | <50 | RGTA 0040 | DS commercial | <50 |
| RGTA 1009 | $CM_2DS_{0,75}$ | <50 | RGTA 1024 | $DS_{0,5}$ équiv | <50 |
| RGTA 1010 | $CM_2DS_1$ | <50 | RGTA 1025 | $DS_{0,25}$ équiv | <50 |
| RGTA 1011 | $CM_2DS_{1,5}$ | <50 | RGTA 1026 | $DS_{0,125}$ équiv | <50 |
| RGTA 1012 | $CM_2DS_2$ | <50 | RGTA 0001 | Dextran T40 | <50 |

Activités anticoagulantes des polymères

Figure 12

| Traitement | 20° C | 20° C | 20° C | 20° C | 37° C | 37° C |
|---|---|---|---|---|---|---|
| Valeur de l'ED50 à | 0 jour | 1 jour | 7 jours | 15 jours | 1 jour | 7 jours |
| FGF1 seul | 6 | 8 | 14 | >20 | 7 | >20 |
| FGF1 +Héparine | 0,8 | 1,2 | 6 | 16 | 1,4 | 15 |
| FGF1 + Dextran T40 | 6 | 10 | >20 | >20 | 7 | >20 |
| FGF1 + DS commercial | 6 | 8 | >20 | >20 | 7 | >20 |
| FGF1 + $DS_{0,5}$ équiv | 6 | 8 | >20 | >20 | 7 | >20 |
| FGF1 + $DS_{0,125}$ équiv | 6 | 10 | >20 | >20 | 7 | >20 |
| Pcoo- | 8 | >20 | >20 | >20 | 18 | >20 |
| P1S | 3 | 6 | 10 | 17 | 5 | 15 |
| P2S | 1 | 3 | 9 | 14 | 3 | 11 |
| FGF1 + $CM_1D$ | 6 | 9 | >20 | >20 | 7 | >20 |
| FGF1 + CM2D | 6 | 7 | >20 | >20 | 7 | >20 |
| FGF1 + CM1DS2 | 0,5 | 1,1 | 6 | 17 | 2,1 | 16 |
| FGF1 + CM2DS2 | 2 | 8 | 15 | >20 | 5 | >20 |
| FGF1 + $CM_2DPhS$ | 8 | 15 | >20 | >20 | 8 | >20 |
| FGF1 + $CM_2DPhSS1$ | 2 | 6 | 18 | >20 | 3 | 14 |
| FGF1 + $CM_2DES1$ | 1 | 3 | 8 | 17 | 9 | >20 |
| FGF1 + $CM_2DPheS2$ | 0,9 | 2 | 4 | 13 | 8 | 17 |
| FGF1 + $CM_3DTyrS2$ | 3 | 5 | >20 | >20 | 9 | >20 |
| FGF1 + $CM_1DPalmS1$ | 4 | 4 | 16 | >20 | 14 | >20 |

Effets stabilisateurs des polymères sur le FGF1

Figure 13

| Références polymères | Conditions | concentrations ($\mu$g/ml) | ED50 FGF1 (ng/ml) | ED50 FGF2 (pg/ml) |
|---|---|---|---|---|
| | FGF seul | 0 | 8 | 56 |
| | héparine | 1 | 2 | 35 |
| RGTA 2010 | Pcoo- | 100 | 4 | 56 |
| RGTA 2011 | P1S | 100 | 2.5 | 38 |
| RGTA 2012 | P2S | 100 | 4 | 41 |
| RGTA 0040 | DS commercial | 100 | 3 | 30 |
| RGTA 1024 | DS$_{0,5}$ équiv | 100 | 4 | 36 |
| RGTA 1026 | DS$_{0,125}$ équiv | 100 | 6 | 48 |
| RGTA 1000 | CM$_1$D | 10 | 12 | 168 |
| RGTA 1007 | CM2D | 10 | 16 | 297 |
| RGTA 1005 | CM1DS2 | 10 | 1 | 40 |
| RGTA 1012 | CM2DS2 | 10 | 1,5 | 31 |
| RGTA 1110 | CM$_2$DPhS1 | 10 | 8 | 53 |
| RGTA 1111 | CM$_2$DES1 | 10 | 5 | 45 |
| RGTA 1112 | CM$_2$DPheS2 | 10 | 3 | 38 |
| RGTA 1113 | CM$_3$DTyrS2 | 10 | 2 | 30 |
| RGTA 1114 | CM$_1$DPalmS1 | 10 | 9 | 42 |

Effets potentialisateurs sur le FGF1 et le FGF2

Figure 14

| concentration (μg/ml) | polymère + FGF2 | | | | polymère + FGF1 | | | | polymère + TGFβ | | | |
|---|---|---|---|---|---|---|---|---|---|---|---|---|
| | 500 | 50 | 5 | 0.5 | 500 | 50 | 5 | 0.5 | 100 | 50 | 5 | 0.5 |
| FGF2 seul | 100 | | | | 100 | | | | 100 | | | |
| FGF2 + trypsine | <1 | | | | <1 | | | | <1 | | | |
| héparine (10 μg/ml) | 100 | | | | 100 | | | | <1 | | | |
| trypsine + Pcoo- | 14.4 | 24.4 | 18 | 22 | 29.7 | 25 | 18 | 16 | 5 | <1 | <1 | <1 |
| trypsine + P1S | 61.3 | 100 | 97 | 87.5 | 85 | 96 | 100 | 63 | 58 | 84 | 70 | 22 |
| trypsine + P2S | 59 | 68 | 65 | 57 | 72 | 84 | 91 | 49 | 33 | 75 | 92 | 67 |

Pourcentage de FGF1 et de FGF2 et de TGFβ non dégradé par la trypsine
en présence des polymères poly acide B malique

Figure 15

| | % de protection des facteurs | | Polymères | % de protection des facteurs | |
|---|---|---|---|---|---|
| Polymères | FGF2 | TGFβ | Polymères | FGF2 | TGFβ |
| Héparine | 100 | 15 | $CM_3D$ | 22 | 23 |
| $CM_1D$ | 20 | 25 | $CM_3DS_{0,5}$ | 29 | 32 |
| $CM_1DS_{0,5}$ | 74 | 65 | $CM_3DS_1$ | 32 | 38 |
| $CM_1DS_{0,75}$ | 77 | 71 | $CM_3DS_{1,5}$ | 35 | 40 |
| $CM_1DS_1$ | 80 | 75 | $CM_3DS_2$ | 40 | 47 |
| $CM_1DS_{1,5}$ | 96 | 78 | $CM_2DPhSS1$ | 76 | 67 |
| $CM_1DS_2$ | 100 | 80 | $CM_2DES1$ | 81 | 71 |
| $CM_1DSex$ | 100 | 81 | $CM_2DPheS2$ | 67 | 56 |
| $CM_2D$ | 20 | 25 | $CM_3DTyrS2$ | 83 | 54 |
| $CM_2DS_{0,5}$ | 87 | 74 | $CM_1DPalmS1$ | 67 | 74 |
| $CM_2DS_{0,75}$ | 90 | 77 | $CM_1DOléicS1$ | 58 | 72 |
| $CM_2DS_1$ | 97 | 80 | DS commercial | 87 | 12 |
| $CM_2DS_{1,5}$ | 95 | 79 | $DS_{0,5}$ équiv | 66 | 9 |
| $CM_2DS_2$ | 90 | 80 | $DS_{0,125}$ équiv | 51 | 10 |
| $CM_2DSex$ | 88 | 74 | Dextran T40 | 6 | 5 |

Pourcentage de FGF2 et de TGF β non dégradé par la trypsine en présence des polymères dérivés de dextranes

Figure 16

| Polymères | IC 50 en mg/ml | | Polymères | IC 50 en mg/ml | |
|---|---|---|---|---|---|
| | Elastase | plasmine | | Elastase | plasmine |
| Héparine | 1,8 | 1 | $CM_2DSex$ | 5 | 0,07 |
| Pcoo- | 100 | 53 | $CM_3D$ | >100 | >100 |
| P1S | 2 | 0,98 | $CM_3DS_{0,5}$ | 8 | 6 |
| P2S | 4,7 | 0,82 | $CM_3DS_1$ | 6 | 6 |
| $CM_1D$ | >100 | >100 | $CM_3DS_{1,5}$ | 4 | 6 |
| $CM_1DS_{0,5}$ | 37 | 8 | $CM_3DS_2$ | 2 | 1,5 |
| $CM_1DS_{0,75}$ | 24 | 2,5 | $CM_2DPhS1$ | 12 | 2,4 |
| $CM_1DS_1$ | 20 | 1 | $CM_2DES1$ | 18 | 3,8 |
| $CM_1DS_{1,5}$ | 3 | 0,15 | $CM_2DPheS2$ | 4 | 0,3 |
| $CM_1DS_2$ | 1 | 0.08 | $CM_3DTyrS2$ | 1,8 | 0,15 |
| $CM_1DSex$ | 1 | 0,035 | $CM_1DPalmS1$ | 1,4 | 6 |
| $CM_2D$ | >100 | >100 | $CM_1DOléicS1$ | 2 | 9 |
| $CM_2DS_{0,5}$ | 7 | 1 | DS commercial | >100 | >100 |
| $CM_2DS_{0,75}$ | 5 | 0,7 | $DS_{0,5}$ équiv | >100 | >100 |
| $CM_2DS_1$ | 2 | 0,5 | $DS_{0,25}$ équiv | >100 | >100 |
| $CM_2DS_{1,5}$ | 2 | 0,1 | $DS_{0,125}$ équiv | >100 | >100 |
| $CM_2DS_2$ | 2 | 0,05 | Dextran T40 | >100 | >100 |

Effets inhibiteurs des polymères sur les activités
de l'élastase leucocytaire et de la plasmine

Figure 17

| Produits | doses µg/ml | effets en % du témoin | Produits | doses µg/ml | effets en % du témoin |
|---|---|---|---|---|---|
| Témoin | | 100 | CM1DS1 | 50 | 134 |
| Dextran T40 | 10 | <100 | | 100 | 189 |
| | 50 | <100 | | 200 | 231 |
| | 100 | <100 | CM2D | 50 | <100 |
| | 200 | <100 | | 100 | <100 |
| Héparine | 10 | <100 | CM2DS2 | 20 | 143 |
| | 50 | 120 | | 50 | 138 |
| | 100 | <100 | | 100 | 191 |
| | 200 | <100 | | 200 | 213 |
| DS commercial | 100 | 112 | CM3D | 50 | <100 |
| | 200 | 124 | | 100 | <100 |
| $DS_{0,5}$ équiv | 100 | <100 | CM3DS2 | 50 | 136 |
| | 200 | 121 | | 100 | 147 |
| $DS_{0,25}$ équiv | 100 | 109 | | 200 | 178 |
| | 200 | 125 | $CM_2DPhS1$ | 50 | 115 |
| $DS_{0,125}$ équiv | 100 | <100 | | 100 | 178 |
| | 200 | 129 | | 200 | 189 |
| Pcoo- | 50 | <100 | $CM_2DES1$ | 50 | 137 |
| | 100 | <100 | | 100 | 144 |
| | 200 | <100 | | 200 | 168 |
| P1S | 50 | 150 | $CM_2DPheS2$ | 50 | 152 |
| | 100 | 199 | | 100 | 196 |
| | 200 | 135 | | 200 | 154 |
| P2S | 50 | 152 | $CM_3DTyrS2$ | 50 | 167 |
| | 100 | 170 | | 100 | 241 |
| | 200 | 177 | | 200 | 203 |
| CM1D | 50 | <100 | $CM_1DPalmS1$ | 50 | 133 |
| | 100 | <100 | | 100 | 157 |
| | 200 | <100 | | 200 | 176 |

Pourcentages de régénération musculaire après injection de doses variables de polymères.

Figure 18

| Conditions expérimentales | Activité de la SOD en unités arbitraires |
|---|---|
| SOD témoin à pH = 7 | 100 |
| 50 microg/mL de CM1DS2 + SOD à pH = 7 | 132 |
| 250 microg/mL de CM1DS2 + SOD à pH = 7 | 165 |
| 500 microg/mL de CM1DS2 + SOD à pH = 7 | 196 |
| 50 microg/mL de CM3DTyrS2 + SOD à pH = 7 | 105 |
| 250 microg/mL de CM3DTyrS2 + SOD à pH = 7 | 122 |
| 500 microg/mL de CM3DTyrS2 + SOD à pH = 7 | 118 |
| SOD témoin à pH = 3 | 20 |
| 50 microg/mL de CM1DS2 + SOD à pH = 3 | 65 |
| 250 microg/mL de CM1DS2 + SOD à pH = 3 | 115 |
| 500 microg/mL de CM1DS2 + SOD à pH = 3 | 130 |
| 50 microg/mL de CM3DTyrS2 + SOD à pH = 3 | 85 |
| 250 microg/mL de CM3DTyrS2 + SOD à pH = 3 | 133 |
| 500 microg/mL de CM3DTyrS2 + SOD à pH = 3 | 150 |
| SOD témoin à pH = 11 | 30 |
| 50 microg/mL de CM1DS2 + SOD à pH = 11 | 40 |
| 250 microg/mL de CM1DS2 + SOD à pH = 11 | 95 |
| 500 microg/mL de CM1DS2 + SOD à pH = 11 | 122 |
| 50 microg/mL de CM3DTyrS2 + SOD à pH = 11 | 65 |
| 250 microg/mL de CM3DTyrS2 + SOD à pH = 11 | 93 |
| 500 microg/mL de CM3DTyrS2 + SOD à pH = 11 | 110 |

Modulation de l'activité in vitro de la SOD par les polymères :
Effets protecteurs et potentialisateurs de deux RGTA, le RGTA 1005
(CM1DS2) et le RGTA 1113 (CM3DTyrS2)
sur l'activité de la SOD *in vitro*

Figure 19

| Polymères à 100 mg/ml | % d'activité résiduelle | | Polymères à 100 mg/ml | % d'activité résiduelle | |
|---|---|---|---|---|---|
| | SOD + Trypsine | SOD à 60 °C | | SOD + Trypsine | SOD à 60 °C |
| Rien | 0 | 0 | $CM_3D$ | 0 | 0 |
| Héparine | 60 | 45 | $CM_3DS_{0,5}$ | 55 | 60 |
| Pcoo- | 0 | 0 | $CM_3DS_1$ | 30 | 40 |
| P1S | 70 | 80 | $CM_3DS_2$ | 10 | 20 |
| P2S | 80 | 80 | $CM_4D$ | 0 | 0 |
| $CM_1D$ | 0 | 0 | $CM_4DS_{0,5}$ | 60 | 100 |
| $CM_1DS_{0,5}$ | 90 | 70 | $CM_4DS_1$ | 80 | 100 |
| $CM_1DS_{0,75}$ | 100 | 70 | $CM_2DPhSS1$ | 50 | 60 |
| $CM_1DS_1$ | 100 | 90 | $CM_2DES1$ | 60 | 80 |
| $CM_1DS_{1,5}$ | 95 | 75 | $CM_2DPheS2$ | 80 | 100 |
| $CM_1DS_2$ | 100 | 85 | $CM_3DTyrS2$ | 80 | 100 |
| $CM_1DSex$ | 90 | 90 | $CM_1DPalmS1$ | 75 | 60 |
| $CM_2D$ | 0 | 0 | $CM_1DOléicS1$ | 70 | 50 |
| $CM_2DS_{0,5}$ | 90 | 55 | DS commercial | 20 | 10 |
| $CM_2DS_1$ | 100 | 70 | $DS_{0,5}$ équiv | 30 | 20 |
| $CM_2DS_{1,5}$ | 70 | 85 | $DS_{0,25}$ équiv | 20 | 0 |
| $CM_2DS_2$ | 90 | 60 | $DS_{0,125}$ équiv | 20 | 0 |
| $CM_2DSex$ | 70 | 40 | Dextran T40 | 0 | 0 |

Effets protecteurs des polymères sur la SOD après traitement par la trypsine et choc thermique

Figure 20

Effets potentialisateurs de la SOD produite *in vitro*
par des monocytes activés

Figure 21

| Polymères | % d'activité résiduelle |
|---|---|
| Dextran T40 | 100 |
| $CM_1D$ | 100 |
| $CM_1DS_{0,5}$ | 30 |
| $CM_1DS_1$ | 10 |
| $CM_1DS_{1,5}$ | 0 |
| $CM_1DS_2$ | 0 |
| $CM_1DSex$ | 20 |
| $CM_2D$ | 100 |
| $CM_2DS_{0,5}$ | 10 |
| $CM_2DS_1$ | 0 |
| $CM_2DS_2$ | 0 |
| $CM_2DSex$ | 10 |
| $CM_2DPhSS1$ | 10 |
| $CM_2DES1$ | 0 |
| $CM_2DPheS2$ | 0 |
| $CM_3DTyrS2$ | 0 |
| $CM_1DPalmS1$ | 35 |
| $CM_1DOléicS1$ | 50 |
| DS commercial | 100 |

Effets inhibiteurs des RGTA sur la calpaïne

Figure 22

| % d'activité résiduelle | héparanase | % d'activité résiduelle | héparanase |
|---|---|---|---|
| Dextran T40 | 0 | $CM_3D$ | 0 |
| $CM_1D$ | 0 | $CM_3DS_{0,5}$ | 90 |
| $CM_1DS_{0,5}$ | 60 | $CM_3DS_1$ | 100 |
| $CM_1DS_1$ | 100 | $CM_3DS_2$ | 100 |
| $CM_1DS_{1,5}$ | 100 | $CM_4D$ | 0 |
| $CM_1DS_2$ | 100 | $CM_4DS_1$ | 75 |
| $CM_1DSex$ | 100 | $CM_4DS_2$ | 60 |
| $CM_2D$ | 0 | $CM_2DPhSS1$ | 80 |
| $CM_2DS_{0,5}$ | 80 | $CM_2DES1$ | 90 |
| $CM_2DS_1$ | 100 | $CM_2DPheS2$ | 100 |
| $CM_2DS_2$ | 100 | $CM_3DTyrS2$ | 100 |
| $CM_2DSex$ | 100 | $CM_1DPalmS1$ | 60 |
| DS commercial | 100 | Héparine | 100 |

Effets inhibiteurs des RGTA sur l'héparitinase

Figure 23

Actions des RGTA sur la sécrétion des collagènes *in vitro* par les cellules HISM soumises à des radiations ionisantes de $^{60}$Co.

Figure 24

Action des RGTA sur la synthèse des collagènes de type I, II et V par les cellules HISM soumises à des radiations ionisantes de $^{60}$Co

Figure 25

Effets protecteurs des RGTA sur la survie de cellules
soumises à des irradiations au $^{60}$ Co

Figure 26

| | Inhibition de prolifé- ration | $IC_{50}$ Inhibition en $\mu g$ M | (%) synthèse collagène/ protéines | Collagène Type 1 en % du total 1+3+5 | Collagène Type 3 en % du total 1+3+5 | Collagène Type 5 en % du total 1+3+5 |
|---|---|---|---|---|---|---|
| Dextran T40 | 0 | | 17,1 | 58,7 | 36,9 | 4,4 |
| $CM_1D$ | 0 | | 17,6 | 59,1 | 35,8 | 5,1 |
| $CM_1DS_2$ | 85 | 0,62 | 11,4 | 58,1 | 21,8 | 14,1 |
| $CM_2DS_1$ | 75 | 0,47 | 9,3 | 58,7 | 15,8 | 25,5 |
| $CM_2DPheS2$ | 85 | 1,12 | 12,1 | 68,5 | 18,5 | 13,0 |
| $CM_3DTyrS2$ | 80 | 0,95 | 10,8 | 65,5 | 20,6 | 13,9 |
| Héparine | 82 | 0,36 | 15,5 | 73,0 | 20,9 | 6,1 |

Action antifibrotique des RGTA sur des cellules musculaires lisses
d'aorte de porc

Fig.27

Fig.28

Fig.29

Fig.30

Effets des RGTA sur la régulation de la masse osseuse et sur la
qualité de son remodelage :
Exemple d'une parodontie chronique

# EP 1 117 695 B1

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description

- FR 2461724
- US 4740594 A
- FR 2644066
- FR 2718023
- FR 2718024
- FR 2718026
- FR 2718025
- DE 1152396
- US 5693625 A

### Littérature non-brevet citée dans la description

- **TARDIEU.** *Journal of Cellular Physiology,* 1992, vol. 150, 194-203
- **MAIGA-REVEL et al.** *Carbohydrate polymers,* 1997, vol. 32, 89-93
- **WIESSLER M et al.** *Carcinogenesis,* 1983, vol. 4 (7), 867-871
- **SINGER GM et al.** *J Med. Chem.,* 1983, vol. 26 (3), 309-312
- **BLANQUAERT F et al.** *Bone,* 1995, vol. 17 (6), 499-506
- **SHAHEN.** Effects of various stressors on the level of lipid peroxide antioxidants and Na+, K+ - ATPases activity in brain. *Experentia,* 1996, vol. 52, 336-339
- **BOSTANTJOPOULOS.** Super oxide dismutase activity in early and advanced Parkinson's Disease. *Funct. Neurol.,* 1997, vol. 12, 63-68
- **FERNANDEZ NOVOA.** *Methods Find Exper Clin Pharmacol,* 1997, vol. 9, 99-106
- **D'AGNILLO ; CHANG.** Reduction of hydroxyl radical generation in a rat hindlimb model of ischemia - reperfusion injury using cross linked hemoglobin - superoxide dismutase - catalase. *Artif. Cells Blood Subsiti Immobil Biotechnol,* 1997, vol. 25, 163-80
- **NAKAUCHI.** Effects of lecithinized super oxide dismutase on rat spinal cord injury. *J. Neurotrauma,* 1996, vol. 13, 573-82
- **SUPINSKI.** Effect of free radical scavengers on diaphragmeatic fatigue. *Am. J. Respir. Crit. Care Med.,* 1997, vol. 155, 622
- **SZABO.** Direct measurement of free radicals in ischemic/reperfused diabetic rat retina. *Clin. Neurosci.,* 1997, vol. 4, 240-5
- Serum, zinc, copper, magnesium, proteins and super oxide dismutase in leprosy patients on multidrug therapy-a follow up study. *Indian J. Lepr.,* 1996, vol. 68, 325-33
- Hepatic response to the oxydative stress induced by E. Coli endotoxin. *Mol. Cell. Biochem.,* 1996, vol. 159, 115-121
- Prevention of radioinduced cystisi by orgotein; a random ranomized study. *Anticancer Res.,* 1996, vol. 16, 2025-8
- **KANDASAMY.** Involvement of superoxide dismutase and gluthatione peroxydase in attenuation of radiation-induced hyperthermia by interleukin 1 alpha in rats. *Brain res.,* 1993, vol. 606, 106-10
- **OGUNI.** Chronical retinal effects by ultraviolet irradiation with special référence to superoxide dismutase. *Histol. Histopathol,* 1996, vol. 11, 695-702
- **KOCH.** Effects of different antioxidants on lens-induced uveitis. *Ger. J. Ophthalmol.,* 1996, vol. 5, 1185-8
- **JUN KE YAN ; CATALANO.** Increase superoxide anion production in humans, a possible mechanism for the pathogenesis of hypertension. *J. Hum. Hypertens,* 1996, vol. 10, 305-309
- **RAZAK.** Crosslinked hemoglobine-superoxide dismutase-catalase scavenges free radicals in a rat model of intestinal ischemia-reperfusion injury. *Artif. cells Blood substiti immobil. Biotechnol.,* 1997, vol. 25, 181-192
- **GUÉRIN.** Optically active poly($\beta$-malic acid). *Polymer Bulletin,* 1985, vol. 14, 187
- **HOUSSE-FERRARI.** Préparation et caractérisation de silices poreuses modifiées par des polymères et des copolymères de N,N' diméthylacrymide. *Thèse de l'université Paris VI,* 30 Janvier 1990
- **MAUZAC.** Anticoagulant activities of dextran derivatives Part I : Synthesis and characterization. *Biomaterials,* 1984, vol. 6, 61-63
- **BIGGS.** Human blood coagulation. Oxford Blackwell Scientific Publications, 1972
- **LAEMMLI U.K.** Cleavage of structural proteins during assembly of the head of the bacteriophage T4. *Nature,* 1970, vol. 227, 680-685

- **FREUND M ; PICK E.** The mechanism of action of lymphokines. IX. The enzymatic basis of hydrogen peroxide production by lymphokine-activated macrophages. *J Immunol,* 1986, vol. 137 (4), 1312-1318
- **SASAKI, KIKUCHI ; YUMOTO N ; YOSHIMURA ; MURACHI T.** *J. Biol. Chem,* 1984, vol. 259 (20), 12489-12494
- **MARKGRAF C G.** *Stroke,* 1998, vol. 29, 152-158
- **SAIDO et al.** *Neuroscience. Letters,* 1997, vol. 16 (227), 75-78
- **ISHAI-MICHAELI R.** Importance of size and sulfation of heparin in release of basic fibroblast growth factor from the vascular endothelium and extracellular matrix. *Biochemistry,* 1992, vol. 31 (7), 2080-2088
- **ISHAI-MICHAELI.** *Biochemistry,* 1992, vol. 31 (7), 2080-2088
- **GRAHAM M. ; DIEGELMANN R. ; ELSON C. ; BITAR K. ; EHRLICH H.** *Proc. Soc. Exp. Biol. Med.,* 1984, vol. 176, 503
- **ROJKIND M ; GONZALEZ E.** An improved method for determining specific radioactivities of proline-14C and hydroxyproline-14C in collagen and in noncollagenous proteins. *Analytical Biochemistry,* 1974, vol. 57 (1), 1-7
- **HANSEN-SMITH, F.M. ; CARLSON, B.M. ; IRWIN, K.L.** Revascularization of the freely grafted Extensor digitorum Longus muscle in the rat. *Am. J. Anat.,* 1980, vol. 158, 65-82
- **F. BLANQUAERT.** *Bone,* 1995, vol. 17, 499-506